# EUROPEAN PATENT APPLICATION

(11) **EP 3 315 507 A1**
(43) Date of publication of application: **02.05.2018**
(21) Application number: 16814541.5
(22) Date of filing: 27.06.2016
(51) Int. Cl.: C07H 21/00, C12N 15/113

(54) **OLIGONUCLEOTIDE DERIVATIVE**

(30) Priority: 26.06.2015 JP 2015128416
(71) Applicant: Kyowa Hakko Kirin Co., Ltd., Tokyo 100-8185 (JP)
(72) Inventor: YAMAMOTO, Junichiro, Tokyo 100-8185 (JP); SAWADA, Takashi, Tokyo 100-8185 (JP); HAGIWARA, Kenji, Tokyo 100-8185 (JP); ATSUMI, Toshiyuki, Tokyo 100-8185 (JP); SHINOHARA, Fumikazu, Tokyo 100-8185 (JP)
(74) Representative: D Young & Co LLP
(86) International application number: PCT/JP2016/069057
(87) International publication number: WO 2016/208773

(57) **Abstract**

The present invention provides an oligonucleotide derivative having, at an oxygen atom of at least one phosphate group of an oligonucleotide, a group represented by formula (I): 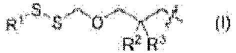 (wherein R¹ represents lower alkyl or the like; and R² and R³ are the same or different and respectively represent an electron-withdrawing group or the like), or a salt thereof.

## Description

### Technical Field

The present invention relates to, for example, an oligonucleotide derivative in which one or more phosphate groups of an oligonucleotide are modified.

### Background Art

Small interfering RNAs (hereinafter siRNAs) are involved in RNA interference (hereinafter RNAi) and serve as a guide for suppressing expression of target genes [Nature, vol. 411, No. 6836, p. 494-498 (2001)]. siRNAs can selectively suppress (knock down) expression of proteins expressed by messenger RNAs (mRNAs), via cleavage of the mRNAs, and thus it is expected that siRNAs are applied for medicaments [Nature Reviews Cancer, vol. 11, p. 59-67 (2011)].

siRNAs are generally incorporated into complexes called RNA induced silencing complexes (RISCs) to exhibit the function thereof. A siRNA incorporated into an RISC becomes a single antisense strand through cleavage of a sense strand and then the antisense strand binds to a target mRNA which is complementary to the antisense strand. It is known that the target mRNA is cleaved by an RNase domain in a protein called Argonaute 2 (AGO2), thereby suppressing expression of a protein from the target mRNA [Silence, vol. 1, p. 3 (2010)].

Meanwhile, oligonucleotides such as siRNAs and antisense oligonucleotides (ASOs) have low lipophilicity and poor cell membrane permeability because the oligonucleotides are polyanion molecules having a plurality of phosphodiester moieties. It is known that the cell membrane permeability of siRNAs or ASOs is increased by protecting non-crosslinked oxygen atoms in phosphodiester moieties thereof with protecting groups (Patent Document 1 and Non-Patent Documents 1 to 3). The siRNAs and ASOs disclosed in the above documents exhibit RNAi activity or knockdown activity due to RNase H in cells as the protecting groups are removed in intracellular environment by, for example, glutathione existing in cells at high concentration and they are converted into compounds having phosphodiester moieties, which are required for RNAi activity or knockdown activity due to RNase H (Patent Document 1 and Non-Patent Documents 1 to 3).

### Citation List

### Patent Document

Patent Document 1: WO 2008/008476

### Non-Patent Documents

Non-Patent Document 1: Chem. Commun., 2009, p. 3216-3218
Non-Patent Document 2: Nature Biotechnology, 2014, vol. 32, p. 1256-1261
Non-Patent Document 3: Antisense & Nucleic Acid Drug Development, 2002, vol. 12, p. 33-41

### Summary of Invention

Problems to be solved by the invention [0007] An object of the present invention is to provide, for example, an oligonucleotide derivative in which one or more phosphate groups of an oligonucleotide are modified.

Means for Solving the Problem [0008] The present invention relates to (1) to (41) below.
(1) An oligonucleotide derivative having, at an oxygen atom of at least one phosphate group of an oligonucleotide, a group represented by formula (I): 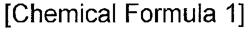 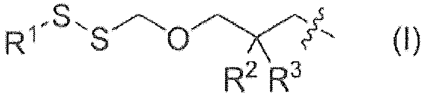 {wherein R¹ represents lower alkyl optionally substituted with lower alkoxy; R² and R³ are the same or different and respectively represent an electron-withdrawing group or R², R³ and the carbon atom adjacent to them together represent formula (II): 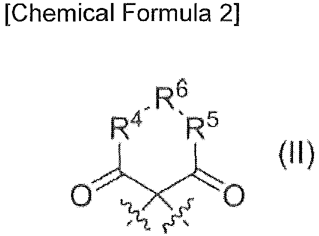 [wherein R⁴ and R⁵ are the same or different and respectively represent oxygen atom, CH₂ or NR⁷ (wherein R⁷ represents hydrogen atom or lower alkyl); and R⁶ represents a bond or CR⁸R⁹ (wherein R⁸ and R⁹ are the same or different and respectively represent hydrogen atom or lower alkyl)]}, or a salt thereof.
(2) The oligonucleotide derivative or a salt thereof according to (1), having, at an oxygen atom of one phosphate group of the oligonucleotide, the group represented by formula (I).
(3) An oligonucleotide derivative containing one or more structures represented by the following formula (III): 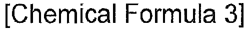 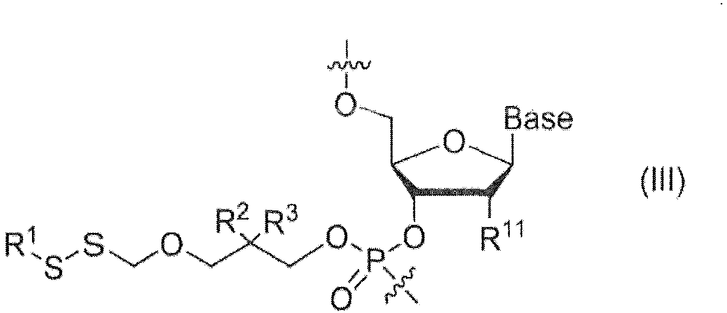 (wherein Base represents a nucleobase; R¹, R² and R³ respectively are as defined above; and R¹¹ represents hydrogen atom, fluorine atom or lower alkoxy) or a salt thereof (provided that when the oligonucleotide derivative or a salt thereof has two or more structures represented by formula (III) which are one or more different types, Base, R¹, R², R³ and R¹¹ may respectively be the same or different between the structures).
(4) The oligonucleotide derivative or a salt thereof according to (1), containing one or more structures represented by formula (III): 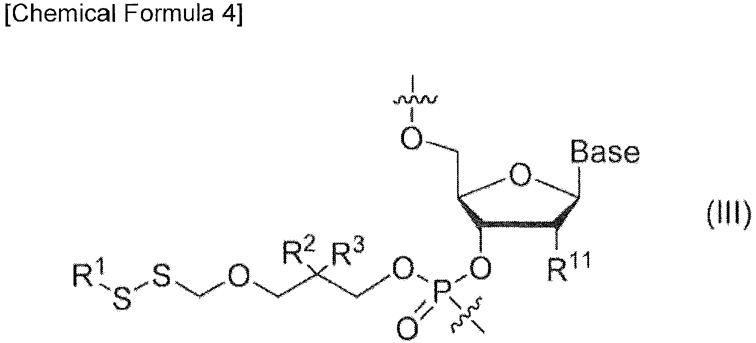 wherein Base represents a nucleobase; R¹, R² and R³ respectively are as defined above; and R¹¹ represents hydrogen atom, fluorine atom or lower alkoxy (provided that when the oligonucleotide derivative or a salt thereof has two or more structures represented by formula (III), Base, R¹, R², R³ and R¹¹ may respectively be the same or different between the structures).
(5) The oligonucleotide derivative or a salt thereof according to any one of (1) to (4), wherein R¹ represents lower alkyl.
(6) The oligonucleotide derivative or a salt thereof according to any one of (1) to (5), wherein R² and R³ are the same or different and respectively represent carboxy or lower alkoxycarbonyl.
(7) The oligonucleotide derivative or a salt thereof according to any one of (1) to (5), wherein R² and R³ are the same or different and respectively represent cyano, nitro, lower alkanoyl or lower alkylsulfonyl.
(8) The oligonucleotide derivative or a salt thereof according to any one of (3) to (7), containing only one structure represented by formula (III).
(9) The oligonucleotide derivative or a salt thereof according to any one of (1) to (8), having a base length of 5 to 100.
(10) The oligonucleotide derivative or a salt thereof according to any one of (1) to (8), having a base length of 10 to 80.
(11) The oligonucleotide derivative or a salt thereof according to any one of (1) to (10), wherein the oligonucleotide is a double strand.
(12) The oligonucleotide derivative or a salt thereof according to any one of (1) to (10), wherein the oligonucleotide is a single strand.
(13) The oligonucleotide derivative or a salt thereof according to any one of (1) to (12), wherein the oligonucleotide is a small interfering RNA (siRNA).
(14) An oligonucleotide derivative having, at an oxygen atom of at least one phosphate group of an oligonucleotide, a group represented by formula (I-0): 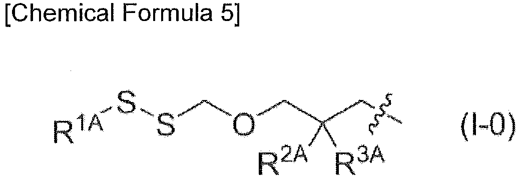 {wherein R^{1A} represents optionally substituted lower alkyl, optionally substituted lower alkenyl or optionally substituted lower alkynyl; R^{2A} and R^{3A} are the same or different and respectively represent an electron-withdrawing group or R^{2A}, R^{3A} and the carbon atom adjacent to them together represent formula (II-0): 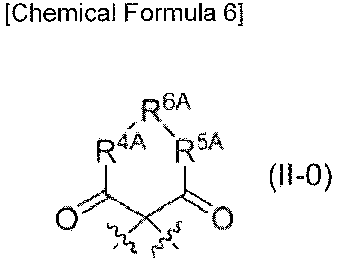 [wherein R^{4A} and R^{5A} are the same or different and respectively represent oxygen atom, CH₂ or NR^{7A} (wherein R^{7A} represents hydrogen atom or lower alkyl); and R^{6A} represents a bond or CR^{8A}R^{9A} (wherein R^{8A} and R^{9A} are the same or different and respectively represent hydrogen atom or lower alkyl)]}, or a salt thereof.
(15) The oligonucleotide derivative or a salt thereof according to (14), having, at an oxygen atom of one phosphate group of the oligonucleotide, the group represented by formula (I-0).
(16) The oligonucleotide derivative or a salt thereof according to (13), having one or more structures represented by formula (III-0): 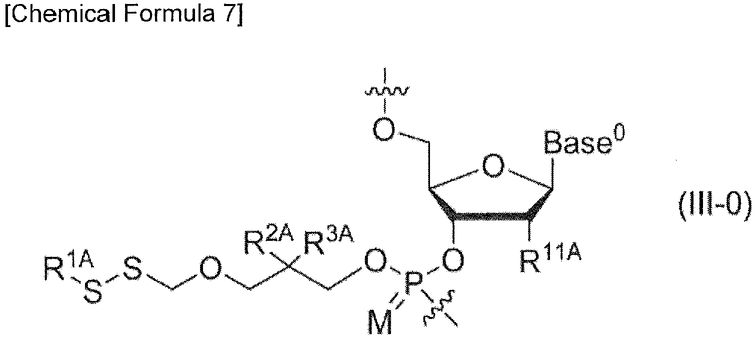 (wherein Base⁰ represents a nucleobase; M represents oxygen atom or sulfur atom; R^{1A}, R^{2A} and R^{3A} respectively are as defined above; R^{11A} represents hydrogen atom, fluorine atom or lower alkoxy)(provided that when the oligonucleotide derivative or a salt thereof has two or more structures represented by formula (III-0), Base⁰, M, R^{1A}, R^{2A}, R^{3A} and R^{11A} may respectively be the same or different between the structures).
(17) The oligonucleotide derivative or a salt thereof according to any one of (14) to (16), wherein R^{1A} represents optionally substituted lower alkyl.
(18) The oligonucleotide derivative or a salt thereof according to any one of (14) to (17), wherein R^{2A} and R^{3A} are the same or different and respectively represent carboxy, optionally substituted aralkyloxycarbonyl, lower alkoxycarbonyl, lower alkenyloxycarbonyl or lower alkynyloxycarbonyl.
(19) The oligonucleotide derivative or a salt thereof according to any one of (14) to (17), wherein R^{2A} and R^{3A} are the same or different and respectively represent cyano, nitro, lower alkanoyl or lower alkylsulfonyl.
(20) The oligonucleotide derivative or a salt thereof according to any one of (16) to (19), containing only one structure represented by formula (III-0).
(21) The oligonucleotide derivative or a salt thereof according to (14), containing a structure represented by formula (III-1): 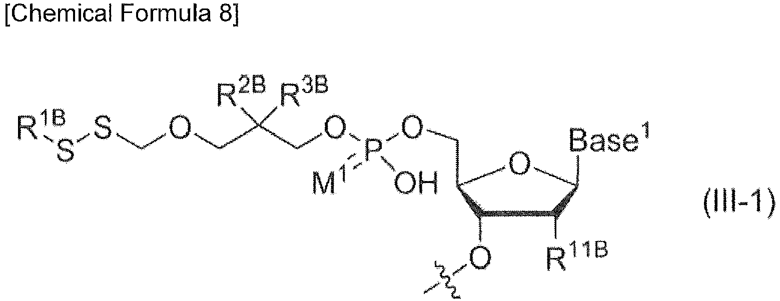 {wherein Base¹ represents a nucleobase; M¹ represents oxygen atom or sulfur atom; R^{1B} represents optionally substituted lower alkyl, optionally substituted lower alkenyl or optionally substituted lower alkynyl; R^{2B} and R^{3B} are the same or different and respectively represent an electron-withdrawing group or R^{2B}, R^{3B} and the carbon atom adjacent to them together represent formula (II-1): 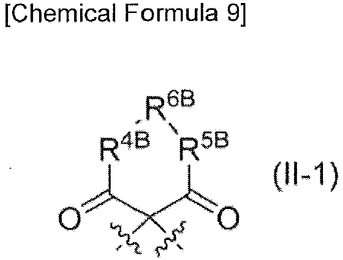 [wherein R^{4B} and R^{5B} are the same or different and respectively represent oxygen atom, CH₂ or NR^{7B} (wherein R^{7B} represents hydrogen atom or lower alkyl); R^{6B} represents a bond or CR^{8B}R^{9B} (wherein R^{8B} and R^{9B} are the same or different and respectively represent hydrogen atom or lower alkyl)]; and R^{11B} represents hydrogen atom, fluorine atom or lower alkoxy}.
(22) The oligonucleotide derivative or a salt thereof according to (21), wherein R^{1B} represents optionally substituted lower alkyl.
(23) The oligonucleotide derivative or a salt thereof according to (21) or (22), wherein R^{2B} and R^{3B} are the same or different and respectively represent carboxy, optionally substituted aralkyloxycarbonyl, lower alkoxycarbonyl, lower alkenyloxycarbonyl or lower alkynyloxycarbonyl.
(24) The oligonucleotide derivative or a salt thereof according to (21) or (22), wherein R^{2B} and R^{3B} are the same or different and respectively represent cyano, nitro, lower alkanoyl or lower alkylsulfonyl.
(25) The oligonucleotide derivative or a salt thereof according to any one of (14) to (24), having a base length of 5 to 100.
(26) The oligonucleotide derivative or a salt thereof according to any one of (14) to (24), having a base length of 10 to 80.
(27) The oligonucleotide derivative or a salt thereof according to any one of (14) to (26), wherein the oligonucleotide is a double strand.
(28) The oligonucleotide derivative or a salt thereof according to any one of (14) to (26), wherein the oligonucleotide is a single strand.
(29) The oligonucleotide derivative or a salt thereof according to any one of (14) to (28), wherein the oligonucleotide is a small interfering RNA (siRNA).
(30) A compound represented by formula (IV): 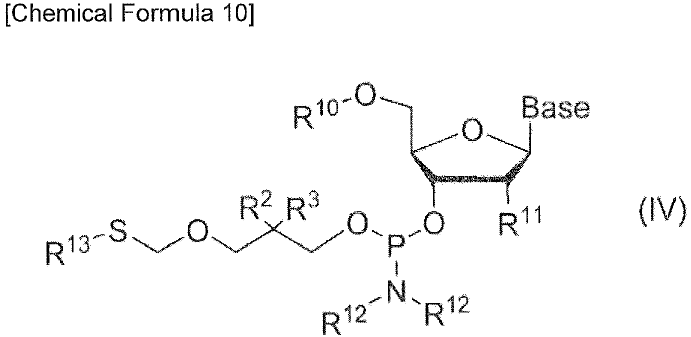 {wherein Base represents a nucleobase; R² and R³ are the same or different and respectively represent an electron-withdrawing group or R², R³ and the carbon atom adjacent to them together represent formula (II): 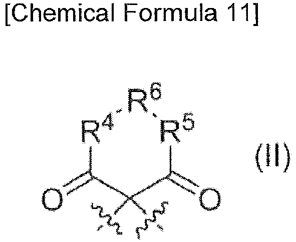 [wherein R⁴ and R⁵ are the same or different and respectively represent oxygen atom, CH₂ or NR⁷ (wherein R⁷ represents hydrogen atom or lower alkyl), R⁶ represents a bond or CR⁸R⁹ (wherein R⁸ and R⁹ are the same or different and respectively represent hydrogen atom or lower alkyl)]; R¹⁰ represents a protecting group of hydroxy group; R¹¹ represents hydrogen atom, fluorine atom or lower alkoxy; R¹² represents lower alkyl; and R¹³ represents lower alkylthio or formula (V): 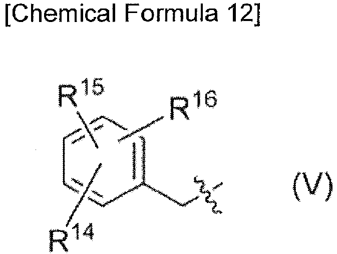 [wherein R¹⁴, R¹⁵ and R¹⁶ are the same or different and respectively represent hydrogen atom, lower alkyl, lower alkoxy or NR¹⁷R¹⁸ (wherein R¹⁷ and R¹⁸ are the same or different and respectively represent lower alkyl)]}, or a salt thereof.
(31) A compound represented by formula (IV-0): 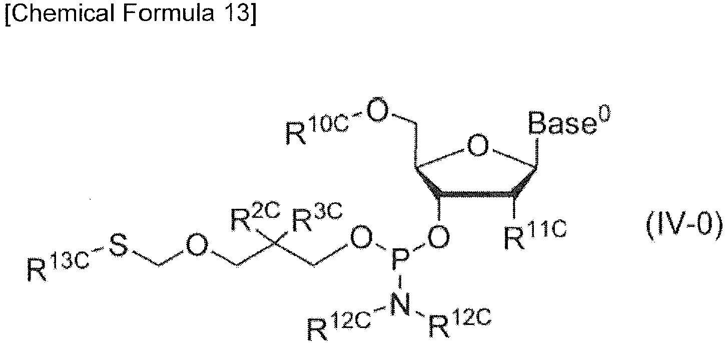 {wherein Base⁰ represents a nucleobase, R^{2C} and R^{3C} are the same or different and respectively represent an electron-withdrawing group or R^{2C}, R^{3C} and the carbon atom adjacent to them together represent formula (II-2): 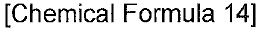 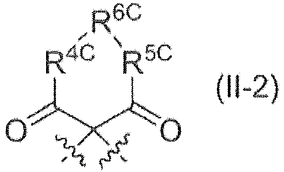 [wherein R^{4C} and R^{5C} are the same or different and respectively represent oxygen atom, CH₂ or NR^{7C} (wherein R^{7C} represents hydrogen atom or lower alkyl); R^{6C} represents a bond or CR^{8C}R^{9C} (wherein R^{8C} and R^{9C} are the same or different and respectively represent hydrogen atom or lower alkyl)], R^{10C} represents a protecting group of hydroxy group; R^{11C} represents hydrogen atom, fluorine atom or lower alkoxy; R^{12C} represents lower alkyl; and R^{13C} represents optionally substituted lower alkylthio, optionally substituted lower alkenylthio, optionally substituted lower alkynylthio or formula (V-0): 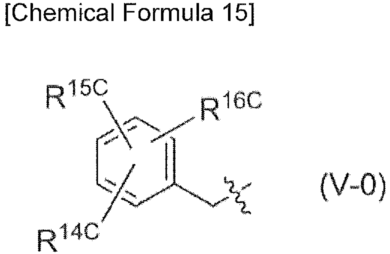 [wherein R^{14C}, R^{15C} and R^{16C} are the same or different and respectively represent hydrogen atom, lower alkyl, lower alkoxy or NR^{17C}R^{18C} (wherein R^{17C} and R^{18C} are the same or different and respectively represent lower alkyl)]}, or a salt thereof.
(32) The compound or a salt thereof according to (31), wherein R^{2C} and R^{3C} are the same or different and respectively represent carboxy, optionally substituted aralkyloxycarbonyl, lower alkoxycarbonyl, lower alkenyloxycarbonyl or lower alkynyloxycarbonyl.
(33) The compound or a salt thereof according to (31), wherein R^{2C} and R^{3C} are the same or different and respectively represent cyano, nitro, lower alkanoyl or lower alkylsulfonyl.
(34) The compound or a salt thereof according to any one of (31) to (33), wherein R^{10C} represents trityl, 4-methoxytrityl or 4,4'-dimethoxytrityl.
(35) A compound represented by formula (IV-1): 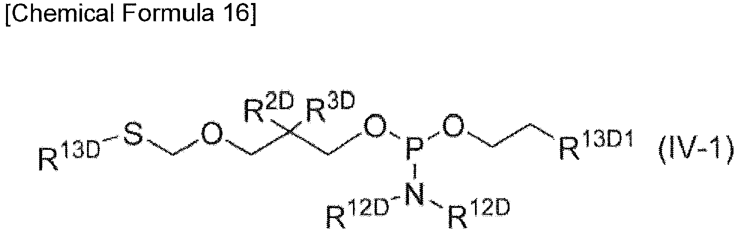 {wherein R^{2D} and R^{3D} are the same or different and respectively represent an electron-withdrawing group or R^{2D}, R^{3D} and the carbon atom adjacent to them together represent formula (II-3): 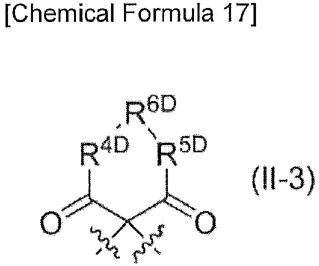 [wherein R^{4D} and R^{5D} are the same or different and respectively represent oxygen atom, CH₂ or NR^{7D} (wherein R^{7D} represents hydrogen atom or lower alkyl); R^{6D} represents a bond or CR^{8D}R^{9D} (wherein R^{8D} and R^{9D} are the same or different and respectively represent hydrogen atom or lower alkyl)], R^{12D} represents lower alkyl; R^{13D} represents optionally substituted lower alkylthio, optionally substituted lower alkenylthio, optionally substituted lower alkynylthio or formula (V-1): 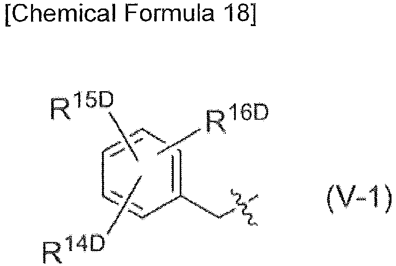 [wherein R^{14D}, R^{15D} and R^{16D} are the same or different and respectively represent hydrogen atom, lower alkyl, lower alkoxy or NR^{17D}R^{18D} (wherein R^{17D} and R^{18D} are the same or different and respectively represent lower alkyl)] and R^{13D1} represents cyano, nitro, carboxy, lower alkoxycarbonyl, lower alkylsulfonyl or optionally substituted arylsulfonyl, or a salt thereof.
(36) The compound or a salt thereof according to (35), wherein R^{2D} and R^{3D} are the same or different and respectively represent carboxy, optionally substituted aralkyloxycarbonyl, lower alkoxycarbonyl, lower alkenyloxycarbonyl or lower alkynyloxycarbonyl.
(37) The compound or a salt thereof according to (35), wherein R^{2D} and R^{3D} are the same or different and respectively represent cyano, nitro, lower alkanoyl or lower alkylsulfonyl.
(38) A compound represented by formula (VI): 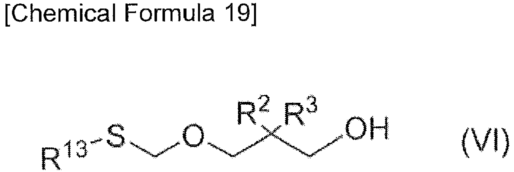 {wherein R² and R³ are the same or different and respectively represent an electron-withdrawing group or R², R³ and the carbon atom adjacent to them together represent formula (II): 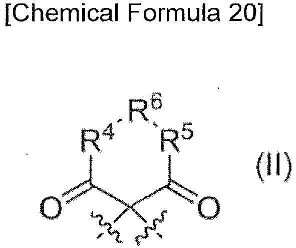 [wherein R⁴ and R⁵ are the same or different and respectively represent oxygen atom, CH₂ or NR⁷ (wherein R⁷ represents hydrogen atom or lower alkyl); R⁶ represents a bond or CR⁸R⁹ (wherein R⁸ and R⁹ are the same or different and respectively represent hydrogen atom or lower alkyl)] and R¹³ represents lower alkylthio or formula (V): 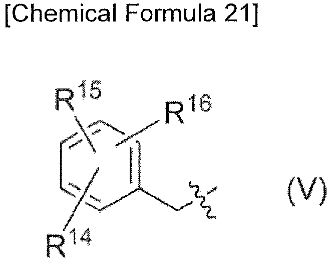 [wherein R¹⁴, R¹⁵ and R¹⁶ are the same or different and respectively represent hydrogen atom, lower alkyl, lower alkoxy or NR¹⁷R¹⁸ (wherein R¹⁷ and R¹⁸ are the same or different and respectively represent lower alkyl)]}, or a salt thereof.
(39) A compound represented by formula (VI-0): 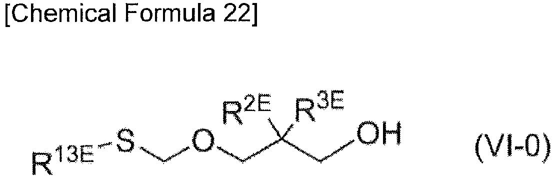 {wherein R^{2E} and R^{3E} are the same or different and respectively represent an electron-withdrawing group or R^{2E}, R^{3E} and the carbon atom adjacent to them together represent formula (II-4): 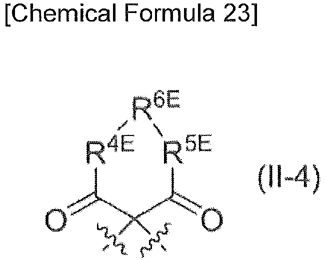 [wherein R^{4E} and R^{5E} are the same or different and respectively represent oxygen atom, CH₂ or NR^{7E} (wherein R^{7E} represents hydrogen atom or lower alkyl); R^{6E} represents a bond or CR^{8E}R^{9E} (wherein R^{8E} and R^{9E} are the same or different and respectively represent hydrogen atom or lower alkyl)] and R^{13E} represents optionally substituted lower alkylthio, optionally substituted lower alkenylthio, optionally substituted lower alkynylthio or formula (V-2): 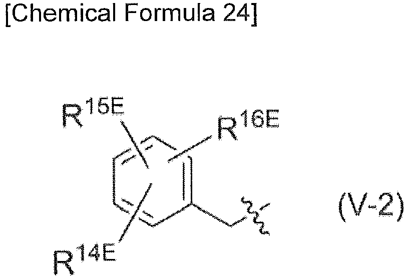 [wherein R^{14E}, R^{15E} and R^{16E} are the same or different and respectively represent hydrogen atom, lower alkyl, lower alkoxy or NR^{17E}R^{18E} (wherein R^{17E} and R^{18E} are the same or different and respectively represent lower alkyl)]}, or a salt thereof.
(40) The compound or a salt thereof according to (39), wherein R^{2E} and R^{3E} are the same or different and respectively represent carboxy, optionally substituted aralkyloxycarbonyl, lower alkoxycarbonyl, lower alkenyloxycarbonyl or lower alkynyloxycarbonyl.
(41) The compound or a salt thereof according to (39), wherein R^{2E} and R^{3E} are the same or different and respectively represent cyano, nitro, lower alkanoyl or lower alkylsulfonyl.

Effects of the Invention [0012] According to the present invention, an oligonucleotide derivative or the like is provided in which one or more phosphate groups of an oligonucleotide are modified.

### Brief Description of Drawings

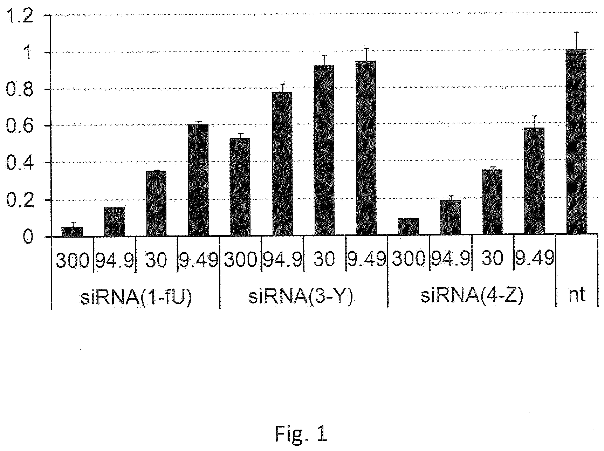

Fig. 1 shows knockdown activity by hypoxanthine-guanine phosphoribosyltransferase 1(HPRT1)-targeting siRNA (1-fU), siRNA (3-Y) and siRNA (4-Z) in HepG2 cells. The horizontal axis in Fig. 1 indicates the test results of introduction of siRNA (1-fU), siRNA (3-Y) and siRNA (4-Z) respectively at concentrations of 300 pmol/L, 94.9 pmol/L, 30 pmol/L and 9.49 pmol/L into cultured cells using a transfection reagent and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group) which was regarded as 1. siRNA (1-fU) serves as a positive control group and siRNA (3-Y) serves as a negative control group.

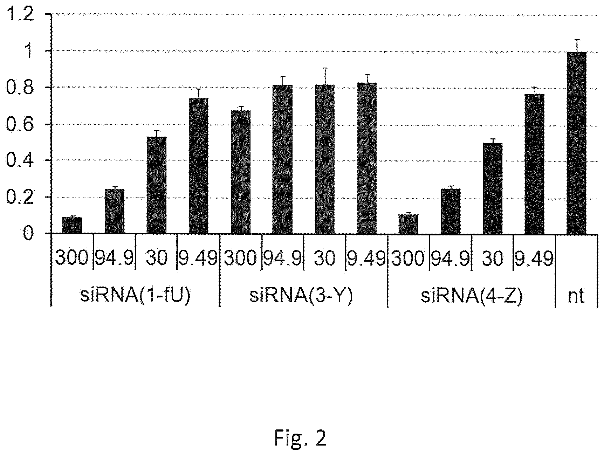

Fig. 2 shows knockdown activity by HPRT1-targeting siRNA (1-fU), siRNA (3-Y) and siRNA (4-Z) in HeLa cells. The horizontal axis in Fig. 2 indicates the test results of introduction of siRNA (1-fU), siRNA (3-Y) and siRNA (4-Z) respectively at concentrations of 300 pmol/L, 94.9 pmol/L, 30 pmol/L and 9.49 pmol/L into cultured cells using a transfection reagent and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group) which was regarded as 1. siRNA (1-fU) serves as a positive control group and siRNA (3-Y) serves as a negative control group.

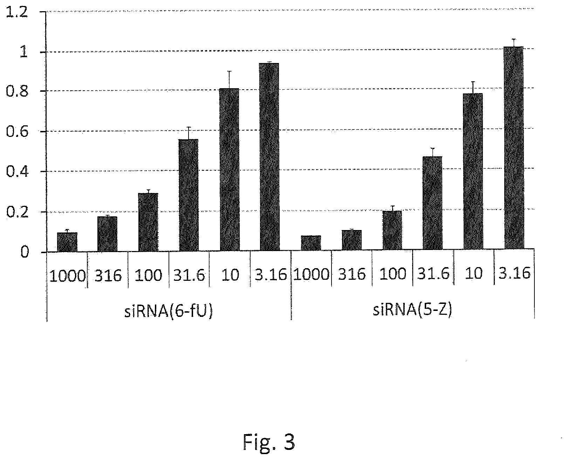

Fig. 3 indicates knockdown activity by HPRT1-targeting cholesterol modified siRNA (6-fU) and siRNA (5-Y) in HeLa cells. The horizontal axis in Fig. 3 indicates the test results of introduction of siRNA (6-fU) and siRNA (5-Y) respectively at concentrations of 1000 nmol/L, 316 nmol/L, 100 nmol/L, 31.6 nmol/L, 10 nmol/L and 3.16 nmol/L into cultured cells. The vertical axis indicates the proportion, expressed as average ± standard deviation, of the HPRT1 mRNA level of the respective siRNA-introduced samples relative to the HPRT1 mRNA level of the non-siRNA-introduced group (negative control group) which was regarded as 1. siRNA (6-fU) serves as a positive control group and siRNA (5-Y) serves as a negative control group.

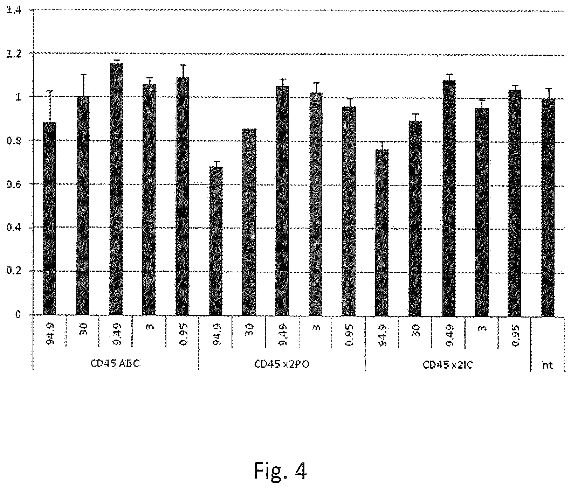

Fig. 4 shows knockdown activity by CD45-targeting ASOs, CD45 ABC, CD45 x2PO and CD45 x2IC, in THP-1 cells. The horizontal axis in Fig. 4 indicates the test results of introduction of CD45 ABC, CD45 x2PO and CD45 x2IC respectively at concentrations of 94.9 nmol/L, 30.0 nmol/L, 9.49 nmol/L, 3.00 nmol/L and 0.95 nmol/L into cultured cells and nt indicates the test result for non-ASO-introduced group (negative control group). The vertical axis indicates the proportion, expressed as average ± standard deviation, of the CD45 mRNA level of the respective ASO-introduced samples relative to the ACTB mRNA amplification level (nt) which was regarded as 1.

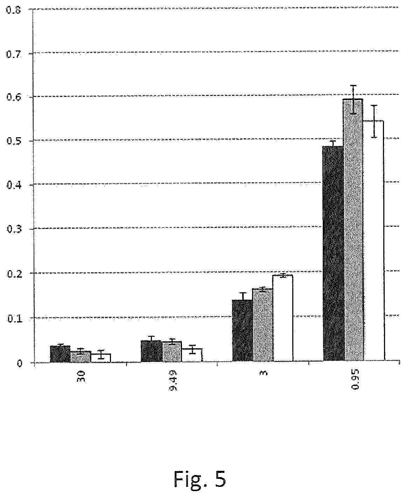

Fig. 5 shows knockdown activity by FGFR4-targeting ASOs, FGFR4 PS (black), FGFR PO (grey) and FGFR4 IC (white), in HepG2 cells. The horizontal axis in Fig. 5 indicates the test results of introduction of FGFR4 PS, FGFR PO and FGFR4 IC respectively at concentrations of 30.0 nmol/L, 9.49 nmol/L, 3.00 nmol/L and 0.95 nmol/L into cultured cells. The vertical axis indicates the proportion, expressed as average ± standard deviation, of the FGFR4 mRNA level of the respective ASO-introduced samples relative to the ACTB mRNA amplification level which was regarded as 1.

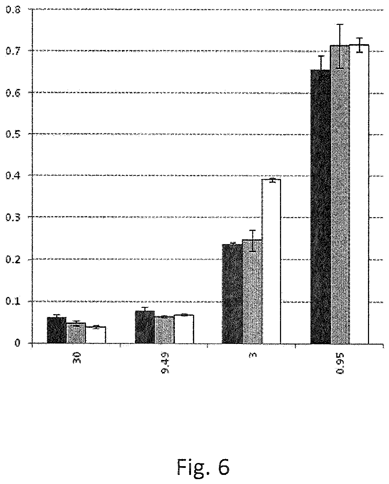

Fig. 6 shows knockdown activity by FGFR4-targeting ASOs, FGFR4 PS (black), FGFR PO (grey) and FGFR4 IC (white), in HuH-7 cells. The horizontal axis in Fig. 6 indicates the test results of introduction of FGFR4 PS, FGFR PO and FGFR4 IC respectively at concentrations of 30.0 nmol/L, 9.49 nmol/L, 3.00 nmol/L and 0.95 nmol/L into cultured cells. The vertical axis indicates the proportion, expressed as average ± standard deviation, of the FGFR4 mRNA level of the respective ASO-introduced samples relative to the ACTB mRNA amplification level which was regarded as 1.

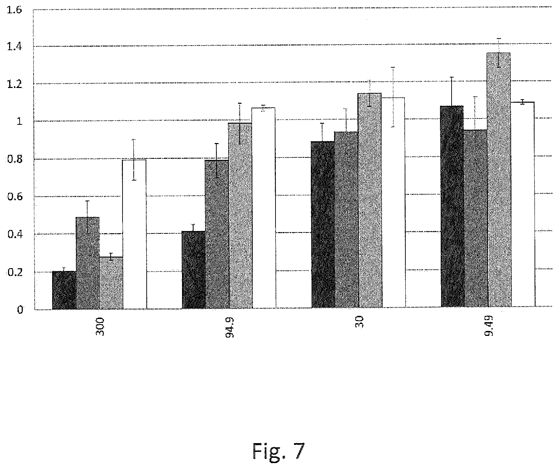

Fig. 7 shows knockdown activity by Phosphatase and Tensin Homolog Deleted from Chromosome 10 (PTEN)-targeting ASOs, KON708 (black), wt KON708 (dark grey), KON715 (light grey) and wt KON715 (white), in HeLa cells. The horizontal axis in Fig. 7 indicates the test results of introduction of KON708, wt KON708, KON715 and wt KON715 respectively at concentrations of 300 nmol/L, 94.9 nmol/L, 30.0 nmol/L and 9.49 nmol/L into cultured cells. The vertical axis indicates the proportion, expressed as average ± standard deviation, of the PTEN mRNA level of the respective ASO-introduced samples relative to the ACTB mRNA amplification level which was regarded as 1.

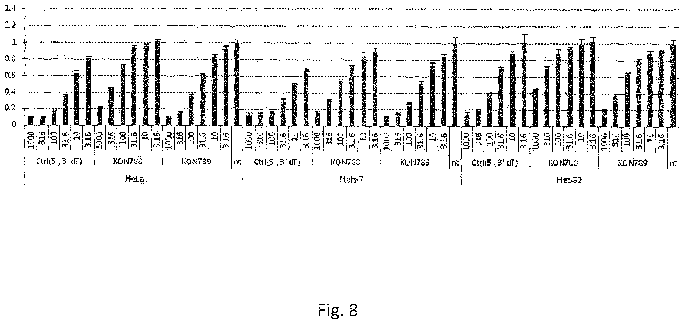

Fig. 8 shows knockdown activity by HPRT1-targeting siRNAs, Ctrl (5', 3' dT), KON788 and KON789, in HeLa cells, HuH-7 cells and HepG2 cells. The horizontal axis in Fig. 8 indicates the test results of introduction of Ctrl (5', 3' dT), KON788 and KON789 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

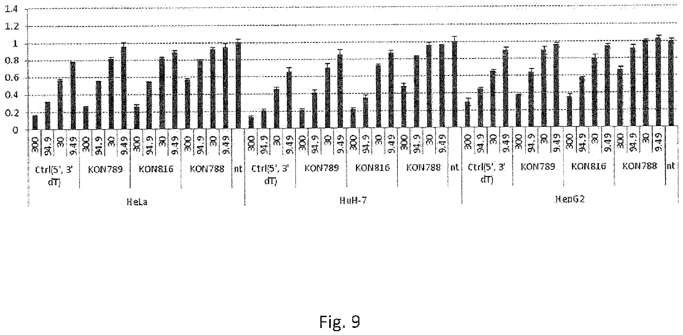

Fig. 9 shows knockdown activity by HPRT1-targeting siRNAs, Ctrl (5', 3' dT), KON789, KON816 and KON788, in HeLa cells, HuH-7 cells and HepG2 cells. The horizontal axis in Fig. 9 indicates the test results of introduction of Ctrl (5', 3' dT), KON789, KON816 and KON788 respectively at concentrations of 300 pmol/L, 94.9 pmol/L, 30 pmol/L and 9.49 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

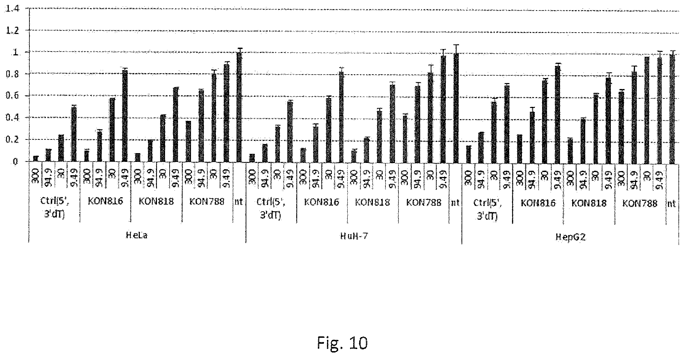

Fig. 10 shows knockdown activity by HPRT1-targeting siRNAs, Ctrl (5', 3' dT), KON816, KON818 and KON788, in HeLa cells, HuH-7 cells and HepG2 cells. The horizontal axis in Fig. 10 indicates the test results of introduction of Ctrl (5', 3' dT), KON816, KON818 and KON788 respectively at concentrations of 300 pmol/L, 94.9 pmol/L, 30 pmol/L and 9.49 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

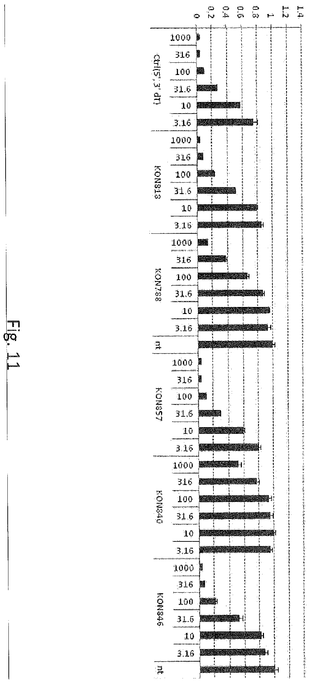

Fig. 11 shows knockdown activity by HPRT1-targeting siRNAs, Ctrl (5', 3' dT), KON818, KON788, KON857, KON840 and KON846, in HeLa cells. The horizontal axis in Fig. 11 indicates the test results of introduction of Ctrl (5', 3' dT), KON818, KON788, KON857, KON840 and KON846 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

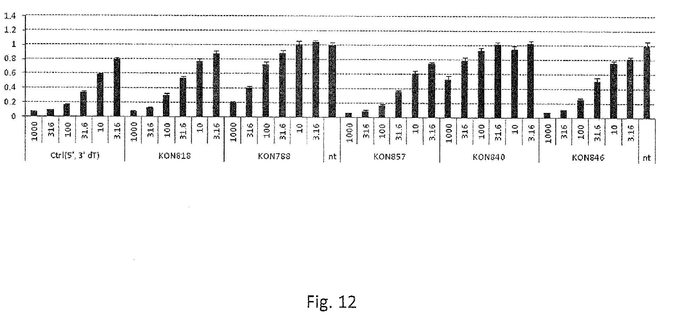

Fig. 12 shows knockdown activity by HPRT1-targeting siRNAs, Ctrl (5', 3' dT), KON818, KON788, KON857, KON840 and KON846, in HuH-7 cells. The horizontal axis in Fig. 12 indicates the test results of introduction of Ctrl (5', 3' dT), KON818, KON788, KON857, KON840 and KON846 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

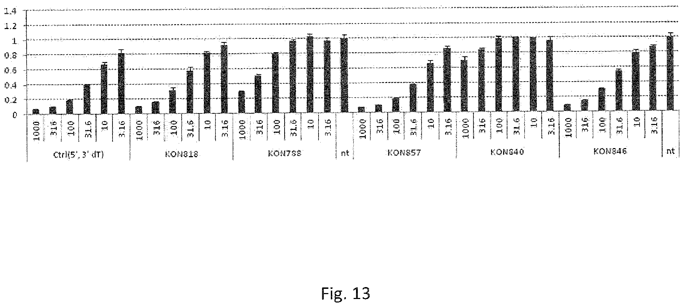

Fig. 13 shows knockdown activity by HPRT1-targeting siRNAs, Ctrl (5', 3' dT), KON818, KON788, KON857, KON840 and KON846, in HepG2 cells. The horizontal axis in Fig. 13 indicates the test results of introduction of Ctrl (5', 3' dT), KON818, KON788, KON857, KON840 and KON846 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

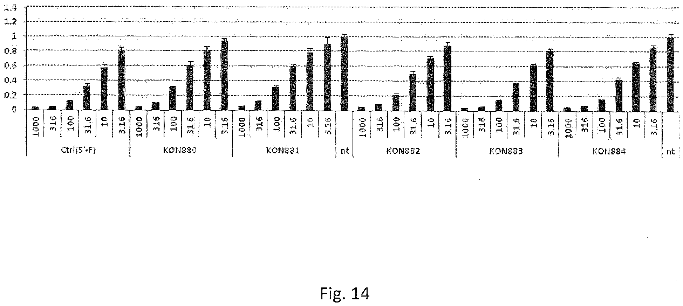

Fig. 14 shows knockdown activity by HPRT1-targeting siRNAs, Ctrl (5'-F), KON880, KON881, KON882, KON883 and KON884, in HeLa cells. The horizontal axis in Fig. 14 indicates the test results of introduction of Ctrl (5'-F), KON880, KON881, KON882, KON883 and KON884 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5'-F) serves as a positive control group.

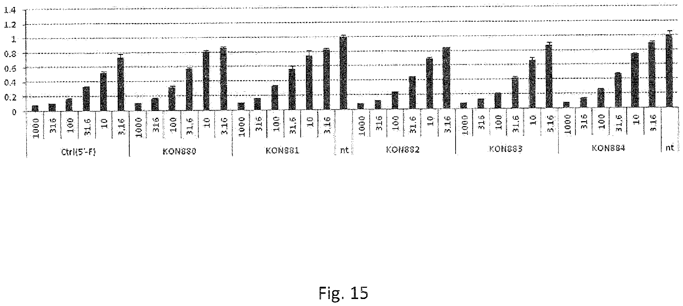

Fig. 15 shows knockdown activity by HPRT1-targeting siRNAs, Ctrl (5'-F), KON880, KON881, KON882, KON883 and KON884, in HuH-7 cells. The horizontal axis in Fig. 15 indicates the test results of introduction of Ctrl (5'-F), KON880, KON881, KON882, KON883 and KON884 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5'-F) serves as a positive control group.

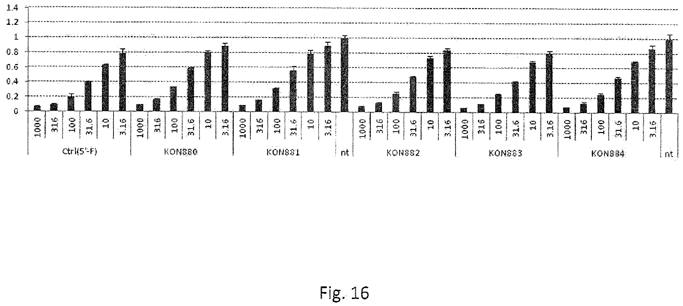

Fig. 16 shows knockdown activity by HPRT1-targeting siRNAs, Ctrl (5'-F), KON880, KON881, KON882, KON883 and KON884, in HepG2 cells. The horizontal axis in Fig. 16 indicates the test results of introduction of Ctrl (5'-F), KON880, KON881, KON882, KON883 and KON884 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5'-F) serves as a positive control group.

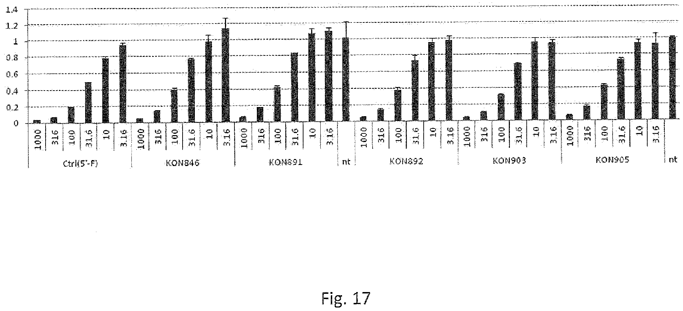

Fig. 17 shows knockdown activity by HPRT1-targeting siRNAs, Ctrl (5'-F), KON846, KON891, KON892, KON903 and KON905, in HeLa cells. The horizontal axis in Fig. 17 indicates the test results of introduction of Ctrl (5'-F), KON846, KON891, KON892, KON903 and KON905 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5'-F) serves as a positive control group.

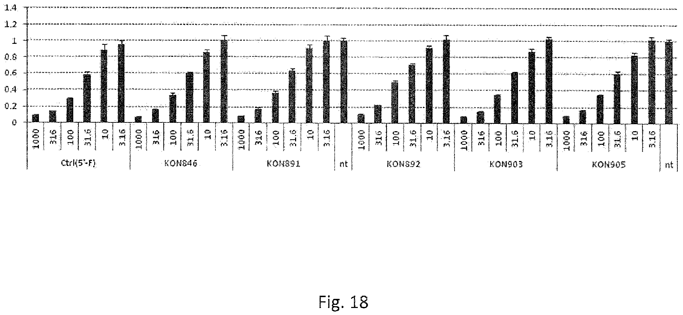

Fig. 18 shows knockdown activity by HPRT1-targeting siRNAs, Ctrl (5'-F), KON846, KON891, KON892, KON903 and KON905, in HuH-7 cells. The horizontal axis in Fig. 18 indicates the test results of introduction of Ctrl (5'-F), KON846, KON891, KON892, KON903 and KON905 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5'-F) serves as a positive control group.

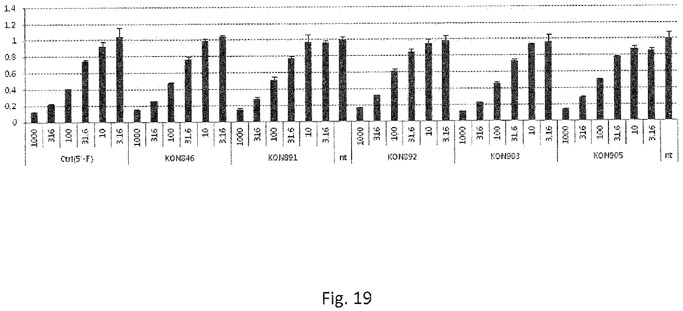

Fig. 19 shows knockdown activity by HPRT1-targeting siRNAs, Ctrl (5'-F), KON846, KON891, KON892, KON903 and KON905, in HepG2 cells. The horizontal axis in Fig. 19 indicates the test results of introduction of Ctrl (5'-F), KON846, KON891, KON892, KON903 and KON905 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5'-F) serves as a positive control group.

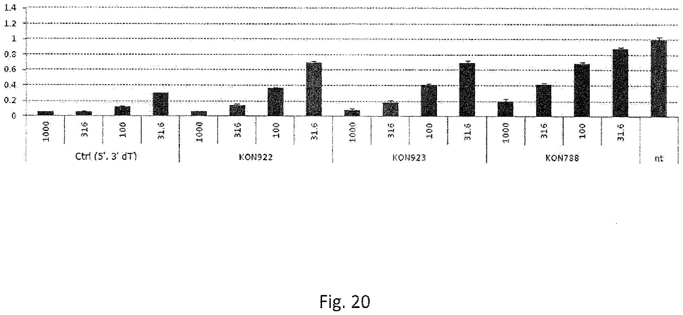

Fig. 20 shows knockdown activity by HPRT1-targeting siRNAs, Ctrl (5', 3' dT), KON922, KON923 and KON788, in HeLa cells. The horizontal axis in Fig. 20 indicates the test results of introduction of Ctrl (5', 3' dT), KON922, KON923 and KON788 respectively at concentrations of 1000 pmol/L, 316 pmol/L, 100 pmol/L and 31.6 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

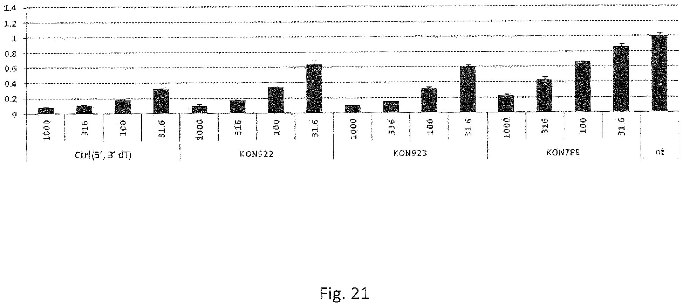

Fig. 21 shows knockdown activity by HPRT1-targeting siRNAs, Ctrl (5', 3' dT), KON922, KON923 and KON788, in HuH-7 cells. The horizontal axis in Fig. 21 indicates the test results of introduction of Ctrl (5', 3' dT), KON922, KON923 and KON788 respectively at concentrations of 1000 pmol/L, 316 pmol/L, 100 pmol/L and 31.6 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

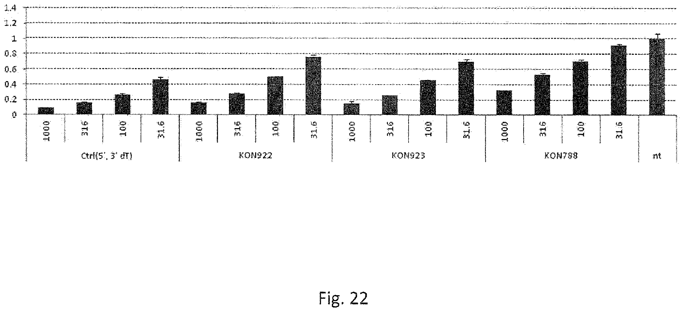

Fig. 22 shows knockdown activity by HPRT1-targeting siRNAs, Ctrl (5', 3' dT), KON922, KON923 and KON788, in HepG2 cells. The horizontal axis in Fig. 22 indicates the test results of introduction of Ctrl (5', 3' dT), KON922, KON923 and KON788 respectively at concentrations of 1000 pmol/L, 316 pmol/L, 100 pmol/L and 31.6 pmol/L into cultured cells and nt indicates the test result for non-siRNA-introduced group (negative control group). The vertical axis indicates the proportion, expressed as average ± standard deviation, of the HPRT1 mRNA level of the respective siRNA-introduced samples relative to the HPRT1 mRNA level of the non-siRNA-introduced group (negative control group: nt) which was regarded as 1. Ctrl (5', 3' dT) serves as a positive control group and KON788 serves as a siRNA-introduced group as well as a negative control group.

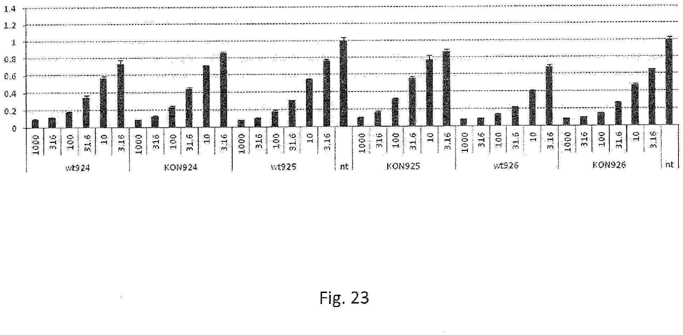

Fig. 23 shows knockdown activity by apolipoprotein B (ApoB)-targeting siRNAs, wt924, KON924, wt925, KON925, wt926 and KON926, in HepG2 cells. The horizontal axis in Fig. 23 indicates the test results of introduction of wt924, KON924, wt925, KON925, wt926 and KON926 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the ApoB mRNA level of the respective siRNA-introduced samples relative to the ApoB mRNA level of the non-siRNA-introduced group (negative control group: nt) which was regarded as 1.

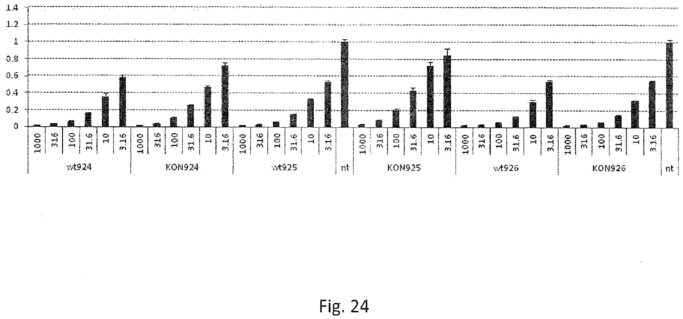

Fig. 24 shows knockdown activity by ApoB-targeting siRNAs, wt924, KON924, wt925, KON925, wt926 and KON926, in HuH-7 cells. The horizontal axis in Fig. 24 indicates the test results of introduction of wt924, KON924, wt925, KON925, wt926 and KON926 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the ApoB mRNA level of the respective siRNA-introduced samples relative to the ApoB mRNA level of the non-siRNA-introduced group (negative control group: nt) which was regarded as 1.

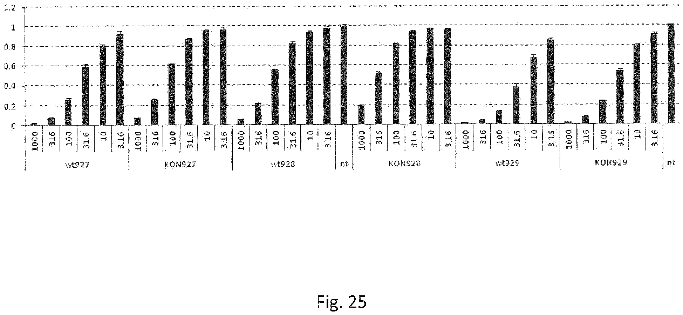

Fig. 25 shows knockdown activity by luciferase-targeting siRNAs, wt927, KON927, wt928, KON928, wt929 and KON929, in luciferase expressing HeLa cells (HeLa-Luc) into which a luciferase expression vector was introduced. The horizontal axis in Fig. 25 indicates the test results of introduction of wt927, KON927, wt928, KON928, wt929 and KON929 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the luminescence (cps) of the respective siRNA-introduced samples relative to the luminescence (cps) of the non-siRNA-introduced group (negative control group: nt) which was regarded as 1.

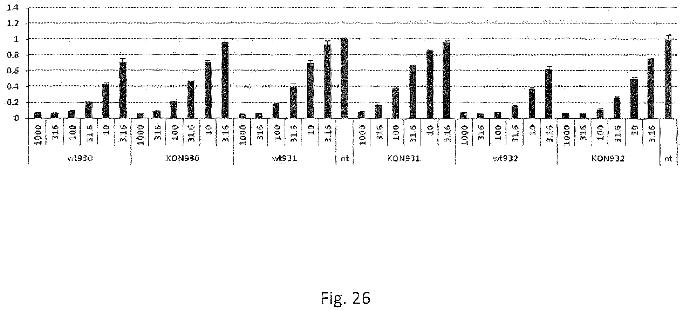

Fig. 26 shows knockdown activity by GAPDH-targeting siRNAs, wt930, KON930, wt931, KON931, wt932 and KON932, in HeLa cells. The horizontal axis in Fig. 26 indicates the test results of introduction of wt930, KON930, wt931, KON931, wt932 and KON932 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the GAPDH mRNA level of the respective siRNA-introduced samples relative to the GAPDH mRNA level of the non-siRNA-introduced group (negative control group: nt) which was regarded as 1.

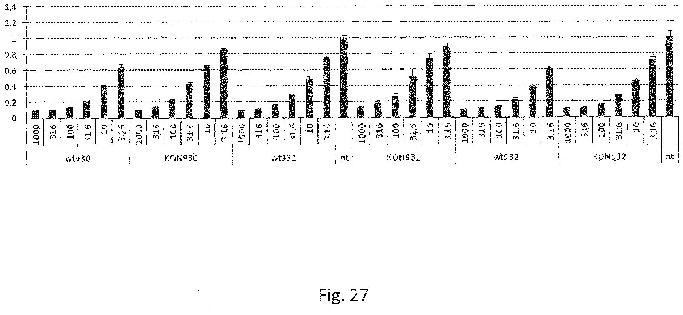

Fig. 27 shows knockdown activity by GAPDH-targeting siRNAs, wt930, KON930, wt931, KON931, wt932 and KON932, in HuH-7 cells. The horizontal axis in Fig. 27 indicates the test results of introduction of wt930, KON930, wt931, KON931, wt932 and KON932 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the GAPDH mRNA level of the respective siRNA-introduced samples relative to the GAPDH mRNA level of the non-siRNA-introduced group (negative control group: nt) which was regarded as 1.

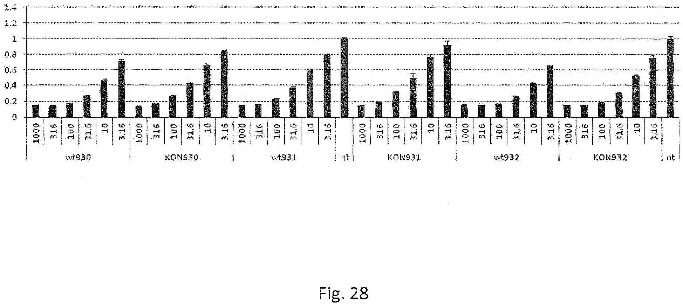

Fig. 28 shows knockdown activity by GAPDH-targeting siRNAs, wt930, KON930, wt931, KON931, wt932 and KON932, in HepG2 cells. The horizontal axis in Fig. 28 indicates the test results of introduction of wt930, KON930, wt931, KON931, wt932 and KON932 respectively at concentrations of 1000 pmol/L, 316 pmol/L, 100 pmol/L, 31.6 pmol/L, 10 pmol/L and 3.16 pmol/L into cultured cells and nt indicates the test result for non-siRNA-introduced group (negative control group). The vertical axis indicates the proportion, expressed as average ± standard deviation, of the GAPDH mRNA level of the respective siRNA-introduced samples relative to the GAPDH mRNA level of the non-siRNA-introduced group (negative control group: nt) which was regarded as 1.

### Description of Embodiments

The oligonucleotide derivatives having the group represented by formula (I) and formula (I-0) at an oxygen atom of at least one phosphate group of an oligonucleotide are hereinafter referred to as compound (I) and compound (I-0), respectively; the oligonucleotide derivatives having one or more structures represented by formula (III), formula (III-0) and formula (III-1) are referred to as compound (III), compound (III-0) and compound (III-1), respectively; and the compounds represented by formula (IV), formula (IV-0), formula (IV-1), formula (V), formula (V-0), formula (V-1), formula (V-2), formula (VI) and formula (VI-0) are referred to as compound (IV), compound (IV-0), compound (IV-1), compound (V), compound (V-0), compound (V-1), compound (V-2), compound (VI) and compound (VI-0), respectively.

The definitions of the groups used herein are as described hereinbelow.
(i) Examples of lower alkyl and a lower alkyl moiety in lower alkoxy, lower alkoxycarbonyl, lower alkylsulfonyl and lower alkylthio include linear or branched C₁₋₁₂ alkyl. Specific examples thereof include methyl, ethyl, propyl, isopropyl, butyl, isobutyl, sec-butyl, tert-butyl, pentyl, isopentyl, neopentyl, hexyl, heptyl, octyl, nonyl, decyl, undecyl and dodecyl.
(ii) Examples of lower alkanoyl include linear or branched C₁₋₁₂ alkanoyl. Specific examples thereof include formyl, acetyl, propionyl, butyryl, isobutyryl, valeryl, isovaleryl, pivaloyl, hexanoyl, heptanoyl, octanoyl, nonanoyl, decanoyl, undecanoyl and dodecanoyl.
(iii) Examples of lower alkenyl and a lower alkenyl moiety in lower alkenyloxycarbonyl and lower alkenylthio include linear or branched C₂₋₁₂ alkenyl. Specific examples thereof include vinyl, allyl, 1-propenyl, butenyl, pentenyl, hexenyl, heptenyl, octenyl, nonenyl, decenyl, undecenyl, and dodecenyl.
(iv) Examples of lower alkynyl and a lower alkynyl moiety in lower alkynyloxycarbonyl and lower alkynylthio include linear or branched C₂₋₁₂ alkynyl. Specific examples thereof include ethynyl, 1-propynyl, propargyl, butynyl, pentynyl, hexynyl, heptynyl, octynyl, nonynyl, decynyl, undecynyl and dodecynyl.
(v) Examples of the aralkyl moiety in aralkyloxycarbonyl include C₇₋₁₆ aralkyl, and specific examples thereof include benzyl, phenethyl, phenylpropyl, phenylbutyl, phenylpentyl, phenylhexyl, phenylheptyl, phenyloctyl, phenylnonyl, phenyldecyl, naphthylmethyl, naphthylethyl, naphthylpropyl, naphthylbutyl, naphthylpentyl, naphthylhexyl, anthrylmethyl and anthrylethyl.
(vi) Examples of the aryl moiety in arylsulfonyl include C₆₋₁₄ aryl, and specific examples thereof include phenyl, naphthyl, azulenyl and anthryl.
(vii) Examples of the electron-withdrawing group include carboxy, cyano, nitro, lower alkanoyl, lower alkoxycarbonyl, carbamoyl, lower alkylcarbamoyl, di-lower alkylcarbamoyl, lower alkylsulfinyl, lower alkylsulfonyl, sulfamoyl, lower alkylsulfamoyl and di-lower alkylsulfamoyl.
(viii) Examples of the protecting group of a hydroxy group include trityl, 4-methoxytrityl and 4,4'-dimethoxytrityl.

Lower alkanoyl and lower alkoxycarbonyl exemplified as electron-withdrawing groups have the same meanings as the above (ii) lower alkanoyl and (i) lower alkoxycarbonyl, respectively, and examples of the lower alkyl moiety in lower alkylcarbamoyl, di-lower alkylcarbamoyl, lower alkylsulfinyl, lower alkylsulfonyl, lower alkylsulfamoyl and di-lower alkylsulfamoyl include the groups exemplified for the above (i) lower alkyl. Two lower alkyl moieties in di-lower alkylcarbamoyl and di-lower alkylsulfamoyl may be the same or different.

The substituents of optionally substituted lower alkyl, optionally substituted lower alkenyl, optionally substituted lower alkynyl, optionally substituted lower alkylthio, optionally substituted lower alkenylthio and optionally substituted lower alkynylthio are the same or different and the number of the substituent is, for example, 1 to 3, and examples of the substituents include substituents selected from the group consisting of halogen, hydroxy, sulfanyl, nitro, cyano, azido, carboxy, carbamoyl, C₃₋₁₀ cycloalkyl, C₆₋₁₄ aryl, an aliphatic heterocyclic group, an aromatic heterocyclic group, optionally substituted C₁₋₁₂ alkoxy [the substituents of optionally substituted C₁₋₁₂ alkoxy are the same or different and the number of the substituent is 1 to 3, and examples of the substituent include optionally substituted C₆₋₁₄ aryl (examples of the substituent of optionally substituted C₆₋₁₄ aryl include C₁₋₁₂ alkoxy)], C₃₋₁₀ cycloalkoxy, C₆₋₁₄ aryloxy, C₇₋₁₆ aralkyloxy, C₁₋₁₂ alkanoyloxy, C₇₋₁₅ aroyloxy, C₁₋₁₂ alkylsulfanyl, -NR^{X}R^{Y} (wherein R^{X} and R^{Y} are the same or different and respectively represent hydrogen atom, C₁₋₁₂ alkyl, C₃₋₁₀ cycloalkyl, C₆₋₁₄ aryl, an aromatic heterocyclic group, C₇₋₁₆ aralkyl, C₁₋₁₂ alkanoyl, C₇₋₁₅ aroyl, C₁₋₁₂ alkoxycarbonyl or C₇₋₁₆ aralkyloxycarbonyl), C₁₋₁₂ alkanoyl, C₇₋₁₅ aroyl, C₁₋₁₂ alkoxycarbonyl, C₆₋₁₄ aryloxycarbonyl, C₁₋₁₂ alkylcarbamoyl and di-C₁₋₁₂ alkylcarbamoyl.

The substituents of optionally substituted arylsulfonyl and optionally substituted aralkyloxycarbonyl are the same or different and the number of the substituent is, for example, 1 to 3, and examples of the substituents include substituents selected from the group consisting of halogen, hydroxy, sulfanyl, nitro, cyano, carboxy, carbamoyl, C₁₋₁₂ alkyl, C₂₋₁₂ alkenyl, C₂₋₁₂ alkynyl, trifluoromethyl, p-toluenesulfonyloxy, methanesulfonyloxy, trifluoromethanesulfonyloxy, C₃₋₁₀ cycloalkyl, C₆₋₁₄ aryl, an aliphatic heterocyclic group, an aromatic heterocyclic group, C₁₋₁₂ alkoxy, C₃₋₁₀ cycloalkoxy, C₆₋₁₄ aryloxy, C₇₋₁₆ aralkyloxy, C₁₋₁₂ alkanoyloxy, C₇₋₁₅ aroyloxy, C₁₋₁₂ alkylsulfanyl, -NR^{Xa}R^{Ya} (wherein R^{Xa} and R^{Ya} are the same or different and respectively represent hydrogen atom, C₁₋₁₂ alkyl, C₃₋₁₀ cycloalkyl, C₆₋₁₄ aryl, an aromatic heterocyclic group, C₇₋₁₆ aralkyl, C₁₋₁₂ alkanoyl, C₇₋₁₅ aroyl, C₁₋₁₂ alkoxycarbonyl or C₇₋₁₆ aralkyloxycarbonyl), C₁₋₁₂ alkanoyl, C₇₋₁₅ aroyl, C₁₋₁₂ alkoxycarbonyl, C₆₋₁₄ aryloxycarbonyl, C₁₋₁₂ alkylcarbamoyl and di-C₁₋₁₂ alkylcarbamoyl.

Examples of C₁₋₁₂ alkyl and a C₁₋₁₂ alkyl moiety in C₁₋₁₂ alkoxy, C₁₋₁₂ alkanoyloxy, C₁₋₁₂ alkylsulfanyl, C₁₋₁₂ alkoxycarbonyl, C₁₋₁₂ alkylcarbamoyl and di-C₁₋₁₂ alkylcarbamoyl include the groups exemplified for the above (i) lower alkyl. Two C₁₋₁₂ alkyl moieties in di-C₁₋₁₂ alkylcarbamoyl may be the same or different.

Examples of C₂₋₁₂ alkenyl include the groups exemplified for the above (iii) lower alkenyl.

Examples of C₂₋₁₂ alkynyl include the groups exemplified for the above (iv) lower alkynyl.

Examples of C₁₋₁₂ alkanoyl include the groups exemplified for the above (ii) lower alkanoyl.

Examples of C₃₋₁₀ cycloalkyl and a cycloalkyl moiety in C₃₋₁₀ cycloalkoxy include C₃₋₁₀ cycloalkyl, and specific examples thereof include cyclopropyl, cyclobutyl, cyclopentyl, cyclohexyl, cycloheptyl, cyclooctyl, cyclononyl and cyclodecanyl.

Examples of C₆₋₁₄ aryl and an aryl moiety in C₆₋₁₄ aryloxy, C₇₋₁₅ aroyl, C₇₋₁₅ aroyloxy and C₆₋₁₄ aryloxycarbonyl include the groups exemplified for the aryl moiety in the above (vi) arylsulfonyl.

Examples of the aryl moiety in C₇₋₁₆ aralkyloxy, C₇₋₁₆ aralkyl and C₇₋₁₆ aralkyloxycarbonyl include the groups exemplified for the aryl moiety in the above (vi) arylsulfonyl, and examples of the alkyl moiety include C₁₋₁₂ alkylene, and specific examples thereof include groups exemplified for the above (i) lower alkyl from which one hydrogen atom has been removed.

Examples of the aliphatic heterocyclic group include a 5-membered or 6-membered monocyclic aliphatic heterocyclic group containing at least one atom selected from nitrogen atom, oxygen atom and sulfur atom, a bicyclic or tricyclic condensed aliphatic heterocyclic group which is obtained by condensation of 3- to 8-membered rings and which contains at least one atom selected from nitrogen atom, oxygen atom and sulfur atom. Specific examples thereof include aziridinyl, azetidinyl, pyrrolidinyl, piperidino, piperidinyl, azepanyl, 1,2,5,6-tetrahydropyridyl, imidazolidinyl, pyrazolidinyl, piperazinyl, homopiperazinyl, pyrazolinyl, oxylanyl, tetrahydrofuranyl, tetrahydro-2H-pyranyl, 5,6-dihydro-2H-pyranyl, oxazolidinyl, morpholino, morpholinyl, thioxazolidinyl, thiomorpholinyl, 2H-oxazolyl, 2H-thioxazolyl, dihydroindolyl, dihydroisoindolyl, dihydrobenzofuranyl, benzimidazolinyl, dihydrobenzoxazolyl, dihydrobenzothioxazolyl, benzodioxolyl, tetrahydroquinolyl, tetrahydroisoquinolyl, dihydro-2H-chromanyl, dihydro-1H-chromanyl, dihydro-2H-thiochromanyl, dihydro-1H-thiochromanyl, tetrahydroquinoxalinyl, tetrahydroquinazolinyl and benzodioxanyl.

Examples of the aromatic heterocyclic group include a 5-membered or 6-membered monocyclic aromatic heterocyclic group containing at least one atom selected from nitrogen atom, oxygen atom and sulfur atom, a bicyclic or tricyclic condensed aromatic heterocyclic group which is obtained by condensation of 3- to 8-membered rings and which contains at least one atom selected from nitrogen atom, oxygen atom and sulfur atom. Specific examples thereof include furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyridazinyl, pyrimidinyl, pyrazinyl, triazinyl, benzofuranyl, benzothiophenyl, benzoxazolyl, benzothiazolyl, isoindolyl, indolyl, indazolyl, benzimidazolyl, benzotriazolyl, oxazolopyrimidinyl, thiazolopyrimidinyl, pyrrolopyridinyl, pyrrolopyrimidinyl, imidazopyridinyl, purinyl, quinolinyl, isoquinolinyl, cinnolinyl, phthalazinyl, quinazolinyl, quinoxalinyl and naphthylizinyl.

The term halogen means any atom of fluorine, chlorine, bromine and iodine.

The oligonucleotide derivative of the present invention means a polymer or oligomer formed from nucleotide residues.

The oligonucleotide derivative of the present invention encompasses both a single-stranded oligonucleotide and a double-stranded oligonucleotide. In a double-stranded oligonucleotide, each oligonucleotide strands may have different base lengths. A double-stranded oligonucleotide may contain one or more mismatch base pairs. The oligonucleotide derivative of the present invention further encompasses a complex formed with three or more oligonucleotide strands.

The oligonucleotide derivative of the present invention preferably has knockdown activity against a mRNA encoding, for example, a protein involved in a disease. The terms "have knockdown activity" as used herein means to suppress expression of a gene (target gene) encoding a protein or the like.

Examples of the double-stranded oligonucleotide include double-stranded DNAs such as structural genes and double-stranded RNAs such as small molecule RNAs typically including siRNAs and miRNAs; the present invention, however, is not limited thereto.

Examples of the single-stranded oligonucleotide include antisense oligonucleotides, micro RNAs, aptamers, antagomers, single-stranded RNAi agents (siRNAs and the like having a hairpin structure); the present invention, however, is not limited thereto.

The oligonucleotide derivative of the present invention has preferably a length of 5 to 100 bases, more preferably 10 to 80 bases, still more preferably 10 to 50 bases, particularly preferably 20 to 50 bases and most preferably 20 to 30 bases.

The oligonucleotide derivative of the present invention, which have the group represented by formula (I) or formula (I-0) at an oxygen atom of at least one phosphate group, may further be modified at one or more nucleotide residues. The modification may be included at any site of nucleobase, saccharide and phosphate.

Examples of the nucleobase include adenine, guanine, cytosine, thymine and uracil, which may optionally be protected with one or more protecting groups. When two or more protecting groups exist, the protecting groups may be the same or different. Examples of the protecting group include allyloxycarbonyl, benzoyl, benzyl, acetyl, phenoxyacetyl and tert-butylphenoxyacetyl for amino at the 4-position of cytosine or amino at the 6-position of adenine, and isobutyryl, allyloxycarbonyl, phenoxyacetyl and tert-butylphenoxyacetyl for amino at the 2-position of guanine.

Examples of the nucleotide including the modification at a nucleobase site include a nucleotide having the nucleobase and a nucleotide in which a partial or whole chemical structure of the nucleobase (examples of the nucleobase include adenine, guanine, cytosine, thymine and uracil) thereof is substituted with an atom. Specific examples thereof include a nucleotide in which an oxygen atom of the nucleobase (examples of the nucleobase include adenine, guanine, cytosine, thymine and uracil) is substituted with a sulfur atom, a nucleotide in which a hydrogen atom is substituted with C₁₋₁₀ alkyl, a nucleotide in which methyl is substituted with a hydrogen atom or C₂₋₁₀ alkyl, a nucleotide in which amino is protected with a protecting group such as C₁₋₁₀ alkyl and C₁₋₈ alkanoyl.

The nucleotide including the modification at a saccharide site may be any nucleotide as far as it is a nucleotide in which a partial or whole chemical structure of the saccharide is modified with a substituent or substituted with an atom. For example, a LNA (Locked Nucleic Acid) which represents a bicyclic ribonucleic acid in which the oxygen atom at the 2'-position and the carbon atom at the 4'-position of a ribose ring are cross-linked via methylene and a 2'-modified nucleotide, among which a 2'-modified nucleoside is preferably used.

Examples of the 2'-modified nucleotide include a 2'-modified nucleotide in which 2'-OH of ribose is substituted with a substituent selected from the group consisting of hydrogen atom, -OR, -R, -SH, -SR, amino, -NHR, -NR₂, N₃ (azido), cyano and halogen (wherein R is lower alkyl or aryl; halogen means any atom of fluorine, chlorine, bromine and iodine; lower alkyl and aryl are as defined above; and two R in -NR₂ may be the same or different). Specific examples thereof include a 2'-modified nucleotide in which 2'-OH is substituted with a substituent selected from the group consisting of fluorine atom, methoxy, 2-(methoxy)ethoxy, 3-aminopropoxy, 2-[(N,N-dimethylamino)oxy]ethoxy, 3-(N,N-dimethylamino)propoxy, 2-[2-(N,N-dimethylamino)ethoxy]ethoxy, 2-(methylamino)-2-oxoethoxy, 2-(N-methylcarbamoyl)ethoxy and 2-cyanoethoxy.

The nucleotide including the modification at a phosphate site may be any nucleotide as far as it is a nucleotide in which a partial or whole chemical structure of the phosphodiester bond of the oligonucleotide is modified with a substituent other than formula (I) or substituted with an atom. Examples thereof include a nucleotide in which a phosphodiester bond is substituted with an alkylphosphonate bond, a nucleotide having a phosphorothioate with the structure in which some or all oxygen atoms in a phosphate group are substituted with a sulfur atom.

The oligonucleotide derivative having knockdown activity against a mRNA encoding a protein involved in a disease may be any nucleic acid such as double-stranded oligonucleotides including siRNA (short interference RNA) and miRNA (micro RNA), single-stranded oligonucleotides including shRNA (short hairpin RNA), antisense nucleic acid and ribozyme as far as it contains a base sequence complementary to a partial base sequence of the mRNA of the gene (target gene) encoding a protein or the like and suppresses expression of the target gene. However, a double-stranded oligonucleotide is preferred.

An oligonucleotide strand containing a base sequence complementary to a partial base sequence of mRNA of the target gene is referred to as an antisense strand, and an oligonucleotide containing a base sequence complementary to the base sequence of the antisense strand is referred to as sense strand. A sense strand refers to an oligonucleotide that can form a pair with an antisense strand to form a double-stranded part, such as an oligonucleotide per se consisting of a partial base sequence of the target gene. In a double-stranded oligonucleotide containing a base sequence complementary to a partial base sequence of mRNA of the target gene, 5'-terminal of the oligonucleotide means 5'-terminal of the antisense strand.

In the oligonucleotide derivative of the present invention, a double-stranded part formed by pairing of an antisense strand and a sense strand with bases usually have 15 to 27 base pairs, preferably 15 to 25 base pairs, more preferably 15 to 23 base pairs, still more preferably 15 to 21 base pairs and particularly preferably 15 to 19 base pairs. In the double-stranded part, bases in the antisense strand and the sense strand may oppose each other and may form base pairs or may be mismatched. It is preferable that the base at 5'-terminal of the antisense strand and the base in the sense strand opposing thereto are a base pair of adenine-uracil or are mismatched.

Either or both antisense strand oligonucleotide and sense strand oligonucleotide that form a double-stranded oligonucleotide may have an additional nucleotide at 3' or 5' to the double-stranded part that does not form the double strand. The part that does not form the double strand may be referred to as an overhang.

The double-stranded oligonucleotide having an overhang may be, for example, one having an overhang of 1 to 4 nucleotides, usually 1 to 3 nucleotides at 3'-terminal or 5'-terminal of at least one strand, preferably is one having an overhang of 2 nucleotides and more preferably one having an overhang of dTdT or UU. An overhang may be present only in the antisense strand, only in the sense strand or in both antisense strand and sense strand. A double-stranded nucleic acid which contains an antisense strand and a sense strand both having overhangs is preferably used.

The double-stranded part may be followed by a sequence partially or entirely identical to the base sequence of mRNA of the target gene or the double-stranded part may be followed by a sequence partially or entirely identical to the base sequence of a complementary strand of mRNA of the target gene. Further, the double-stranded oligonucleotide may be a nucleic acid molecule that generates a double-stranded oligonucleotide having an overhang as described above by an effect of ribonuclease such as dicer (WO 2005/089287) or a double-stranded oligonucleotide without an overhang at 3'-terminal or 5'-terminal.

The double-stranded oligonucleotide preferably has an antisense strand in which the sequence of at least 2nd to 17th base (nucleoside) from 5' terminal towards 3' terminal is complementary to the sequence of consecutive 16 bases of mRNA in the target gene, and more preferably an antisense strand in which the sequence of 2nd to 19th base from 5' terminal towards 3' terminal is complementary to the sequence of consecutive 18 bases of mRNA in the target gene, or the sequence of 2nd to 21st base is complementary to the sequence of consecutive 20 bases of mRNA in the target gene, or the sequence of 2nd to 25th base is complementary to the sequence of consecutive 24 bases of mRNA in the target gene.

The base sequence at 5'-terminal of the antisense strand may be complementary or mismatched to the base sequence of mRNA of the target gene.

Examples of salts of compounds (I), (III), (I-0), (III-0), (III-1), (IV), (IV-0), (IV-1), (VI) and (VI-0) include acid addition salts, metal salts, ammonium salts, organic amine addition salts and amino acid addition salts.

Examples of acid addition salts include inorganic acid salts such as hydrochloride, sulphate and phosphate and organic acid salts such as acetate, malate, fumarate, citrate and methanesulphonate. Examples of metal salts include alkali metal salts such as sodium salts and potassium salts; alkaline earth metal salts such as magnesium salts and calcium salts; aluminium salts and zinc salts. Examples of ammonium salts include salts of ammonium and tetramethylammonium. Examples of organic amine addition salts include addition salts of morpholine and piperidine. Examples of amino acid addition salts include addition salts of lysine, glycine and phenylalanine.

Some of compounds (I), (III), (I-0), (III-0), (III-1), (IV), (IV-0), (IV-1), (VI) and (VI-0) may include stereoisomers such as geometric isomers and optical isomers and tautomers. All possible isomers and mixtures thereof including the foregoing may be used for the present invention.

Some or all atoms in compounds (I), (III), (I-0), (III-0), (III-1), (IV), (IV-0), (IV-1), (VI) and (VI-0) may be replaced by isotopes thereof and compounds in which atoms are replaced by isotopes may be used for the present invention. For example, some or all hydrogen atoms in compounds (I), (III), (I-0), (III-0), (III-1), (IV), (IV-0), (IV-1), (VI) and (VI-0) may be a hydrogen atom having the atomic weight of 2 (deuterium).

Compounds (I), (III), (I-0), (III-0), (III-1), (IV), (IV-0), (IV-1), (VI) and (VI-0) in which some or all atoms are replaced by isotopes thereof may be produced according to a method similar to the production method described above using commercially available building blocks. Compounds (I), (III), (I-0), (III-0), (III-1), (IV), (IV-0), (IV-1), (VI) and (VI-0) in which some or all hydrogen atoms are replaced by deuteriums may also be synthesised by, for example, 1) a method which involves deuteration of carboxylic acid and the like using deuterium peroxide under basic conditions (see U.S. Patent No. 3849458 (Specification)); 2) a method which involves deuteration of alcohol, carboxylic acid and the like with a catalyst of an iridium complex and a deuterium source of heavy water [see J. Am. Chem. Soc., Vol. 124, No. 10, 2092 (2002)]; 3) a method which involves deuteration of fatty acid with a catalyst of palladium carbon and a deuterium source of deuterium gas only [see LIPIDS, Vol. 9, No. 11, 913 (1974)]; 4) a method which involves deuteration of acrylic acid, methyl acrylate, methacrylic acid, methyl methacrylate and the like with a catalyst of metal such as platinum, palladium, rhodium, ruthenium and iridium, and a deuterium source of heavy water or heavy water and deuterium gas (see JP Patent Publication No. H05-19536, JP Patent Publication No. S61-277648 and JP Patent Publication No. S61-275241); 5) a method which involves deuteration of acrylic acid, methyl methacrylate and the like with a catalyst of palladium, nickel, copper or copper chromite and a deuterium source of heavy water (see JP Patent Publication No. S63-198638) and the like.

When salts of compounds (I), (III), (I-0), (III-0), (III-1), (IV), (IV-0), (IV-1), (VI) and (VI-0) are sought to be obtained, salts of compounds (I), (III), (I-0), (III-0), (III-1), (IV), (IV-0), (IV-1), (VI) and (VI-0) obtained may be purified, or free forms of compounds (I), (III), (I-0), (III-0), (III-1), (IV), (IV-0), (IV-1), (VI) and (VI-0) obtained may be dissolved or suspended in an appropriate solvent to which an acid or base may be added to form salts which may be isolated and purified.

Compounds (I), (III), (I-0), (III-0), (III-1), (IV), (IV-0), (IV-1), (VI) and (VI-0) may exist in the form of adducts with water or various solvents, which adducts may also be used in the present invention.

Production methods of compounds (I), (III), (I-0), (III-0), (III-1), (IV), (IV-0), (IV-1), (VI) and (VI-0) of the present invention are hereinafter described.

Compounds (I), (I-0), (III) and (III-0) may be produced according to, for example, the production method below.

Compounds (IV) and (IV-0) may be produced as, for example, compound (B-1) in production method 2 or compound (B-2) in production method 4. Compound (IV-1) may be produced as, for example, compound (B-3) in production method 5. Compounds (VI) and (VI-0) may be produced as, for example, compound (R) in production method 2 or compound (X) in production method 4.

### Production method 1

### Production method of oligonucleotide derivative

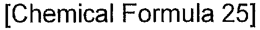

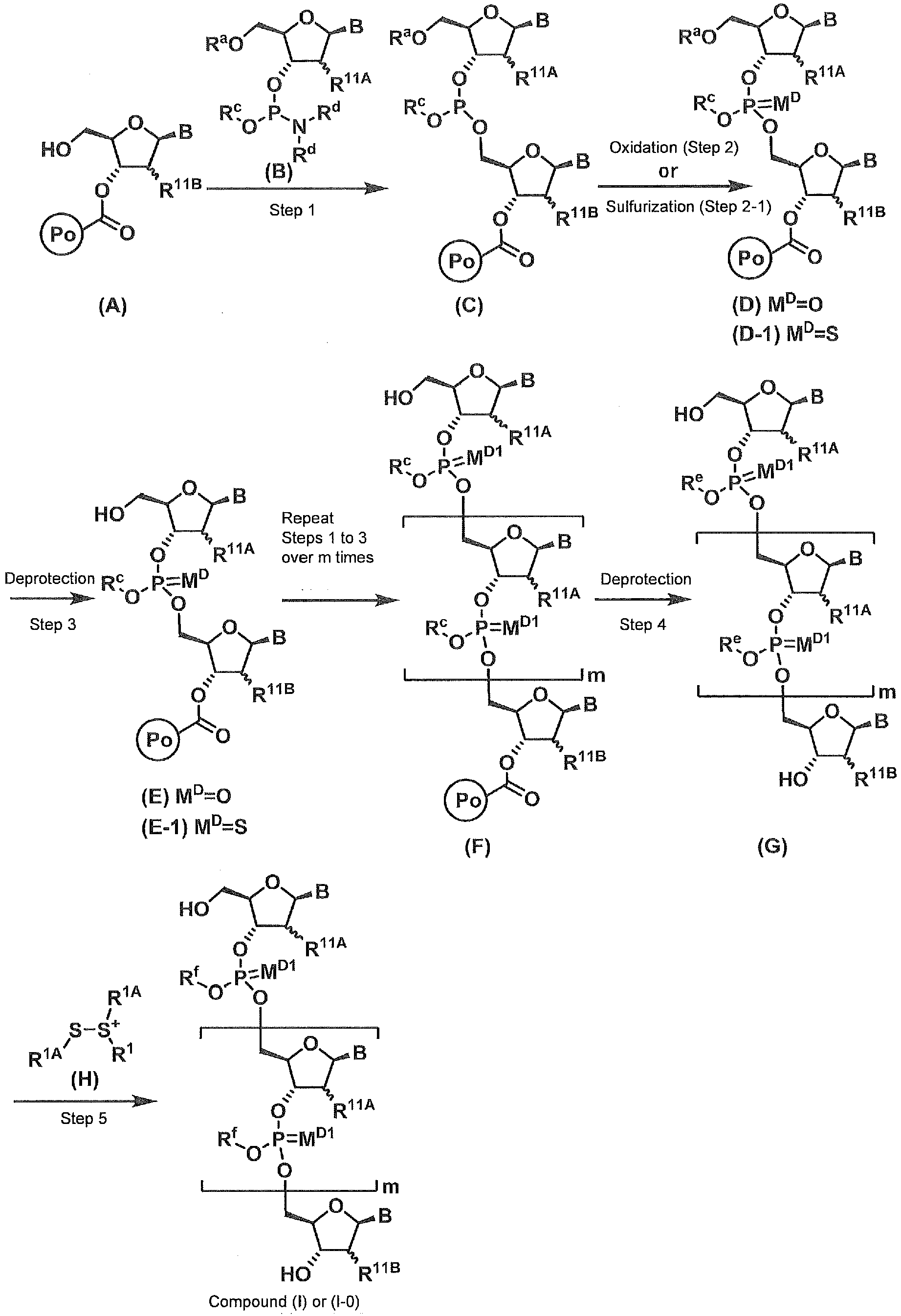

{In the formulae, m represents an integer such as 3 to 99; Po represents a solid support such as CPG (controlled pore glass); R^{a} represents a protecting group that can be eliminated by acid treatment, such as trityl and 4,4'-dimethoxytrityl; R¹ is as defined above; R^{1A} represents lower alkyl; R^{c} represents a protecting group that can be eliminated by base treatment, such as 2-cyanoethyl or a substituent represented by formula (C-1): 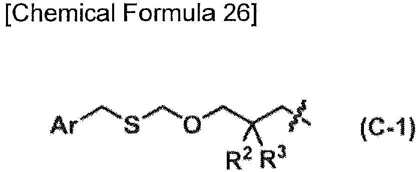 (wherein Ar represents aryl substituted with lower alkyl or aryl substituted with lower alkoxy; R² and R³ are as defined above) or formula (D-1): 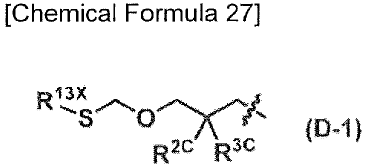 (wherein R^{2C} and R^{3C} are as defined above; and R^{13X} represents optionally substituted lower alkylthio, optionally substituted lower alkenylthio or optionally substituted lower alkynylthio); R^{d} represents lower alkyl; R^{e} represents hydrogen atom or a substituent represented by formula (C-1) [provided that when R^{c} in compound (F) corresponding to R^{e} in compound (G) is a protecting group that can be eliminated by base treatment, such as 2-cyanoethyl, R^{e} represents hydrogen atom, when R^{c} in compound (F) corresponding to R^{e} in compound (G) is a substituent represented by formula (C-1), R^{e} represents a substituent represented by formula (C-1), when R^{c} in compound (F) corresponding to R^{e} in compound (G) is a substituent represented by formula (D-1), R^{e} represents a substituent represented by formula (D-1)]; R^{f} represents hydrogen atom or a substituent represented by formula (I-1): 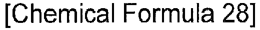 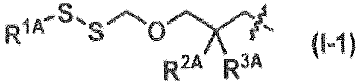 (wherein R^{1A}, R^{2A} and R^{3A} are as defined above) [provided that when R^{e} in compound (G) corresponding to R^{f} in compound (I) is hydrogen atom, R^{f} represents hydrogen atom and when R^{e} in compound (G) corresponding to R^{f} in compound (I) is a substituent represented by formula (C-1), R^{f} represents a substituent represented by formula (I-1)]; B represents a nucleobase;
R^{11A} represents hydrogen atom, hydroxy, fluorine atom, tri-lower alkylsilyl or lower alkoxy [provided that when R^{c} and R^{e} on the phosphate group adjacent to R^{11A} are substituents represented by formula (C-1) or when R^{f} on the phosphate group adjacent to R^{11A} is a substituent represented by formula (I-1), R^{11A} represents hydrogen atom, fluorine atom, tri-lower alkylsilyl or lower alkoxy, and R^{c}, R^{e} or R^{f} on the phosphate group adjacent to R^{11A} is hydrogen atom, R^{11A} represents hydrogen atom, hydroxy, fluorine atom, tri-lower alkylsilyl or lower alkoxy]; R^{11B} represents hydrogen atom, hydroxy, fluorine atom, tri-lower alkylsilyl or lower alkoxy;
M^{D} and M^{D1} are the same or different and respectively represent oxygen atom or sulfur atom;
when m in compounds (F), (G) and (I) is 2 or more, m+1 B's, m+1 R^{11A}'s, m+1 R^{c'}s, m+1 R^{e}'s, m+1 M^{D1}'s and m+1 R^{f}'s may be the same or different and R^{a}'s in the respective steps may be the same or different, wherein the lower alkyl, aryl, lower alkoxy and nucleobase respectively have the same meanings as the lower alkyl, aryl, lower alkoxy and nucleobase described above and three lower alkyl groups in the tri-lower alkylsilyl are the same or different and respectively have the same meaning as the lower alkyl described above.}

### Step 1

Compound (C) may be produced by reacting compound (A) with preferably 5 to 1000 equivalents of compound (B) in a solvent in the presence of preferably 5 to 1000 equivalents of an additive at a temperature between 0°C and a boiling point of the solvent for 10 seconds to 30 minutes.

Examples of the solvent include dichloromethane, acetonitrile, toluene, ethyl acetate, tetrahydrofuran (THF), 1,4-dioxane, N,N-dimethylformamide (DMF), N-methylpyrrolidone (NMP) and water, which may be used alone or as a mixture.

Examples of the additive include 1 H-tetrazole, 4,5-dicyanoimidazole, 5-ethylthiotetrazole and 5-benzylthiotetrazole.

Compound (A) may be, for example, commercially available.

Compound (B) wherein R^{c} is 2-cyanoethyl may be commercially available and compound (B) wherein R^{c} is a substituent represented by formula (C-1) may be obtained as compound (B-1) in the following production method 2.

It is preferable to perform the following step 1-1 after step 1 because an involvement of unreacted compound (A) to the reactions can be prevented when steps 1 to 3 are repeated.

### Step 1-1

The 5' hydroxy group of unreacted compound (A) can be protected by reacting the crude product of compound (C) obtained in step 1 with preferably 5 to 1000 equivalents of an acylating reagent (AA) and preferably 5 to 1000 equivalents of a base in a solvent at a temperature between 0°C and a boiling point of the solvent for 10 seconds to 30 minutes. An appropriate additive may be added during the step in order to promote the reaction.

Examples of the acylating reagent include acetic anhydride and phenoxyacetic anhydride.

Examples of the solvent include dichloromethane, acetonitrile, ethyl acetate, THF, 1,4-dioxane and DMF, which may be used alone or as a mixture.

Examples of the base include pyridine and 2,6-lutidine.

Examples of the additive include 4-dimethylaminopyridine and 1-methylimidazole.

### Step 2

Compound (D) may be produced by reacting compound (C) with preferably 1 to 10 equivalents of an oxidizing agent in a solvent at a temperature between 0°C and a boiling point of the solvent for 10 seconds to 30 minutes in the presence of preferably 5 to 1000 equivalents of a base.

Examples of the oxidizing agent include iodine, hydrogen peroxide solution, m-chloroperoxybenzoic acid, peracetic acid, tert-butylhydroperoxide and (+)-camphorsulfonyloxaziridine (CSO), which may be used alone or as a mixture.

Examples of the base and the solvent include those described for step 1 above.

### Step 2-1

Compound (D-1) may be produced by reacting compound (C) with preferably 5 to 1000 equivalents of a sulfurizing agent in a solvent at a temperature between 0°C and a boiling point of the solvent for 10 seconds to 30 minutes in the presence of preferably 5 to 1000 equivalents of a base.

Examples of the sulfurizing agent include beaucage reagent (3H-1,2-benzodithiol-3-one-1,1-dioxide),
3-((N,N-dimethylaminomethylidene)amino)-3H-1,2,4-dithiazole-5-thione (DDTT) and phenylacetyl disulphide (PADS), which may be used alone or as a mixture.

Examples of the solvent include THF, acetonitrile, pyridine and picoline, which may be used alone or as a mixture.

### Step 3

Compound (E) may be produced by treating compound (D) with preferably 5 to 1000 equivalents of an acid in a solvent at a temperature between 0°C and a boiling point of the solvent for 10 seconds to 30 minutes.

Compound (E-1) may be produced by treating compound (D-1) with preferably 1 to 10 equivalents of an acid in a solvent at a temperature between 0°C and a boiling point of the solvent for 10 seconds to 30 minutes.

Examples of the acid include dichloroacetic acid, trichloroacetic acid and trifluoroacetic acid.

Examples of the solvent include dichloromethane and chloroform.

Compound (F) may be produced by repeating steps 1, 2, 2-1 and 3 over m times.

### Step 4

Compound (G) may be produced by treating compound (F) with preferably 5 to 1000 equivalents of a base in a solvent at a temperature between -80°C and 200°C for 10 seconds to 72 hours.

Examples of the base include ammonia, methylamine, dimethylamine, ethylamine, diethylamine, isopropylamine, diisopropylamine, piperidine, triethylamine, ethylenediamine, 1,8-diazabicyclo[5.4.0]-7-undecene (DBU) and potassium carbonate.

Examples of the solvent include water, methanol, ethanol and THF.

When R^{e} in compound (G) is a substituent represented by formula (C-1), compound (I) may be produced according to the following step 5.

### Step 5

Compound (I) may be produced by reacting compound (G) with preferably 5 to 1000 equivalents of compound (H) in a solvent at a temperature between 0°C and a boiling point of the solvent for 10 minutes to 24 hours.

Examples of the solvent include acetate buffer, Tris buffer, citrate buffer, phosphate buffer and water, which may be used alone or as a mixture.

Compound (H) may be produced according to the following step 5-1.

### Step 5-1

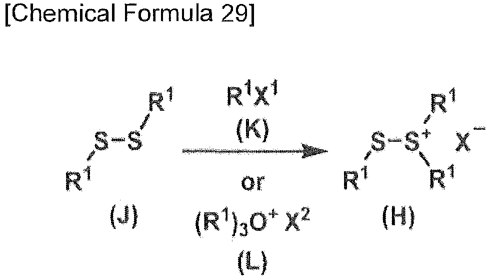

(wherein R¹ is as defined above; X⁻ represents an anion corresponding to X¹ or X²; X¹ represents, for example, trifluoromethanesulfonyloxy; and X² represents, for example, tetrafluoroborate or hexafluorophosphate.)

Compound (H) may be produced by reacting compound (J) with preferably 5 to 1000 equivalents of compound (K) or compound (L) in a solvent at a temperature between 0°C and a boiling point of the solvent for 10 minutes to 48 hours.

Examples of the solvent include dichloromethane, acetonitrile, toluene, ethyl acetate, THF, 1,4-dioxane, DMF, DMA and NMP, which may be used alone or as a mixture.

Examples of compound (K) include methyltriflate and ethyltriflate and examples of compound (L) include triethyloxonium hexafluorophosphate, trimethyloxonium tetrafluoroborate and triethyloxonium tetrafluoroborate.

Steps 1 to 5 and step 1-1 may be carried out by a nucleic acid synthesizer.

Compound (B-1), which is compound (B) wherein R^{c} is a substituent represented by formula (C-1), may be produced according to the following production method 2.

### Production method 2

### Production method of compound (B-1)

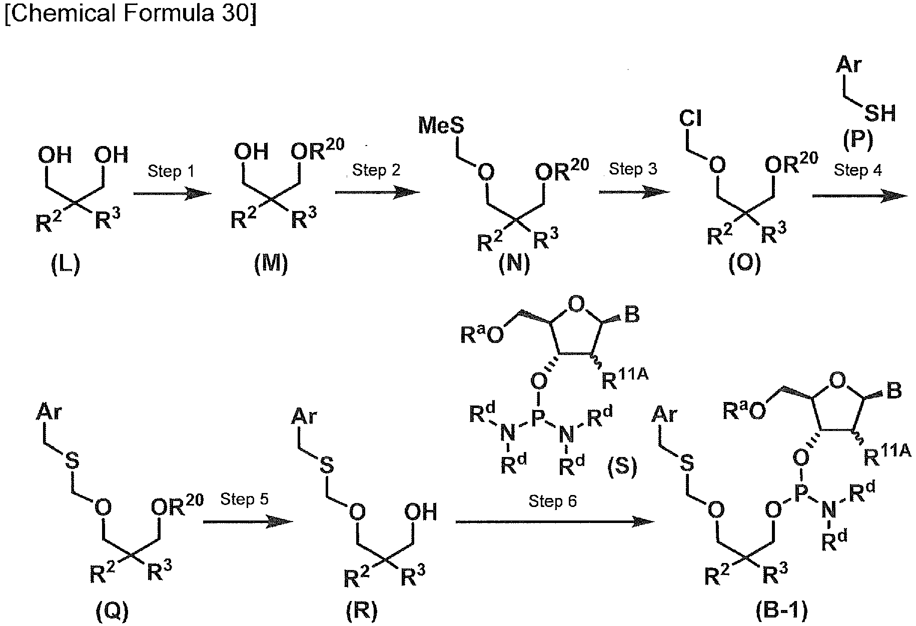

[wherein R²⁰ represents tri-lower alkylsilyl (three lower alkyl groups in the tri-lower alkylsilyl are the same or different and respectively have the same meaning as the lower alkyl above); R², R³, R^{11A}, R^{a}, R^{d}, B and Ar respectively are as defined above; and MeS represents methylthio.]

### Step 1

Compound (M) may be produced by reacting compound (L) with preferably 1 to 10 equivalents of a silylation agent in a solvent in the presence of preferably 1 to 10 equivalents of a base at a temperature between 0°C and a boiling point of the solvent for 10 minutes to 3 days. Preferably and optionally 0.1 to 10 equivalents of an additive may be added to promote the reaction.

Examples of the solvent include DMF, pyridine, dichloromethane, THF, ethyl acetate, 1,4-dioxane and NMP, which may be used alone or as a mixture.

Examples of the base include pyridine, triethylamine, N-ethyl-N,N-diisopropylamine and 2,6-lutidine.

Examples of the silylation agent include tert-butyldimethylsilyl chloride, triisopropylsilyl chloride and tert-butyldimethylsilyl triflate.

Examples of the additive include 4-dimethylaminopyridine.

Compound (L) may be, for example, commercially available.

### Step 2

Compound (N) may be produced by reacting compound (M) with preferably 1 to 10 equivalents of a methylthio methylation agent in a solvent at a temperature between 0°C and a boiling point of the solvent for 10 minutes to 3 days in the presence of preferably 0.1 to 10 equivalents of an additive.

Examples of the solvent include acetic acid, acetic anhydride and dimethylsulphoxide (DMSO).

Examples of the methylthio methylation agent include:
i) a combination of DMSO and an activating agent at 1 to 10 equivalents relative to DMSO; and
ii) a combination of MeSCH₂X³ (wherein X³ represents a chlorine atom, a bromine atom or an iodine atom; and MeS represents methylthio) and a base at 1 to 10 equivalents relative to MeSCH₂X³.

Examples of the activating agent include trifluoroacetic anhydride and acetic anhydride.

Examples of the base include trimethylamine and N,N-diisopropylethylamine.

### Step 3

Compound (O) may be produced by reacting compound (N) with preferably 1 to 10 equivalents of a chlorinating agent in a solvent at a temperature between 0°C and a boiling point of the solvent for 10 minutes to 3 days.

Examples of the solvent include dichloromethane, chloroform and 1,2-dichloroethane, which may be used alone or as a mixture.

Examples of the chlorinating agent include sulfuryl chloride.

### Step 4

Compound (Q) may be produced by reacting compound (O) with preferably 1 to 10 equivalents of compound (P) in a solvent in the presence of preferably 1 to 10 equivalents of a base at a temperature between -20°C and a boiling point of the solvent for 10 minutes to 24 hours.

Examples of the solvent include dichloromethane, acetonitrile, toluene, ethyl acetate, THF, 1,4-dioxane, DMF, DMA and NMP, which may be used alone or as a mixture.

Examples of the base include sodium hydride, DBU, trimethylamine and N,N-diisopropylethylamine, which may be used alone or as a mixture.

Compound (P) may be, for example, commercially available.

### Step 5

Compound (R) may be produced by reacting compound (Q) with preferably 1 to 10 equivalents of an additive in a solvent at a temperature between 0°C and a boiling point of the solvent for 10 minutes to 10 days.

Examples of the solvent include dichloromethane, acetonitrile, toluene, ethyl acetate, THF, 1,4-dioxane, DMF, DMA and NMP, which may be used alone or as a mixture.

Examples of the additive include tetrabutylammonium fluoride and triethylamine trihydrofluoride.

### Step 6

Compound (B-1) may be produced by reacting compound (R) with preferably 1 to 10 equivalents of compound (S) in a solvent at a temperature between 0°C and a boiling point of the solvent for 10 minutes to 48 hours in the presence of preferably 1 to 10 equivalents of an additive.

Examples of the solvent include dichloromethane, acetonitrile, toluene, ethyl acetate, THF, 1,4-dioxane, DMF, DMA and NMP, which may be used alone or as a mixture.

Examples of the additive include 1 H-tetrazole, 4,5-dicyanoimidazole, 5-ethylthiotetrazole and 5-benzylthiotetrazole.

Compound (S) may be produced according to, for example, a method disclosed in Chem. Commun., 2014, 50, 15063.

### Production method 3

### Production method of double-stranded oligonucleotide

A double-stranded oligonucleotide may be produced by reacting compound (I) with an equimolar single-stranded oligonucleotide in a solvent at a temperature between 30°C and 120°C for 10 seconds to 72 hours followed by gradually cooling to room temperature over 10 minutes to 24 hours.

Examples of the solvent include acetate buffer, Tris buffer, citrate buffer, phosphate buffer and water, which may be used alone or as a mixture.

The single-stranded oligonucleotide to be reacted with compound (I) is complementary to compound (I) and may contain one or more mismatched bases. The single-stranded oligonucleotide may be different in the base length from compound (I).

By variously modifying the nucleobase, reaction conditions in each step and the like in the above schemes, a desired oligonucleotide can be obtained.

The modifications may be carried out according to, for example, methods disclosed in:
(i) Tetrahedron, vol. 48, No. 12, p. 2223-2311 (1992);
(ii) Current Protocols in Nucleic Acids Chemistry, John Wiley & Sons (2000);
(iii) Protocols for Oligonucleotides and Analogs, Human Press (1993);
(iv) Chemistry and Biology of Artificial Nucleic Acids, Wiley-VCH (2012);
(v) Genomic Chemistry: Chemical Approach to Take Advantage of the Artificial Nucleic Acid, Kodansha Ltd. (2003); and
(vi) New Trend of Nucleic Acid Chemistry, Kagaku-Dojin Publishing Company, Inc. (2011).

Compound (B-2), which is compound (B) wherein R^{c} is a substituent represented by the following formula (D-1): 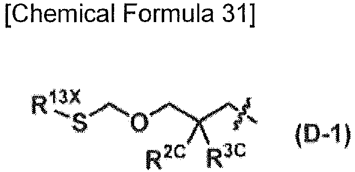 (wherein R^{2C}, R^{3C} and R^{13X} respectively are as defined above)
may be produced according to the following production method 4.

### Production method 4

### Production method of compound (B-2)

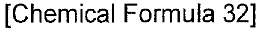

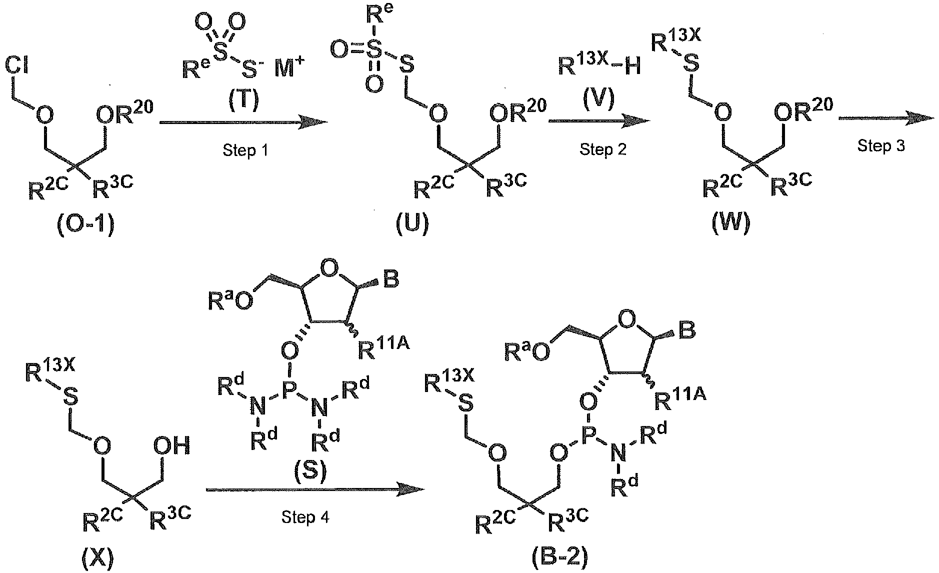

[wherein R^{2C}, R^{3C}, R^{11A}, R^{13X}, R²⁰, R^{a}, R^{d} and B respectively are as defined above; R^{e} represents lower alkyl or optionally substituted phenyl; and M represents a sodium atom or a potassium atom; lower alkyl is as defined above and the substituent in the optionally substituted phenyl has the same meaning as the substituent in the optionally substituted aryl described above.]

### Step 1

Compound (U) may be produced by reacting compound (O-1) with preferably 1 to 10 equivalents of compound (T) in a solvent at a temperature between 0°C and a boiling point of the solvent for 10 minutes to 3 days. Optionally and preferably, 0.1 to 10 equivalents of an additive may be added to promote the reaction.

Examples of the solvent include acetone and THF, which may be used alone or as a mixture.

Examples of the additive include sodium iodide and tetrabutylammonium iodide.

Compound (O-1) may be produced in the same manner as compound (O) in production method 3.

Compound (T) may be, for example, commercially available.

### Step 2

Compound (W) may be produced by reacting compound (U) with preferably 1 to 10 equivalents of compound (V) in a solvent at a temperature between 0°C and a boiling point of the solvent for 10 minutes to 3 days in the presence of preferably 0.1 to 10 equivalents of a basic agent.

Examples of the solvent include DMF, dichloromethane, THF, ethyl acetate, 1,4-dioxane, NMP and dimethylsulphoxide (DMSO).

Examples of the base include pyridine, trimethylamine and N-ethyl-N, N-d iisopropylamine.

Compound (V) may be, for example, commercially available.

### Step 3

Compound (X) may be produced in the same manner as in step 5 in production method 2 using compound (W).

### Step 4

Compound (B-2) may be produced in the same manner as in step 6 in production method 2 using compound (X).

Compound (B-3), which is compound (IV-1) wherein R^{13D1} is cyano, may be produced according to the following production method 5.

### Production method 5

### Production method of compound (B-3)

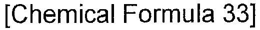

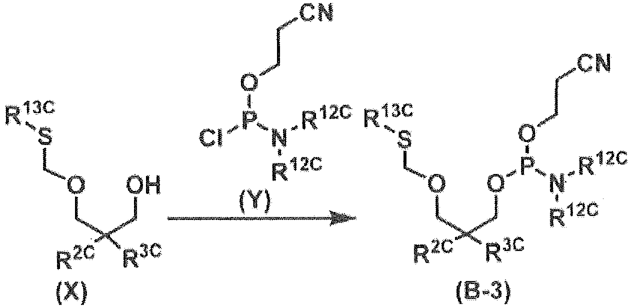

(wherein R^{13C}, R^{2C}, R^{3C} and R^{12C} respectively are as defined above.)

Compound (B-3) may be produced by reacting compound (X) with preferably 1 to 10 equivalents of compound (Y) in a solvent at a temperature between 0°C and a boiling point of the solvent for 10 minutes to 3 days in the presence of preferably 1 to 10 equivalents of a base.

Examples of the solvent include DMF, THF and dichloromethane.

Examples of the base include pyridine, trimethylamine and N-ethyl-N,N-diisopropylamine.

Compound (X) may be produced according to step 3 in production method 4.

Compound (I) or compound (I-0) having a group represented by formula (I) or formula (I-0) at an oxygen atom of a phosphate group at 5'-terminal of the oligonucleotide, namely compound (III-1), may be produced according to the following production method 6.

### Production method 6

A desired compound (I) or compound (I-0) may be obtained by using compound (B-3) in place of compound (B) in step 1 of production method 1.

When a defined group in the above production methods 1 to 6 is changed under the conditions of the production methods or is unsuitable for carrying out the production methods, a desired compound may be produced by using methods for introducing and eliminating protecting groups conventionally used in organic synthesis chemistry [such as Protective Groups in Organic Synthesis, fourth edition, T. W. Greene, John Wiley & Sons Inc. (2006)]. The order of the reaction steps for introduction of substituents and the like may also be optionally modified.

The present invention is specifically described hereinafter by way of Examples and Reference Examples which do not limit the present invention.

### Reference Example 1

### Step 1

### Diethyl 2-(((tert-butyldimethylsilyl)oxy)methyl)-2-(hydroxymethyl)malonate (compound 1B)

Commercially available diethyl 2,2-bis(hydroxymethyl)malonate (compound 1A, 6.18 g, 28.1 mmol) was dissolved in dichloromethane (90 mL) to which pyridine (4.44 mL, 56.1 mmol) and tert-butyldimethylsilyl chloride (6.34 g, 42.1 mmol) were added and stirred at room temperature for 4 days. Water was added to the reaction solution and it was extracted with chloroform. The organic layer was washed with water and saturated saline, dried over anhydrous sodium sulphate, and the solvent was distilled off under reduced pressure. The residue was purified by silica gel column chromatography (hexane/ethyl acetate) to give compound 1B (8.88 g, 95%).
ESI-MS (m/z): 335 (M+1)

### Step 2

### Diethyl

### 2-(((tert-butyldimethylsilyl)oxy)methyl)-2-(((methylthio)methoxy)methyl)malonate (compound 1C)

To compound 1B (9.73 g, 29.1 mmol) were added DMSO (60 mL), acetic acid (30 mL) and acetic anhydride (30 mL) and stirred at room temperature for 5 hours. 28% Aqueous ammonia was added to the reaction solution and it was stirred at room temperature for 1 hour. Water was added to the reaction solution and it was extracted with ethyl acetate. The organic layer was washed with water and saturated saline, dried over anhydrous sodium sulphate, and the solvent was distilled off under reduced pressure. The residue was purified by silica gel column chromatography (hexane/ethyl acetate) to give compound 1C (7.81 g, 68%).
ESI-MS (m/z): 395 (M+1)

### Step 3

### Diethyl-2-[(tert-butyldimethylsilyloxy)methyl]-2-[(chloromethoxy)methyl]malonate (compound 1 D)

Compound 1C (4.40 g, 11.2 mmol) was dissolved in dichloromethane (50 mL) to which sulfuryl chloride (1.18 mL, 14.5 mmol) was added and stirred at room temperature for 45 minutes. The reaction solution was concentrated under reduced pressure to give a crude product, compound 1 D (4.57 g).

### Step 4

### Diethyl

### 2-(((tert-butyldimethylsilyl)oxy)methyl)-2-((((2,4,6-trimethoxybenzyl)thio)methoxy)met hyl)malonate (compound 1E)

To a suspension of 60% sodium hydride (0.580 g, 14.5 mmol) in DMA (50 mL) was added (2,4,6-trimethoxyphenyl)methanethiol (3.11 g, 14.5 mmol) at 0°C and stirred at room temperature for 1 hour. To the mixture was added a solution of the crude product compound 1 D (4.57 g) in DMA (50 mL) and stirred at room temperature for 1 hour. Water was added to the reaction solution and it was extracted with ethyl acetate. The organic layer was washed with water and saturated saline, dried over anhydrous sodium sulphate, and the solvent was distilled off under reduced pressure. The residue was purified by silica gel column chromatography (hexane/ethyl acetate) to give compound 1E (3.78 g, 60%).
ESI-MS (m/z): 561 (M+1)

### Reference Example 2

### 1-((2R,3R,4R,SR)-5-((bis(4-methoxyphenyl)(phenyl)methoxy)methyl)-4-(bis(diisoprop ylamino)phosphinooxy)-3-fluorotetrahydrofuran-2-yl)pyrimidine-2,4(1H,3H)-dione (compound 2B)

Commercially available 1-((2R,3R,4R,5R)-5-((bis(4-methoxyphenyl)(phenyl)methoxy)methyl)-3-fluoro-4-hydr oxytetrahydrofuran-2-yl)pyrimidine-2,4(1H,3H)-dione (compound 2A, 20.7 g, 37.9 mmol) was dissolved in dichloromethane (360 mL) to which N,N-diisopropylethylamine (6.60 mL, 37.9 mmol) and bis(diisopropylamino)chlorophosphine (12.3 g, 46.1 mmol) were added under ice cooling and stirred for 3 hours. The solvent was distilled off from the reaction solution under reduced pressure, the residue was purified by silica gel column chromatography (ethyl acetate 100%) followed by slurry purification using n-heptane to give compound 2B (25.4 g, 86%).
³¹P-NMR (CDCl₃, 162 MHz) δ (ppm): 120.6.

### Reference Example 3

### 1-((2R,3R,4R,5R)-5-((bis(4-methoxyphenyl)(phenyl)methoxy)methyl)-4-(bis(diisoprop ylamino)phosphinooxy)-3-methoxytetrahydrofuran-2-yl)pyrimidine-2,4(1H,3H)-dione (compound 3B)

Compound 3B (10.9 g, 43%) was obtained in the same manner as in Reference Example 2 using commercially available 1-((2R,3R,4R,5R)-5-((bis(4-methoxyphenyl)(phenyl)methoxy)methyl)-4-hydroxy-3-m ethoxytetrahydrofuran-2-yl)pyrimidine-2,4(1H,3H)-dione (compound 3A, 17.8 g, 31.8 mmol).
³¹P-NMR (CDCl₃, 162 MHz) δ (ppm): 119.2.

### Reference Example 4

### 1-((2R,4S,5R)-5-((bis(4-methoxyphenyl)(phenyl)methoxy)methyl)-4-(bis(diisopropyla mino)phosphinooxy)-tetrahydrofuran-2-yl)-5-methylpyrimidine-2,4(1H,3H)-dione (compound 4B)

Compound 4B (3.80 g, 53%) was obtained in the same manner as in Reference Example 2 using commercially available 1-((2R,4S,5R)-5-((bis(4-methoxyphenyl)(phenyl)methoxy)methyl)-4-hydroxytetrahydr ofuran-2-yl)-5-methylpyrimidine-2,4(1H,3H)-dione (compound 4A, 5.00 g, 9.18 mmol).
³¹P-NMR (CDCl₃, 162 MHz) δ (ppm): 116.6.

### Reference Example 5

### 1,2-Diethyl-1-methyldisulphanium tetrafluoroborate (compound 5B)

Trimethyloxonium tetrafluoroborate (compound 5A, 1.10 g, 7.45 mmol) was dissolved in acetonitrile (5.5 mL) to which a solution of 1,2-diethyl disulphane (0.911 g, 7.45 mmol) in acetonitrile (2.0 mL) was added under ice cooling and stirred for 2 days. The reaction solution was concentrated under reduced pressure to give compound 5B (1.67 g).
¹H-NMR (CDCl₃, 400 MHz) δ (ppm): 3.57 - 3.61 (m, 2H), 3.32 (q, J = 7.1 Hz, 2H), 3.25 (s, 3H), 1.57 (t, J = 7.1 Hz, 3H), 1.50 (t, J = 7.1 Hz, 3H).

siRNA (1-fU) targeting HPRT1 was synthesized according to the following Reference Examples 6 to 8.

### Reference Example 6

The sense strand (SEQ ID NO: 1) of siRNAs (1-fU), (3-Y) and (4-Z) was synthesized according to the method disclosed in, for example, Protocols for Oligonucleotides and Analogs: Synthesis and Properties (Methods in Molecular Biology: Volume 20, 1993).

### Reference Example 7

The antisense strand (SEQ ID NO: 2) of siRNA (1-fU) was synthesized according to the method disclosed in, for example, Protocols for Oligonucleotides and Analogs: Synthesis and Properties (Methods in Molecular Biology: Volume 20, 1993).

### Reference Example 8

With the sense strand (SEQ ID NO: 1) and the antisense strand (SEQ ID NO: 2) of siRNA (1-fU) obtained in Reference Examples 6 and 7, siRNA (1-fU) was obtained according to the method disclosed in, for example, Protocols for Oligonucleotides and Analogs: Synthesis and Properties (Methods in Molecular Biology: Volume 20, 1993).

**[Table 1]**

| si RNA | **SEQ ID NO** | **Strand** | **Sequence (5'→3')** |
|---|---|---|---|
| 1-fU | 1 | **Sense** | GmCCmAGmACmUUmUGmUUmGGmAUmUAmGT |
| | 2 | **Antisense** | fUAmAU mCCmAAmCAmAAmGUmCUmGGmCUT |

(In the above table, A, G, C, T and U represent adenosine, guanosine, cytidine, thymidine and uridine, respectively; mA, mG, mC and mU represent 2'-O-methyladenosine, 2'-O-methylguanosine, 2'-O-methylcytidine and 2'-O-methyluridine, respectively; and fU represents 2'-fluorouridine.)
siRNA (6-fU) targeting HPRT1 was synthesized according to the following Reference Examples 9 to 11.

### Reference Example 9

The sense strand (SEQ ID NO: 6) of siRNAs (5-Z) and (6-fU) was synthesized using cholesteryl-TEG-phosphoramidite (Catalogue No. 10-1975-xx: Glen Research Corporation) according to the method disclosed in, for example, Protocols for Oligonucleotides and Analogs: Synthesis and Properties (Methods in Molecular Biology: Volume 20, 1993).

### Reference Example 10

The antisense strand (SEQ ID NO: 8) of siRNA (6-fU) was synthesized according to the method disclosed in, for example, Protocols for Oligonucleotides and Analogs: Synthesis and Properties (Methods in Molecular Biology: Volume 20, 1993).

### Reference Example 11

With the sense strand (SEQ ID NO: 6) and antisense strand (SEQ ID NO: 8) of siRNA (6-fU) obtained in Reference Examples 9 and 10, siRNA (6-fU) was obtained according to the method disclosed in, for example, Protocols for Oligonucleotides and Analogs: Synthesis and Properties (Methods in Molecular Biology: Volume 20, 1993).

**[Table 2]**

| si RNA | **SEQ ID NO** | **Strand** | **Sequence (5'→3')** |
|---|---|---|---|
| 6-fU | 6 | **Sense** | Ch-GmCCmAGmACmUfUmUGmUfUmGGmAfUmUAmGT |
| | 8 | **Antisense** | fUAmAfUmCCmAAmCAmAAmGfUmCfUmGGmCfUT |

(In the above table, A, G, C, T and U represent adenosine, guanosine, cytidine, thymidine and uridine, respectively; mA, mG, mC and mU represent 2'-O-methyladenosine, 2'-O-methylguanosine, 2'-O-methylcytidine and 2'-O-methyluridine, respectively; fU represents 2'-fluorouridine; Ch-G represents a residue corresponding to the compound represented by the following formula (wherein Me represents methyl).

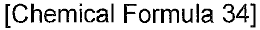

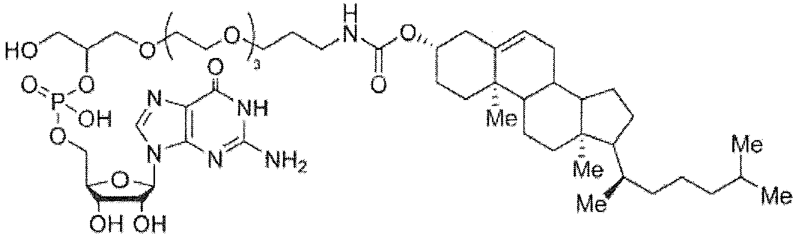

### Reference Example 12

### Step 1

### Diethyl

### 2-(((tert-butyldimethylsilyl)oxy)methyl)-2-(((tosylthio)methoxy)methyl)malonate (compound 12A)

Compound 1 D (1.73 g, 4.52 mmol) was dissolved in acetone (18 mL) to which commercially available potassium p-toluenethiosulphonate (1.23 g, 5.42 mmol) and sodium iodide (68.0 mg, 0.452 mmol) were added and stirred at room temperature for 30 minutes. Water was added to the reaction solution, the organic layer was extracted with ethyl acetate, washed with saturated saline, dried over anhydrous magnesium sulphate and concentrated under reduced pressure. The residue was purified by silica gel column chromatography (hexane/ethyl acetate) to give compound 12A (2.26 g, 94%).
¹H-NMR (CDCl₃, 400 MHz) δ (ppm): 7.83 - 7.79 (m, 2H), 7.35 - 7.31 (m, 2H), 5.29 (s, 2H), 4.14 (q, J = 7.1 Hz, 4H), 3.94 (s, 2H), 3.92 (s, 2H), 2.44 (s, 3H), 1.22 (t, J = 7.1 Hz, 6H), 0.82 (s, 9H), -0.01 (s, 6H).

### Step 2

### Diethyl

### 2,11,11,12,12-pentamethyl-6,10-dioxa-3,4-dithia-11-silatridecan-8,8-dicarboxylate (compound 12B)

Compound 12A (500 mg, 0.935 mmol) was dissolved in dichloromethane (4.6 mL) to which triethylamine (0.261 mL, 1.87 mmol) and propane-2-thiol (0.130 mL, 1.43 mmol) were added and stirred at room temperature for 15 hours. The reaction solution was directly purified by silica gel column chromatography (hexane/ethyl acetate) to give compound 12B (403 mg, 95%) as a colourless oily substance. ESI-MS (m/z): 455 (M+1)
¹H-NMR (CDCl₃, 400 MHz) δ (ppm): 4.81 (s, 2H), 4.18 (q, J = 7.2 Hz, 4H), 4.09 (s, 2H), 4.06 (s, 2H), 3.06 - 2.96 (m, 1H), 1.29 (d, J = 6.8 Hz, 6H), 1.25 (t, J = 7.2 Hz, 6H), 0.86 (s, 9H), 0.04 (s, 6H).

### Reference Example 13

### Diethyl

### 2,2,3,3-tetramethyl-4,8-dioxa-10,11-dithia-3-silahexadec-15-yn-6,6-dicarboxylate (compound 13A)

Compound 13A (31 mg, 65%) was obtained in the same manner as in step 2 in Reference Example 12 using compound 12A (53 mg, 0.10 mmol).
ESI-MS (m/z): 479 (M+1)
¹H-NMR (CDCl₃, 400 MHz) δ (ppm): 4.83 (s, 2H), 4.19 (q, J = 7.2 Hz, 4H), 4.09 (s, 2H), 4.06 (s, 2H), 2.83 (t, J = 6.8 Hz, 2H), 2.37 - 2.29 (m, 2H), 1.97 (t, J = 2.4 Hz, 1 H), 1.95 - 1.88 (m, 2H), 1.25 (t, J = 7.2 Hz, 6H), 0.86 (s, 9H), 0.04 (s, 6H).

### Reference Example 14

### Step 1

### Diethyl

### 2-(((tert-butyldimethylsilyl)oxy)methyl)-2-((((3-hydroxypropyl)disulfanyl)methoxy)meth yl)malonate (compound 14A)

Compound 14A (44.0 mg, quantitative) was obtained in the same manner as in step 2 in Reference Example 12 using compound 12A (50.0 mg, 94.0 µmol) and commercially available 3-mercapto-1-propanol.
¹H-NMR (CDCl₃, 400 MHz) δ (ppm): 4.84 (s, 2H), 4.19 (q, J = 7.2 Hz, 4H), 4.09 (s, 2H), 4.06 (s, 2H), 3.75 (t, J = 6.1 Hz, 2H), 2.85 (t, J = 7.2 Hz, 2H), 1.98 - 1.90 (m, 2H), 1.25 (t, J = 7.2 Hz, 6H), 0.86 (s, 9H), 0.04 (s, 6H).

### Step 2

### Diethyl

### 2-((((3-(bis(4-methoxyphenyl)(phenyl)methoxy)propyl)disulfanyl)methoxy)methyl)-2-(( (tert-butyldimethylsilyl)oxy)methyl)malonate (compound 14B)

Compound 14A (150 mg, 319 µmol) was dissolved in dichloromethane (2 mL) to which triethylamine (133 µL, 956 µmol) and 4,4'-dimethoxytrityl chloride (119 mg, 351 µmol) were added and stirred at room temperature overnight. Water was added to the reaction solution, the organic layer was extracted with ethyl acetate, washed with saturated saline, dried over anhydrous magnesium sulphate and concentrated under reduced pressure. The residue was purified by silica gel column chromatography (hexane/ethyl acetate) to give compound 14B (212 mg, 86%).
¹H-NMR (CDCl₃, 400 MHz) δ (ppm): 7.44 - 7.39 (m, 2H), 7.33 - 7.27 (m, 6H), 7.23 - 7.19 (m, 1 H), 6.85 - 6.79 (m, 4H), 4.79 (s, 2H), 4.16 (q, J = 7.2 Hz, 4H), 4.08 (s, 2H), 4.05 (s, 2H), 3.79 (s, 6H), 3.15 (t, J = 6.1 Hz, 2H), 2.83 (t, J = 7.2 Hz, 2H), 1.98 - 1.92 (m, 2H), 1.23 (t, J = 7.2 Hz, 6H), 0.85 (s, 9H), 0.03 (s, 6H).

### Reference Example 15

### Step 1

### Diethyl

### 17-hydroxy-2,2,3,3-tetramethyl-4,8-dioxa-10,11-dithia-3-silaheptadecan-6,6-dicarbox ylate (compound 15A)

Compound 15A (438 mg, 93%) was obtained in the same manner as in step 2 in Reference Example 12 using compound 12A (615 mg, 1.29 mmol).
ESI-MS (m/z): 513 (M+1)
¹H-NMR (CDCl₃, 400 MHz) δ (ppm): 4.82 (s, 2H), 4.18 (q, J = 7.2 Hz, 4H), 4.09 (s, 2H), 4.06 (s, 2H), 3.65 (t, J = 5.6 Hz, 2H), 2.74 (t, J = 7.2 Hz, 2H), 1.72 - 1.65 (m, 2H), 1.61 - 1.54 (m, 2H), 1.47 - 1.33 (m, 5H), 1.25 (d, J = 7.2 Hz, 6H), 0.86 (s, 9H), 0.04 (s, 6H).

### Step 2

### Diethyl

### 2,2,3,3-tetramethyl-17-(tosyloxy)-4,8-dioxa-10,11-dithia-3-silaheptadecan-6,6-dicarb oxylate (compound 15B)

Compound 15A (452 mg, 883 µmol) was dissolved in dichloromethane (8.8 mL) to which pyridine (143 µL, 1.76 mmol) and p-toluenesulfonyl chloride (252 mg, 1.32 mmol) were added and stirred at room temperature for 24 hours. Water was added to the reaction solution and it was stirred for 30 minutes and then the organic layer was extracted with chloroform, dried over anhydrous magnesium sulphate and concentrated under reduced pressure. The residue was purified by silica gel column chromatography (hexane/ethyl acetate) to give compound 15B (300 mg, 51%).
ESI-MS (m/z): 667 (M+1)
¹H-NMR (CDCl₃, 400 MHz) δ (ppm): 7.80 - 7.77 (m, 2H), 7.36 - 7.33 (m, 2H), 4.81 (s, 2H), 4.18 (q, J = 7.2 Hz, 4H), 4.09 (s, 2H), 4.05 (s, 2H), 4.02 (t, J = 6.4 Hz, 2H), 2.68 (t, J = 6.8 Hz, 2H), 2.45 (s, 3H), 1.68 - 1.58 (m, 4H), 1.34 - 1.30 (m, 4H), 1.24 (t, J = 7.2 Hz, 6H), 0.86 (s, 9H), 0.04 (s, 6H).

### Step 3

### Diethyl

### 17-azido-2,2,3,3-tetramethyl-4,8-dioxa-10,11-dithia-3-silaheptadecan-6,6-dicarboxyla te (compound 15C)

Compound 15B (296 mg, 0.443 mmol) was dissolved in DMF (4.4 mL) to which sodium azide (144 mg, 2.22 mmol) was added and stirred at room temperature for 18 hours. Water was added to the reaction solution, the organic layer was extracted with ethyl acetate, dried over anhydrous magnesium sulphate and concentrated under reduced pressure. The residue was purified by silica gel column chromatography (hexane/ethyl acetate) to give compound 15C (105 mg, 44%).
ESI-MS (m/z): 560 (M+Na)
¹H-NMR (CDCl₃, 400 MHz) δ (ppm): 4.82 (s, 2H), 4.18 (q, J = 7.2 Hz, 4H), 4.09 (s, 2H), 4.06 (s, 2H), 3.27 (t, J = 6.8 Hz, 2H), 2.73 (t, J = 7.2 Hz, 2H), 1.72 - 1.57 (m, 4H), 1.49 - 1.34 (m, 4H), 1.25 (t, J = 7.2 Hz, 6H), 0.86 (s, 9H), 0.04 (s, 6H).

### Reference Example 16

### Diethyl 2,2,3,3-tetramethyl-4,8-dioxa-10,11-dithia-3-silatricosan-6,6-dicarboxylate (compound 16A)

Compound 16A (488 mg, 88%) was obtained in the same manner as in step 2 in Reference Example 12 using compound 12A (510 mg, 954 µmol).
¹H-NMR (CDCl₃, 400 MHz) δ (ppm): 4.82 (s, 2H), 4.18 (q, J = 7.2 Hz, 4H), 4.09 (s, 2H), 4.06 (s, 2H), 2.72 (t, J = 7.2 Hz, 2H), 1.69 - 1.61 (m, 2H), 1.42 - 1.32 (m, 2H), 1.30 - 1.23 (m, 16H), 1.25 (t, J = 7.2 Hz, 6H), 0.88 (t, J = 6.4 Hz, 3H), 0.86 (s, 9H), 0.04 (s, 6H).

### Reference Example 17

### Step 1

### Di(prop-2-yn-1-yl)malonate (compound 17A)

Malonic acid (6.00 g, 57.7 mmol) was dissolved in a mixed solvent of dichloromethane (180 mL) and DMF (18 mL) to which 1-ethyl-3-(3-dimethylaminopropyl)carbodiimide hydrochloride (25.4 g, 133 mmol), 4-dimethylaminopyridine (0.704 g, 5.77 mmol) and 2-propyn-1-ol (8.51 mL, 144 mmol) were added in this order at room temperature and stirred at room temperature overnight. Saturated sodium bicarbonate water was added to the reaction solution, the organic layer was extracted with ethyl acetate, washed with saturated saline, dried over anhydrous magnesium sulphate and concentrated under reduced pressure. The residue was purified by silica gel column chromatography (hexane/ethyl acetate) to give compound 17A (9.51 g, 92%). ¹H-NMR (CDCl₃, 400 MHz) δ (ppm): 4.76 (d, J = 2.5 Hz, 4H), 3.49 (s, 2H), 2.51 (t, J = 2.5 Hz, 2H).

### Step 2

### Di(prop-2-yn-1-yl) 2,2-bis(hydroxymethyl)malonate (compound 17B)

Compound 17A (100 mg, 555 µmol) was dissolved in THF (2 mL) to which a 37% formaldehyde aqueous solution (165 µL, 2.22 mmol) and triethylamine (3.9 µL, 28 µmol) were added and stirred at room temperature overnight. 1 mol/L Hydrochloric acid was added to the reaction solution, the organic layer was extracted with ethyl acetate, washed with saturated saline, dried over anhydrous magnesium sulphate and concentrated under reduced pressure. The residue was purified by silica gel column chromatography (chloroform/methanol) to give compound 17B (96.5 mg, 72%).
¹H-NMR (CDCl₃, 400 MHz) δ (ppm): 4.79 (d, J = 2.5 Hz, 4H), 4.17 (d, J = 7.1 Hz, 4H), 2.65 (t, J = 7.1 Hz, 2H), 2.51 (t, J = 2.5 Hz, 2H).

### Step 3

### Di(prop-2-yn-1-yl) 2-(((tert-butyldimethylsilyl)oxy)methyl)-2-(hydroxymethyl)malonate (compound 17C)

Compound 17C (73.1 mg, 52%) was obtained in the same manner as in step 1 in Reference Example 1 using compound 17B (95.0 mg, 395 µmol).
¹H-NMR (CDCl₃, 400 MHz) δ (ppm): 4.76 (d, J = 2.4 Hz, 2H), 4.75 (d, J = 2.4 Hz, 2H), 4.16 (d, J = 6.8 Hz, 2H), 4.11 (s, 2H), 2.48 (t, J = 2.4 Hz, 2H), 2.40 (t, J = 6.8 Hz, 1 H), 0.86 (s, 9H), 0.07 (s, 6H).

### Step 4

### Di(prop-2-yn-1-yl)

### 2-(((tert-butyldimethylsilyl)oxy)methyl)-2-(((methylthio)methoxy)methyl)malonate (compound 17D)

Compound 17D (0.795 g, 65%) was obtained in the same manner as in step 2 in Reference Example 1 using compound 17C (1.04 g, 2.93 mmol).
¹H-NMR (CDCl₃, 400 MHz) δ (ppm): 4.72 (d, J = 2.4 Hz, 4H), 4.64 (s, 2H), 4.13 (s, 2H), 4.06 (s, 2H), 2.46 (t, J = 2.4 Hz, 2H), 2.10 (s, 3H), 0.86 (s, 9H), 0.05 (s, 6H).

### Step 5

### Di(prop-2-yn-1-yl)

### 2-(((tert-butyldimethylsilyl)oxy)methyl)-2-((chloromethoxy)methyl)malonate (compound 17E)

Compound 17D (6.26 g, 15.1 mmol) was dissolved in dichloromethane (44 mL) to which cyclohexene (1.99 mL, 19.6 mmol) and sulfuryl chloride (1.59 mL, 19.6 mmol) were added under ice cooling and stirred at room temperature for 2.5 hours. The reaction solution was concentrated under reduced pressure to give a crude product, compound 17E (10.6 g). The crude product was used in the next step without purification.
¹H-NMR (CDCl₃, 400 MHz) δ (ppm): 5.45 (s, 2H), 4.73 (d, J = 2.5 Hz, 4H), 4.22 (s, 2H), 4.11 (s, 2H), 2.47 (t, J = 2.5 Hz, 2H), 0.86 (s, 9H), 0.06 (s, 6H).

### Step 6

### Di(prop-2-yn-1-yl)

### 2-(((tert-butyldimethylsilyl)oxy)methyl)-2-(((tosylthio)methoxy)methyl)malonate (compound 17F)

Compound 17F (6.23 g, 2 steps 70%) was obtained in the same manner as in step 1 in Reference Example 12 using the crude product compound 17E (10.6 g).
¹H-NMR (CDCl₃, 400 MHz) δ (ppm): 7.81 (d, J = 8.7 Hz, 2H), 7.34 (d, J = 8.7 Hz, 2H), 5.28 (s, 2H), 4.70 (dd, J = 15.5, 2.5 Hz, 2H), 4.65 (dd, J = 15.5, 2.5 Hz, 2H), 3.96 (s, 2H), 3.93 (s, 2H), 2.47 (t, J = 2.5 Hz, 2H), 2.45 (s, 3H), 0.82 (s, 9H), -0.01 (s, 6H).

### Step 7

### Di(prop-2-yn-1-yl)

### 2-(((tert-butyldimethylsilyl)oxy)methyl)-2-(((isopropyldisulfanyl)methoxy)methyl)malon ate (compound 17G)

Compound 17G (0.883 g, 86%) was obtained in the same manner as in step 2 in Reference Example 12 using compound 17F (1.20 g, 2.16 mmol) and 2-propanethiol (0.241 mL, 2.60 mmol).
¹H-NMR (CDCl₃, 400 MHz) δ (ppm): δ: 4.82 (s, 2H), 4.73 (d, J = 2.5 Hz, 2H), 4.72 (d, J = 2.5 Hz, 2H), 4.13 (s, 2H), 4.10 (s, 2H), 3.02 (sep, J = 6.8 Hz, 1 H), 2.46 (t, J = 2.5 Hz, 2H), 1.30 (d, J = 6.8 Hz, 6H), 0.86 (s, 9H), 0.06 (s, 6H).

### Reference Example 18

### Step 1

### Bis(4-ethynylbenzyl)malonate (compound 18A)

To dichloromethane (100 mL) were dissolved 4-ethynylbenzyl alcohol (7.00 g, 53.0 mmol) and N,N-diisopropylethylamine (18.5 mL, 106 mmol) under ice cooling to which malonyl chloride (3.86 mL, 39.7 mmol) was added and stirred under ice cooling for 45 minutes. Saturated sodium bicarbonate water was added to the reaction solution, the organic layer was extracted with dichloromethane, washed with saturated saline, dried over anhydrous sodium sulphate and concentrated under reduced pressure. The residue was purified by silica gel column chromatography (heptane/ethyl acetate) to give compound 18A (4.53 g, 51%).
¹H-NMR (CDCl₃, 400 MHz) δ (ppm): 7.47 (d, J = 8.2 Hz, 4H), 7.27 (d, J = 8.2 Hz, 4H), 5.16 (s, 4H), 3.48 (s, 2H), 3.10 (s, 2H).

### Step 2

### Bis(4-ethynylbenzyl) 2,2-bis(hydroxymethyl)malonate (compound 18B)

A crude product, compound 18B (8.29 g) was obtained in the same manner as in step 2 in Reference Example 17 using compound 18A (5.35 g, 16.1 mmol). The crude product was used in the next step without purification.

### Step 3

### Bis(4-ethynylbenzyl)

### 2-(((tert-butyldimethylsilyl)oxy)methyl)-2-(hydroxymethyl)malonate (compound 18C)

Compound 18C (1.45 g, 2 steps 18%) was obtained in the same manner as in step 1 in Reference Example 1 using the crude product compound 18B (8.29 g) obtained in step 2.
¹H-NMR (CDCl₃, 400 MHz) δ (ppm): 7.45 - 7.41 (m, 4H), 7.22 - 7.18 (m, 4H), 5.13 (s, 4H), 4.16 (s, 2H), 4.10 (s, 2H), 3.09 (s, 2H), 0.83 (s, 9H), 0.01 (s, 6H).

### Step 4

### Bis(4-ethynylbenzyl)

### 2-(((tert-butyldimethylsilyl)oxy)methyl)-2-(((methylthio)methoxy)methyl)malonate (compound 18D)

A crude product, compound 18D (2.10 g) was obtained in the same manner as in step 2 in Reference Example 1 using compound 18C (2.92 g, 5.76 mmol). ¹H-NMR (CDCl₃, 400 MHz) δ (ppm): 7.44 - 7.41 (m, 4H), 7.22 - 7.18 (m, 4H), 5.10 (s, 4H), 4.58 (s, 2H), 4.13 (s, 2H), 4.05 (s, 2H), 3.09 (s, 2H), 2.02 (s, 3H), 0.83 (s, 9H), 0.00 (s, 6H).

### Step 5

### Bis(4-ethynylbenzyl)

### 2-(((tert-butyldimethylsilyl)oxy)methyl)-2-((chloromethoxy)methyl)malonate (compound 18E)

A crude product, compound 18E (2.53 g) was obtained in the same manner as in step 3 in Reference Example 1 using the crude product compound 18D (2.10 g) obtained in step 4.
¹H-NMR (CDCl₃, 400 MHz) δ (ppm): 7.45 - 7.41 (m, 4H), 7.21 - 7.17 (m, 4H), 5.38 (s, 2H), 5.10 (s, 4H), 4.21 (s, 2H), 4.10 (s, 2H), 3.10 (s, 2H), 0.83 (s, 9H), 0.00 (s, 6H).

### Step 6

### Bis(4-ethynylbenzyl)

### 2-(((tert-butyldimethylsilyl)oxy)methyl)-2-(((tosylthio)methoxy)methyl)malonate (compound 18F)

Compound 18F (1.83 g, 3 steps 47%) was obtained in the same manner as in step 1 in Reference Example 12 using compound 18E (2.43 g).
¹H-NMR (CDCl₃, 400 MHz) δ (ppm): 7.79 - 7.75 (m, 2H), 7.44 - 7.40 (m, 4H), 7.30 - 7.26 (m, 2H), 7.18 - 7.14 (m, 4H), 5.22 (s, 2H), 5.06 (d, J = 12.8 Hz, 2H), 5.02 (d, J = 12.8 Hz, 2H), 3.99 (s, 2H), 3.97 (s, 2H), 3.10 (s, 2H), 2.41 (s, 3H), 0.80 (s, 9H), -0.04 (s, 6H).

### Step 7

### Bis(4-ethynylbenzyl)

### 2-(((tert-butyldimethylsilyl)oxy)methyl)-2-(((isopropyldisulfanyl)methoxy)methyl)malon ate (compound 18G)

Compound 18G (1.41 g, 87%) was obtained in the same manner as in step 2 in Reference Example 12 using compound 18F (1.83 g, 2.59 mmol) and 2-propanethiol (0.361 mL, 3.88 mmol).
¹H-NMR (CDCl₃, 400 MHz) δ (ppm): 7.45 - 7.41 (m, 4H), 7.21 - 7.17 (m, 4H), 5.10 (s, 4H), 4.75 (s, 2H), 4.12 (s, 2H), 4.09 (s, 2H), 3.09 (s, 2H), 2.99 (sep, J = 6.9 Hz, 1H), 1.27 (d, J = 6.9 Hz, 6H), 0.83 (s, 9H), 0.01 (s, 6H).

### Reference Example 19

### Step 1

### Ethyl 2-cyano-3-hydroxy-2-(hydroxymethyl)propanoate (compound 19A)

A crude product, compound 19A (97.8 g) was obtained in the same manner as in step 2 in Reference Example 17 using cyanoethyl acetate (56.6 g, 500 mmol). The crude product was used in the next reaction without purification.

### Step 2

### Ethyl 3-((tert-butyldimethylsilyl)oxy)-2-cyano-2-(hydroxymethyl)propanoate (compound 19B)

Compound 19B (33.0 g, 2 steps 22%) was obtained in the same manner as in step 1 in Reference Example 1 using the crude product compound 19A (97.8 g).
¹H-NMR (CDCl₃, 400 MHz) δ (ppm): 4.30 (q, J = 7.2 Hz, 2H), 4.07 (dd, J = 11.0, 6.6 Hz, 1 H), 4.02 (s, 2H), 3.99 (dd, J = 11.0, 6.6 Hz, 1 H), 2.43 (br t, J = 6.6 Hz, 1 H), 1.34 (t, J = 7.2 Hz, 3H), 0.90 (s, 9H), 0.10 (s, 6H).

### Step 3

### Ethyl

### 3-((tert-butyldimethylsilyl)oxy)-2-cyano-2-(((methylthio)methoxy)methyl)propanoate (compound 19C)

Compound 19C (11.1 g, 26%) was obtained in the same manner as in step 2 in Reference Example 1 using compound 19B (33.0 g, 108 mmol).
¹H-NMR (CDCl₃, 400 MHz) δ (ppm): 4.69 (s, 2H), 4.31 - 4.26 (m, 2H), 4.01 (d, J = 9.6 Hz, 1 H), 3.98 (d, J = 9.6 Hz, 1 H), 3.93 (d, J = 9.2 Hz, 1 H), 3.86 (d, J = 9.2 Hz, 1 H), 2.15 (s, 3H), 1.34 (t, J = 7.1 Hz, 3H), 0.89 (s, 9H), 0.09 (s, 3H), 0.09 (s, 3H).

### Step 4

### Ethyl 3-((tert-butyldimethylsilyl)oxy)-2-((chloromethoxy)methyl)-2-cyanopropanoate (compound 19D)

A crude product compound 19D (7.93 g) was obtained in the same manner as in step 3 in Reference Example 1 using compound 19C (8.00 g, 20.5 mmol).
¹H-NMR (CDCl₃, 400 MHz) δ (ppm): 5.49 (d, J = 12.8 Hz, 1H), 5.47 (d, J = 12.8 Hz, 1H), 4.29 (q, J = 7.1 Hz, 2H), 4.05 (s, 2H), 4.00 (s, 2H), 1.34 (t, J = 7.1 Hz, 3H), 0.89 (s, 9H), 0.09 (s, 6H).

### Step 5

### Ethyl

### 3-((tert-butyldimethylsilyl)oxy)-2-cyano-2-((((2,4,6-trimethoxybenzyl)thio)methoxy)me thyl)propanoate (compound 19E)

Compound 19E (6.62 g, 2 steps 63%) was obtained in the same manner as in step 4 in Reference Example 1 using the crude product compound 19D (7.40 g).
ESI-MS (m/z): 536 (M+Na)
¹H-NMR (CDCl₃, 400 MHz) δ (ppm): 6.12 (s, 2H), 4.74 (d, J = 11.6 Hz, 1H), 4.70 (d, J = 11.6 Hz, 1 H), 4.31 - 4.26 (m, 2H), 4.02 (d, J = 9.6 Hz, 1 H), 3.99 (d, J = 9.6 Hz, 1 H), 3.91 (d, J = 9.1 Hz, 1 H), 3.87 (d, J = 9.1 Hz, 1 H), 3.83 (s, 6H), 3.81 (s, 2H), 3.81 (s, 3H), 1.34 (t, J = 7.3 Hz, 3H), 0.90 (s, 9H), 0.10 (s, 3H), 0.09 (s, 3H).

### Step 6

### Ethyl

### 3-((tert-butyldimethylsilyl)oxy)-2-cyano-2-(((isopropyldisulfanyl)methoxy)methyl)prop anoate (compound 19F)

In an argon atmosphere, trimethyloxonium tetrafluoroborate (9.31 g, 63.0 mmol) was dissolved in acetonitrile (65 mL) to which diisopropyl disulphide (10.0 mL, 63.0 mmol) was added dropwise under ice cooling over 2 minutes and stirred under ice cooling for 5.5 hours. While maintaining the internal temperature of the reaction solution to -40°C to -45°C in a dry ice/acetone bath, a solution of compound 19E (6.47 g, 12.6 mmol) in acetonitrile (65 mL) was added dropwise with a dropping funnel over 9 minutes. The dropping funnel was washed with acetonitrile (20 mL) and the solution was stirred at -40°C for 30 minutes. To the reaction solution, saturated sodium bicarbonate water was added at -40°C over 2 minutes followed by heating to room temperature. Water was added to the reaction solution, the organic layer was extracted with ethyl acetate, washed with water, dried over anhydrous sodium sulphate and concentrated under reduced pressure. The residue was purified by silica gel column chromatography (heptane/ethyl acetate) to give compound 19E (1.48 g, 29%).
¹H-NMR (CDCl₃, 400 MHz) δ (ppm): 4.89 (d, J = 11.3 Hz, 1H), 4.86 (d, J = 11.3 Hz, 1H), 4.31 - 4.26 (m, 2H), 4.01 (d, J = 9.1 Hz, 1 H), 3.98 (d, J = 9.1 Hz, 1 H), 3.95 (d, J = 9.6 Hz, 1 H), 3.92 (d, J = 9.6 Hz, 1 H), 3.08 (sep, J = 6.6 Hz, 1 H), 1.33 (t, J = 7.1 Hz, 3H), 1.31 (d, J = 6.6 Hz, 6H), 0.89 (s, 9H), 0.10 (s, 3H), 0.09 (s, 3H).

### Reference Example 20

### Step 1

### Ethyl 3-(tert-butyldimethylsilyloxy)-2-cyano-2-((tosylthiomethoxy)methyl)propanate (compound 20A)

Compound 20A (515 mg, 96%) was obtained in the same manner as in step 1 in Reference Example 12 using compound 19D (371 mg, 1.10 mmol).
ESI-MS (m/z): 488 (M+1)

### Step 2

### Ethyl

### 6-cyano-14-hydroxy-2,2,3,3-tetramethyl-4,8-dioxa-10,11-dithia-3-silatetradecane-6-c arboxylate (compound 20B)

Compound 20B (407 mg, 84%) was obtained in the same manner as in step 1 in Reference Example 12 using compound 20A (555 mg, 1.14 mmol).
ESI-MS (m/z): 424 (M+1)
¹H-NMR (CDCl₃, 400 MHz) δ (ppm): 4.91 (s, 2H), 4.35 - 4.24 (m, 2H), 3.99 (dd, J = 12.0, 9.6 Hz, 2H), 3.93 (q, J = 7.2 Hz, 2H), 3.75 (t br, J = 5.6 Hz, 2H), 2.92 (t, J = 6.8 Hz, 2H), 1.99 - 1.92 (m, 2H), 1.34 (t, J = 7.2 Hz, 3H), 0.89 (s, 9H), 0.09 (d, J = 3.2 Hz, 6H).

### Step 3

### Ethyl

### 11-cyano-1,1-bis(4-methoxyphenyl)-14,14,15,15-tetramethyl-1-phenyl-2,9,13-trioxa-6 ,7-dithia-14-silahexadecane-11-carboxylate (compound 20C)

Compound 20C (2.63 g, 99%) was obtained in the same manner as in step 2 in Reference Example 14 using compound 20B (1.55 g, 3.66 mmol).
ESI-MS (m/z): 748 (M+Na)
¹H-NMR (CDCl₃, 400 MHz) δ (ppm): 7.43 - 7.39 (m, 2H), 7.32 - 7.28 (m, 6H), 7.22 - 7.19 (m, 1 H), 6.84 - 6.80 (m, 4H), 4.85 (d, J = 1.2 Hz, 2H), 4.31 - 4.19 (m, 2H), 3.97 (d, J = 2.0 Hz, 2H), 3.91 (q, J = 7.2 Hz, 2H), 3.79 (s, 6H), 3.16 (t, J = 6.4 Hz, 2H), 2.88 (t, J = 6.8 Hz, 2H), 2.00 - 1.93 (m, 2H), 1.31 (t, J = 7.2 Hz, 3H), 0.88 (s, 9H), 0.08 (d, J = 2.4 Hz, 6H).

### Reference Example 21

### Diisoprop-2-ynyl

### 2,2,3,3-tetramethyl-4,8-dioxa-10,11-dithia-3-silahexadec-15-yn-6,6-dicarboxylate (compound 21A)

Compound 21A (112 mg, 58%) was obtained in the same manner as in step 2 in Reference Example 12 using compound 17F (247 mg, 0.497 mmol).
ESI-MS (m/z): 521 (M+Na)
¹H-NMR (CDCl₃, 400 MHz) δ (ppm): 4.83 (s, 2H), 4.73 (dd, J = 2.4, 1.2 Hz, 4H), 4.13 (s, 2H), 4.10 (s, 2H), 2.84 (t, J = 6.8 Hz, 2H), 2.47 (t, J = 2.4 Hz, 2H), 2.35 - 2.31 (m, 2H), 1.98 (t, J = 2.8 Hz, 1 H), 1.95 - 1.88 (m, 2H), 0.86 (s, 9H), 0.06 (s, 6H).

### Reference Example 22

### Step 1

The sense strand (SEQ ID NO: 19) of Ctrl (5',3'dT) was synthesized according to the method disclosed in, for example, Protocols for Oligonucleotides and Analogs: Synthesis and Properties (Methods in Molecular Biology: Volume 20, 1993).

### Step 2

The antisense strand (SEQ ID NO: 20) of Ctrl (5',3'dT) was synthesized according to the method disclosed in, for example, Protocols for Oligonucleotides and Analogs: Synthesis and Properties (Methods in Molecular Biology: Volume 20, 1993).

### Step 3

With the sense strand and the antisense strand of Ctrl (5',3'dT) obtained in steps 1 and 2, Ctrl (5',3'dT) was obtained according to the method disclosed in, for example, Protocols for Oligonucleotides and Analogs: Synthesis and Properties (Methods in Molecular Biology: Volume 20, 1993).

**[Table 3]**

| si RNA | **SEQ ID NO** | **Strand** | **Sequence (5'→3')** |
|---|---|---|---|
| Ctrl(5',3' dT) | 19 | **Sense** | GCCAGACUUUGUUGGAUUAGT |
| | 20 | **Antisense** | TAAUCCAACAAAGUCUGGCUT |

(In the above table, A, G, C, T and U represent adenosine, guanosine, cytidine, thymidine and uridine, respectively.)

### Reference Example 23

### Step 1

The antisense strand (SEQ ID NO: 21) of KON788 was obtained in the same manner as in step 1 in Example 5 using compound 3.
ESI-MS theoretical: 7065 measured: 7065

### Step 2

KON788 was obtained in the same manner as in step 2 in Example 5 using the antisense strand of KON788.

**[Table 4]**

| si RNA | **SEQ ID NO** | **Strand** | **Sequence (5'→3')** |
|---|---|---|---|
| KON788 | 19 | **Sense** | GCCAGACUUUGUUGGAUUAGT |
| | 21 | **Antisense** | X⁴AAUCCAACAAAGUCUGGCUT |

(In the above table, A, G, C, T and U represent adenosine, guanosine, cytidine, thymidine and uridine, respectively; and X⁴ represents a residue corresponding to the compound represented by the following formula.)

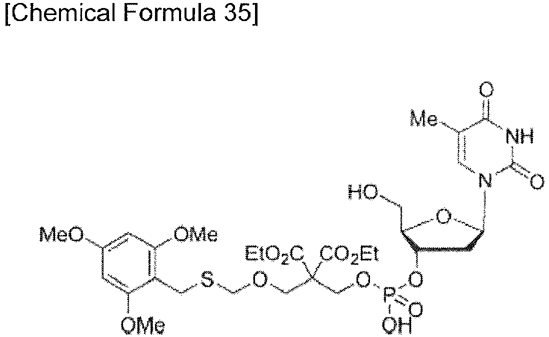

### Reference Example 24

### Step 1

### Diethyl 2-((tert-butyldimethylsilyloxy)methyl)-2-((disulfanylmethoxy)methyl)malonate (compound 23A)

Compound 1D (2.48 g, 6.21 mmol) was dissolved in DMA (54.5 mL) to which disodium disulphide (684 mg, 6.21 mmol) was added and stirred at room temperature for 90 minutes. Water was added to the reaction solution, the organic layer was extracted with ethyl acetate, washed with water and saturated saline, dried over anhydrous sodium sulphate, and the solvent was distilled off under reduced pressure. The residue was purified by silica gel column chromatography (hexane/ethyl acetate) to give compound 23A (1.85 g, 72%).
ESI-MS (m/z): 413 (M+H)

### Step 2

### Diethyl

### 19-azido-2,2,3,3-tetramethyl-4,8,14,17-tetraoxa-10,11-dithia-3-silanonadecane-6,6-di carboxylate (compound 23B)

Compound 23A (1.52 g, 4.68 mmol) was dissolved in DMA (20mL) to which 1-azido-2-(2-(2-iodoethoxy)ethoxy)ethane (1.14 g, 4.00 mmol) and DBU (3.01 mL, 20.0 mmol) were added and stirred at room temperature for 24 hours. Water was added to the reaction solution, the organic layer was extracted with ethyl acetate, washed with water and saturated saline, dried over anhydrous sodium sulphate, and the solvent was distilled off under reduced pressure. The residue was purified by silica gel column chromatography (hexane/ethyl acetate) to give compound 23B (230 mg, 10%).
ESI-MS (m/z): 570 (M+H)

### Step 3

### Diethyl 2-(13-azido-2,8,11-trioxa-4,5-dithiatridecyl)-2-(hydroxymethyl)malonate (compound 23C)

Compound 23C (120 mg, 53%) was obtained in the same manner as in Example 1 using compound 23B (285 mg, 0.500 mmol).
ESI-MS (m/z): 455 (M+H)

### Step 4

### Diethyl

### 19-azido-4-((2R,3S,5R)-2-((bis(4-methoxyphenyl)(phenyl)methoxy)methyl)-5-(5-meth yl-2,4-dioxo-3,4-dihydropyrimidin-1(2H)-yl)tetrahydrofuran-3-yloxy)-3-isopropyl-2-met hyl-5, 9,14,17-tetraoxa-11-thia-3-aza-4-phosphanonadecane-7,7-dicarboxylate

### (compound 23D)

Compound 23D (112 mg, 39%) was obtained in the same manner as in Example 2 using compound 23C (119 mg, 0.261 mmol) and compound 4B (263 mg, 0.343 mmol).
ESI-MS(m/z): 1090 (M+Na)

### Step 5

The antisense strand (SEQ ID NO: 25) of KON840 was obtained in the same manner as in step 1 in Example 5 using compound 23D.
ESI-MS theoretical: 7042 measured: 7041

### Step 6

KON840 was obtained in the same manner as in step 2 in Example 5 using the antisense strand of KON840.

**[Table 5]**

| si RNA | **SEQ ID NO** | **Strand** | **Sequence (5'→3')** |
|---|---|---|---|
| KON840 | 19 | **Sense** | GCCAGACUUUGUUGGAUUAGT |
| | 25 | **Antisense** | X⁵AAUCCAACAAAGUCUGGCUT |

[In the above table, A, G, C, T and U represent adenosine, guanosine, cytidine, thymidine and uridine, respectively; and X⁵ represents a residue corresponding to the compound represented by the following formula (wherein Me represents methyl; Et represents ethyl; and N₃ represents azido).]

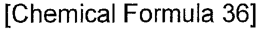

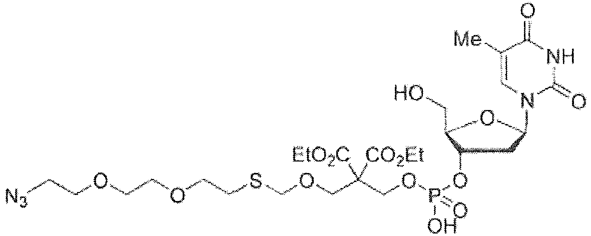

### Reference Example 25

### Step 1

The sense strand (SEQ ID NO: 19) of Ctrl (5'-F) was synthesized according to the method disclosed in, for example, Protocols for Oligonucleotides and Analogs: Synthesis and Properties (Methods in Molecular Biology: Volume 20, 1993).

### Step 2

The antisense strand (SEQ ID NO: 28) of Ctrl (5'-F) was synthesized according to the method disclosed in, for example, Protocols for Oligonucleotides and Analogs: Synthesis and Properties (Methods in Molecular Biology: Volume 20, 1993).

### Step 3

With the sense strand and the antisense strand of Ctrl (5'-F) obtained in steps 1 and 2, Ctrl (5'-F) was obtained according to the method disclosed in, for example, Protocols for Oligonucleotides and Analogs: Synthesis and Properties (Methods in Molecular Biology: Volume 20, 1993).

**[Table 6]**

| si RNA | **SEQ ID NO** | **Strand** | **Sequence (5'→3')** |
|---|---|---|---|
| Ctrl(5'-F) | 19 | **Sense** | GCCAGACUUUGUUGGAUUAGT |
| | 28 | **Antisense** | X⁶AAUCCAACAAAGUCUGGCUT |

(In the above table, A, G, C, T and U represent adenosine, guanosine, cytidine, thymidine and uridine, respectively; and X⁶ represents a residue corresponding to the compound represented by the following formula.)

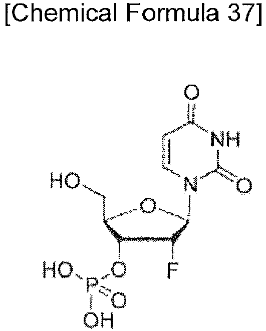

### Reference Example 26

### Step 1

The sense strand (SEQ ID NO: 40) of wt924 was synthesized according to the method disclosed in, for example, Protocols for Oligonucleotides and Analogs: Synthesis and Properties (Methods in Molecular Biology: Volume 20, 1993).

### Step 2

The antisense strand (SEQ ID NO: 41) of wt924 was synthesized according to the method disclosed in, for example, Protocols for Oligonucleotides and Analogs: Synthesis and Properties (Methods in Molecular Biology: Volume 20, 1993).

### Step 3

With the sense strand and the antisense strand of wt924 obtained in steps 1 and 2, wt924 was obtained according to the method disclosed in, for example, Protocols for Oligonucleotides and Analogs: Synthesis and Properties (Methods in Molecular Biology: Volume 20, 1993).

### Reference Example 27

### Step 1

The antisense strand (SEQ ID NO: 42) of wt925 was synthesized according to the method disclosed in, for example, Protocols for Oligonucleotides and Analogs: Synthesis and Properties (Methods in Molecular Biology: Volume 20, 1993).

### Step 2

With the antisense strand and the sense strand of wt925 obtained in step 1 above and step 1 of Reference Example 26, wt925 was obtained according to the method disclosed in, for example, Protocols for Oligonucleotides and Analogs: Synthesis and Properties (Methods in Molecular Biology: Volume 20, 1993).

### Reference Example 28

### Step 1

The antisense strand (SEQ ID NO: 43) of wt926 was synthesized according to the method disclosed in, for example, Protocols for Oligonucleotides and Analogs: Synthesis and Properties (Methods in Molecular Biology: Volume 20, 1993).

### Step 2

With the sense strand and the antisense strand of wt926 obtained in step 1 above and step 1 of Reference Example 26, wt926 was obtained according to the method disclosed in, for example, Protocols for Oligonucleotides and Analogs: Synthesis and Properties (Methods in Molecular Biology: Volume 20, 1993).

**[Table 7]**

| si RNA | **SEQ ID NO** | **Strand** | **Sequence (5'→3')** |
|---|---|---|---|
| wt924 | 40 | **Sense** | GUCAUCACACUGAAUACCAAU |
| | 41 | **Antisense** | TUUGGUAUUCAGUGUGAUGACAT |
| wt925 | 40 | **Sense** | GUCAUCACACUGAAUACCAAU |
| | 42 | **Antisense** | TsUUGGUAUUCAGUGUGAUGACAT |
| wt926 | 40 | **Sense** | GUCAUCACACUGAAUACCAAU |
| | 43 | **Antisense** | pTUUGGUAUUCAGUGUGAUGACAT |

### (In the above table, A, G, C, T and U represent adenosine, guanosine, cytidine, thymidine and uridine, respectively; s represents a phosphorothioate bond; and p represents a 5'-phosphate group.)

### Reference Example 29

### Step 1

The sense strand (SEQ ID NO: 47) of wt927 was synthesized according to the method disclosed in, for example, Protocols for Oligonucleotides and Analogs: Synthesis and Properties (Methods in Molecular Biology: Volume 20, 1993).

### Step 2

The antisense strand (SEQ ID NO: 48) of wt927 was synthesized according to the method disclosed in, for example, Protocols for Oligonucleotides and Analogs: Synthesis and Properties (Methods in Molecular Biology: Volume 20, 1993).

### Step 3

With the sense strand and the antisense strand of wt927 obtained in steps 1 and 2, wt927 was obtained according to the method disclosed in, for example, Protocols for Oligonucleotides and Analogs: Synthesis and Properties (Methods in Molecular Biology: Volume 20, 1993).

### Reference Example 30

### Step 1

The antisense strand (SEQ ID NO: 49) of wt928 was synthesized according to the method disclosed in, for example, Protocols for Oligonucleotides and Analogs: Synthesis and Properties (Methods in Molecular Biology: Volume 20, 1993).

### Step 2

With the antisense strand and the sense strand of wt928 obtained in step 1 above and step 1 of Reference Example 29, wt928 was obtained according to the method disclosed in, for example, Protocols for Oligonucleotides and Analogs: Synthesis and Properties (Methods in Molecular Biology: Volume 20, 1993).

### Reference Example 31

### Step 1

The antisense strand (SEQ ID NO: 50) of wt929 was obtained in the same manner as in step 1 in Reference Example 28.

### Step 2

With the antisense strand and the sense strand of wt929 obtained in step 1 above and step 1 of Reference Example 29, wt929 was obtained according to the method disclosed in, for example, Protocols for Oligonucleotides and Analogs: Synthesis and Properties (Methods in Molecular Biology: Volume 20, 1993).

**[Table 8]**

| si RNA | **SEQ ID NO** | **Strand** | **Sequence (5'→3')** |
|---|---|---|---|
| wt927 | 47 | **Sense** | CCGUCGUAUUCGUGAGCAAGA |
| | 48 | **Antisense** | TUGCUCACGAAUACGACGGUT |
| wt928 | 47 | **Sense** | CCGUCGUAUUCGUGAGCAAGA |
| | 49 | **Antisense** | TsUGCUCACGAAUACGACGGUT |
| wt929 | 47 | **Sense** | CCGUCGUAUUCGUGAGCAAGA |
| | 50 | **Antisense** | pTUGCUCACGAAUACGACGGUT |

(In the above table, A, G, C, T and U represent adenosine, guanosine, cytidine, thymidine and uridine, respectively; s represents a phosphorothioate bond; and p represents a 5'-phosphate group.)

### Reference Example 32

### Step 1

The sense strand (SEQ ID NO: 54) of wt930 was synthesized according to the method disclosed in, for example, Protocols for Oligonucleotides and Analogs: Synthesis and Properties (Methods in Molecular Biology: Volume 20, 1993).

### Step 2

The antisense strand (SEQ ID NO: 55) of wt930 was synthesized according to the method disclosed in, for example, Protocols for Oligonucleotides and Analogs: Synthesis and Properties (Methods in Molecular Biology: Volume 20, 1993).

### Step 3

With the sense strand and the antisense strand of wt930 obtained in steps 1 and 2, wt930 was obtained according to the method disclosed in, for example, Protocols for Oligonucleotides and Analogs: Synthesis and Properties (Methods in Molecular Biology: Volume 20, 1993).

### Reference Example 33

### Step 1

The antisense strand (SEQ ID NO: 56) of wt931 was synthesized according to the method disclosed in, for example, Protocols for Oligonucleotides and Analogs: Synthesis and Properties (Methods in Molecular Biology: Volume 20, 1993).

### Step 2

With the antisense strand and the sense strand of wt931 obtained in step 1 above and step 1 of Reference Example 29, wt931 was obtained according to the method disclosed in, for example, Protocols for Oligonucleotides and Analogs: Synthesis and Properties (Methods in Molecular Biology: Volume 20, 1993).

### Reference Example 34

### Step 1

The antisense strand (SEQ ID NO: 57) of wt932 was synthesized according to the method disclosed in, for example, Protocols for Oligonucleotides and Analogs: Synthesis and Properties (Methods in Molecular Biology: Volume 20, 1993).

### Step 2

With the antisense strand and the sense strand of wt932 obtained in step 1 above and step 1 of Reference Example 29, wt932 was obtained according to the method disclosed in, for example, Protocols for Oligonucleotides and Analogs: Synthesis and Properties (Methods in Molecular Biology: Volume 20, 1993).

**[Table 9]**

| si RNA | **SEQ ID NO** | **Strand** | **Sequence (5'→3')** |
|---|---|---|---|
| wt930 | 54 | **Sense** | CCUUCAUUGACCUCAACUACA |
| | 55 | **Antisense** | TAGUUGAGGUCAAUGAAGGGT |
| wt931 | 54 | **Sense** | CCUUCAUUGACCUCAACUACA |
| | 56 | **Antisense** | TsAGUUGAGGUCAAUGAAGGGT |
| wt932 | 54 | **Sense** | CCUUCAUUGACCUCAACUACA |
| | 57 | **Antisense** | pTAGUUGAGGUCAAUGAAGGGT |

(In the above table, A, G, C, T and U represent adenosine, guanosine, cytidine, thymidine and uridine, respectively; s represents a phosphorothioate bond; and p represents a 5'-phosphate group.)

### Reference Example 35

The oligonucleotide CD45 ABC (SEQ ID NO: 9) targeting CD45 was obtained in the same manner as in steps 1 and 2 in Example 5.

The sulfurizing agent used during production of phosphorothioate was 3-((N,N-dimethylaminomethylidene)amino)-3H-1,2,4-dithiazole-5-thione (DDTT) in a mixed solvent of pyridine and acetonitrile and the reaction was carried out for 5 minutes.

### Reference Example 36

The oligonucleotide CD45 x2PO (SEQ ID NO: 10) targeting CD45 was obtained in the same manner as in Reference Example 35.

**[Table 10]**

| **Oligonucleotide** | **SEQ ID NO** | **Sequence (5'→3')** |
|---|---|---|
| CD45 ABC | 9 | mCsmCsmAsmAsAsTsGsCsCsAsAsGsmAsmGsmUsT |
| CD45 X2PO | 10 | mCsmCsmAsmAsmAsTGsCsCsAsAsGsmAsmGsmUT |

(In the above table, A, G, C and T represent 2'-deoxyadenosine, 2'-deoxyguanosine, 2'-deoxycytidine and thymidine, respectively; mA, mG, mC and mU respectively are as defined above; and s represents a phosphorothioate bond.)

### Reference Example 37

The oligonucleotide FGFR4 PS (SEQ ID NO: 12) targeting FGFR4 was obtained in the same manner as in Reference Example 35.

### Reference Example 38

The oligonucleotide FGFR4 PO (SEQ ID NO: 13) targeting FGFR4 was obtained in the same manner as in Reference Example 35.

**[Table 11]**

| **Oligonucleotide** | **SEQ ID NO** | **Sequence (5'→3')** |
|---|---|---|
| FGFR4 PS | 12 | (T)s[C]s(T)s[C]s(T)sTsTsGsGsTsCsAsCsAsCs[C]s[G]s(T)s[C]sT |
| FGFR4 PO | 13 | (T)s[C]s(T)s[C]s(T)sTsTsGsGsTCsAsCsAsCs[C]s[G]s(T)s[C]sT |

{In the above table, A, G, C and T represent 2'-deoxyadenosine, 2'-deoxyguanosine, 2'-deoxycytidine and thymidine, respectively; s is as defined above; [ ] represents that a nucleoside in the [ ] has methoxy at the 2'-position; and ( ) represents that a nucleoside in the ( ) has LNA at a ribose moiety.}

### Reference Example 39

The oligonucleotide wtKON708 (SEQ ID NO: 15) targeting PTEN was obtained in the same manner as in Reference Example 35.

### Reference Example 40

The oligonucleotide wtKON715 (SEQ ID NO: 16) targeting PTEN was obtained in the same manner as in Reference Example 35.

**[Table 12]**

| **Oligonucleotide** | **SEQ ID NO** | **Sequence (5'→3')** |
|---|---|---|
| wtKON708 | 15 | mTsmTsdAsdGsdCsdAsdCsTsdGsdGsmCsmCsT |
| wtKON715 | 16 | mUmUsdAsdGsdCsdAsdCsTsdGsdGsmCsmCsT |

(In the above table, T represents thymidine; mC, mU, mT and s respectively are as defined above; and dA, dG and dC represent 2'-deoxyadenosine, 2'-deoxyguanosine and 2'-deoxycytidine, respectively.)

### Example 1

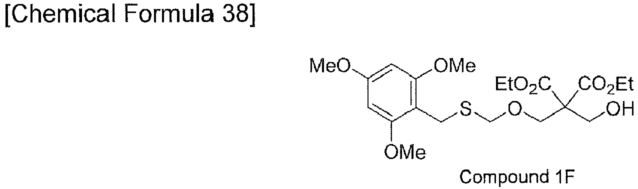

(wherein Me represents methyl; and Et represents ethyl.)

### Diethyl

### 2-(hydroxymethyl)-2-((((2,4,6-trimethoxybenzyl)thio)methoxy)methyl)malonate (compound 1 F)

Compound 1 E (3.78 g, 6.74 mmol) obtained in step 4 of Reference Example 1 was dissolved in THF (35 mL) to which triethylamine trihydrofluoride (3.26 g, 20.2 mmol) was added and stirred at room temperature for 3 days. A 2 mol/L triethylamine acetate aqueous solution and water was added to the reaction solution and it was extracted with ethyl acetate. The organic layer was washed with water and saturated saline, dried over anhydrous sodium sulphate, and the solvent was distilled off under reduced pressure. The residue was purified by silica gel column chromatography (hexane/ethyl acetate) to give compound 1 F (2.43 g, 81%).
ESI-MS (m/z): 447 (M+1)
¹H-NMR (CDCl₃, 400 MHz) δ: 6.12 (s, 2H), 4.70 (s, 2H), 4.23 (q, J = 7.1 Hz, 4H), 4.07 (d, J = 7.0 Hz, 2H), 4.00 (s, 2H), 3.84 (s, 2H), 3.82 (s, 6H), 3.81 (s, 3H), 2.71 (t, J = 7.0 Hz, 1 H), 1.27 (t, J = 7.1 Hz, 3H).

### Example 2

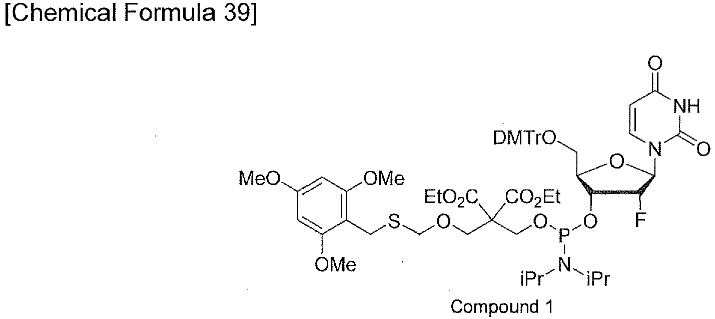

[wherein Me and Et respectively are as defined above; iPr represents isopropyl; and DMTr represents di(p-methoxyphenyl)phenylmethyl.]
Diethyl-9-((2R,3R,4R,5R)-2-((bis(4-methoxyphenyl)(phenyl)methoxy)methyl)-5-(2,4-d ioxo-3,4-dihydropyrimidin-1(2H)-yl)-4-fluorotetrahydrofuran-3-yloxy)-10-isopropyl-11-methyl-1-(2,4,6-trimethoxyphenyl)-4,8-dioxa-2-thia-10-aza-9-phosphadodecane-6,6-carboxylate (compound 1)

Compound 1F (2.43 g, 5.44 mmol) obtained in Example 1 and compound 2B (5.51 g, 7.07 mmol) obtained in Reference Example 2 were dissolved in acetonitrile (31 mL) to which 1 H-tetrazole (0.762 g, 10.9 mmol) was added at room temperature and stirred at room temperature for 1 hour. Water was added to the reaction solution and it was extracted with ethyl acetate. The organic layer was washed with water and saturated saline, dried over anhydrous sodium sulphate, and the solvent was distilled off under reduced pressure. The residue was purified by silica gel column chromatography (hexane/ethyl acetate) to give compound 1 (3.18 g, 52%).
ESI-MS (m/z): 1125 (M+1)
¹H-NMR (CDCl₃, 400 MHz) δ (ppm): 7.96 - 8.06 (m, 2H), 7.13 - 7.43 (m, 12H), 6.81 - 6.86 (m, 4H), 6.01 - 6.10 (m, 2H), 4.90 - 5.30 (m, 2H), 4.48 - 4.55 (m, 2H), 3.92 - 4.27 (m, 9H), 3.75 - 3.80 (m, 15H), 3.43 - 3.70 (m, 5H), 1.05 - 1.27 (m, 18H).

### Example 3

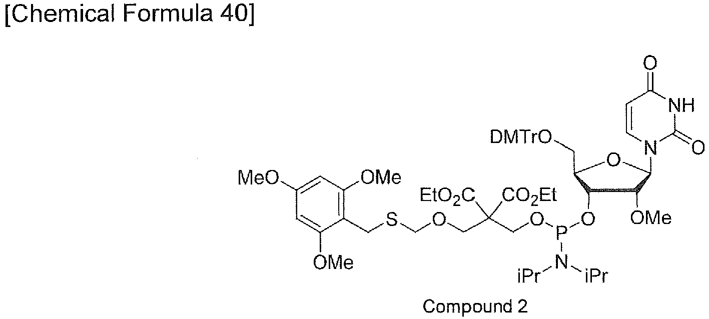

(wherein Me, Et, iPr and DMTr respectively are as defined above.)
Diethyl-9-((2R,3R,4R,5R)-2-((bis(4-methoxyphenyl)(phenyl)methoxy)methyl)-5-(2,4-d ioxo-3,4-dihydropyrimidin-1 (2H)-yl)-4-methoxytetrahydrofuran-3-yloxy)-l 0-isopropyl-11-methyl-1-(2,4,6-trimethoxyphenyl)-4,8-dioxa-2-thia-10-aza-9-phosphadodecane-6 ,6-carboxylate (compound 2)

Compound 2 (0.298 g, 24%) was obtained in the same manner as in step 1 in Example 2 using compound 1F (0.498 g, 1.12 mmol) obtained in Example 1 and compound 3B (0.970 g, 1.23 mmol) obtained in Reference Example 3. ESI-MS (m/z): 1137 (M+1)
¹H-NMR (CDCl₃, 400 MHz) δ (ppm): 7.89 - 8.08 (m, 2H), 7.20 - 7.52 (m, 12H), 6.78 - 6.86 (m, 4H), 6.10 (s, 1.5H), 6.09 (s, 0.5H), 5.93 - 5.99 (m, 1H), 5.18 - 5.28 (m, 1H), 4.38 - 4.60 (m, 2H), 3.91 - 4.31 (m, 7H), 3.40 - 3.80 (25H), 0.96 - 1.28 (m, 18H).

### Example 4

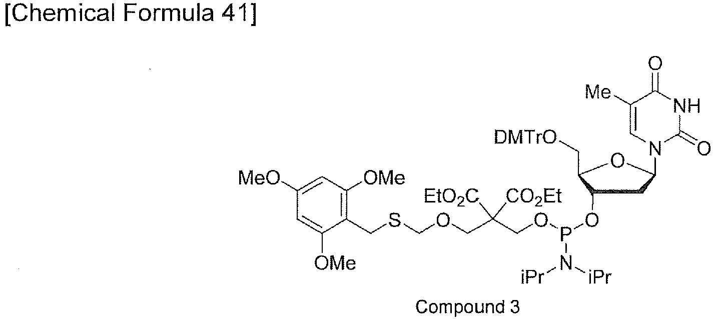

(wherein Me, Et, iPr and DMTr respectively are as defined above.)
Diethyl-9-((2R,3S,5R)-2-((bis(4-methoxyphenyl)(phenyl)methoxy)methyl)-5-(5-methyl -2,4-dioxo-3,4-dihydropyrimidin-1(2H)-yl)-tetrahydrofuran-3-yloxy)-10-isopropyl-11-m ethyl-1-(2,4,6-trimethoxyphenyl)-4,8-dioxa-2-thia-10-aza-9-phosphadodecane-6,6-ca rboxylate (compound 3)

Compound 3 (0.458 g, 46%) was obtained in the same manner as in step 1 in Example 2 using compound 1 F (0.400 g, 0.896 mmol) obtained in Example 1 and compound 4B (0.902 g, 1.17 mmol) obtained in Reference Example 4.
ESI-MS (m/z): 1121 (M+1)
¹H-NMR (CDCl₃, 400 MHz) δ (ppm): 7.92 (br s, 1H), 7.48 - 7.63 (m, 1H), 7.20 - 7.41 (m, 12H), 6.80 - 6.84 (m, 4H), 6.36 - 6.40 (m, 1H), 6.10 (s, 1.5H), 6.10 (s, 0.5H), 4.50 - 4.70 (m, 3H), 3.95 - 4.26 (m, 8H), 3.28 - 3.84 (m, 22H), 2.26 - 2.53 (m, 2H), 1.02 - 1.44 (m, 18H).

### Example 5

siRNA (3-Y) targeting HPRT1 was synthesised according to the following steps 1 and 2.

### Step 1

Compound 1 obtained in Example 2 and a solid carrier, CPG 500 angstroms, dT-Q-CPG (Glen Research Corporation), were used. DMT-2'-O-TBDMS-rA(tac) amidite (SAFC-Proligo), DMT-2'-O-TBDMS-rG(tac) amidite (SAFC-Proligo), DMT-2'-O-TBDMS-rC(tac) amidite (SAFC-Proligo), and DMT-2'-O-TBDMS-rU amidite (SAFC-Proligo) were respectively prepared to be a 0.1 mol/L acetonitrile solution and phosphoramidite (Glen Research Corporation) was prepared to be a 0.1 mol/L acetonitrile solution. A nucleic acid synthesizer (produced by Nihon Techno Service Co., Ltd., hereinafter M-7) and 0.2 µmol of a solid carrier were used for synthesis. An activator of phosphoramidite used was Activator 42 (SAFC-Proligo) and the time for condensation was 10 minutes each. An oxidizing agent used for production of phosphate ester was a solution containing pyridine, THF, water and iodine (such as Oxidizer 0.02 M produced by Aldrich) and the reaction was carried out for 10 seconds. The product was treated with trichloroacetic acid for trityl-off followed by treatment with diisopropylamine, deprotection of a TBS (tert-butyldimethylsilyl) group with triethylamine trihydrofluoride and purification by reverse phase liquid chromatography (Waters, XBridge (R) C18, 4.6 mm × 250 mm, gradient with solution A: 0.1% triethylammonium acetate buffer and solution B: acetonitrile) to give the antisense strand (SEQ ID NO: 4) of siRNA (3-Y).
ESI-MS theoretical: 7196 measured: 7196.

### Step 2

Equal amounts of the antisense strand (SEQ ID NO: 4) and the sense strand (SEQ ID NO: 1) of siRNA (3-Y) were mixed, dissolved in a PBS buffer and left to stand at 85°C for 5 minutes. The reactant was gradually cooled and left to stand at 37°C for 1 hour to give siRNA (3-Y).

**[Table 13]**

| si RNA | **SEQ ID NO** | **Strand** | **Sequence (5'→3')** |
|---|---|---|---|
| 3-Y | 1 | **Sense** | GmCCmAGmACmUUmUGmUUmGGmAUmUAmGT |
| | 4 | **Antisense** | YAmAUmCCmAAmCAmAAmGUmCUmGGmCUT |

[In the above table, A, G, C, T and U represent adenosine, guanosine, cytidine, thymidine and uridine, respectively; mA, mG, mC and mU are respectively as defined above; and Y represents a residue corresponding to the compound represented by the following formula (wherein Me and Et are respectively as defined above).]

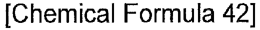

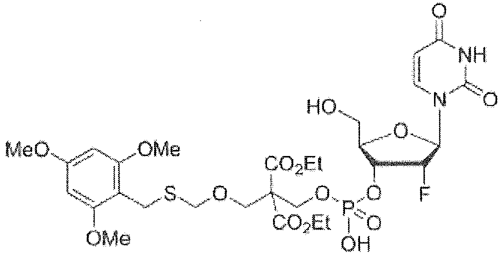

### Example 6

siRNA (4-Z) targeting HPRT1 was synthesized according to the following steps 1 and 2.

### Step 1

The antisense strand (150 µmol/L) of siRNA (3-Y) was dissolved in a 200 mmol/L acetate buffer (pH 4) to which dimethyl(methylthio)sulphonium tetrafluoroborate was added so as to be 30 mmol/L and left to stand at room temperature for 3 hours. Purification was carried out by reverse phase liquid chromatography (Waters, XBridge (R) C18, 4.6 mm x 250 mm, gradient with solution A: 0.1% triethylammonium acetate buffer and solution B: acetonitrile) to give the antisense strand (SEQ ID NO: 5) of siRNA (4-Z).
ESI-MS theoretical: 7061 measured: 7062.

### Step 2

With the antisense strand (SEQ ID NO: 5) and the sense strand (SEQ ID NO: 1) of siRNA (4-Z), siRNA (4-Z) was obtained in the same manner as in step 2 in Example 5.

**[Table 14]**

| si RNA | **SEQ ID NO** | **Strand** | **Sequence (5'→3')** |
|---|---|---|---|
| 4-Z | 1 | **Sense** | GmCCmAGmACmUUmUGmUUmGGmAUmUAmGT |
| | 5 | **Antisense** | ZAmAUmCCmAAmCAmAAmGUmCUmGGmCUT |

[In the above table, A, G, C, T and U represent adenosine, guanosine, cytidine, thymidine and uridine, respectively; mA, mG, mC and mU are respectively as defined above; and Z represents a residue corresponding to the compound represented by the following formula (wherein Me and Et are respectively as defined above).]

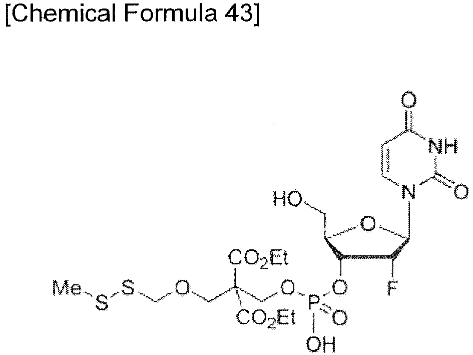

### Example 7

siRNA (5-Z) targeting HPRT1 was synthesized according to the following steps 1 and 2.

### Step 1

A precursor of the antisense strand (precursor of SEQ ID NO: 6) of siRNA (5-Z) having a residue corresponding to the structure represented by the following formula at 5'-terminal was obtained in the same manner as in step 1 in Example 5. ESI-MS theoretical: 7203 measured: 7202. 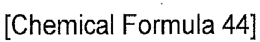 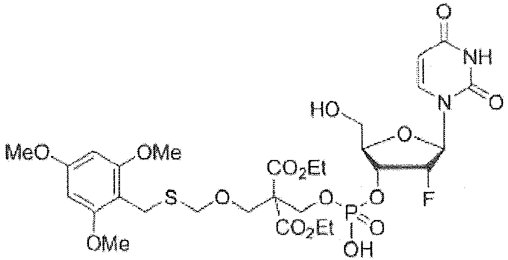 (wherein Me and Et are as defined above.)

### Step 2

From the precursor of the antisense strand (precursor of SEQ ID NO: 6) of siRNA (5-Z), the antisense strand (SEQ ID NO: 7) of siRNA (5-Z) was obtained in the same manner as in step 1 in Example 6.
ESI-MS theoretical: 7069 measured: 7068.

### Step 3

With the antisense strand (SEQ ID NO: 7) of siRNA (5-Z) and the sense strand (SEQ ID NO: 6) of siRNA (5-Z) in Reference Example 9, siRNA (5-Z) was obtained in the same manner as in step 2 in Example 5.

**[Table 15]**

| si RNA | **SEQ ID NO** | **Strand** | **Sequence (5'→3')** |
|---|---|---|---|
| 5-Z | 6 | **Sense** | Ch-GmCCmAGmACmUfUmUGmUfUmGGmAfUmUAmGT |
| | 7 | **Antisense** | ZAmAfUmCCmAAmCAmAAmGfUmCfUmGGmCfUT |

(In the above table, A, G, C, T and U represent adenosine, guanosine, cytidine, thymidine and uridine, respectively; and mA, mG, mC, mU, fU, Ch-G and Z are respectively as defined above.)

### Example 8

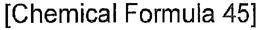

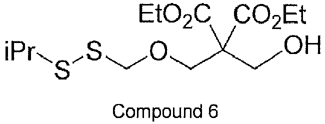

(wherein Et and iPr are as defined above.)

### Diethyl 2-(hydroxymethyl)-2-(((isopropyldisulfanyl)methoxy)methyl)malonate (compound 6)

Compound 6 (0.286 g, 78%) was obtained in the same manner as in step 1 in Example 1 using compound 12B (0.489 g, 1.08 mmol).
ESI-MS(m/z): 341 (M+1)
¹H-NMR (CDCl₃, 400 MHz) δ (ppm): 4.81 (s, 2H), 4.24 (q, J = 7.2 Hz, 4H), 4.11 (d, J = 6.3 Hz, 2H), 4.04 (s, 2H), 3.09 - 3.00 (m, 1 H), 2.46 - 2.26 (m, 1 H), 1.31 - 1.26 (m, 12H).

### Example 9

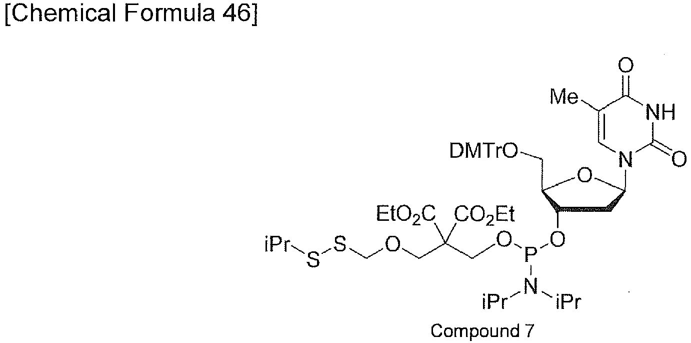

(wherein Me, Et, iPr and DMTr are respectively as defined above.)

### Diethyl

### 4-((2R,3S,5R)-2-((bis(4-methoxyphenyl)(phenyl)methoxy)methyl)-5-(5-methyl-2,4-dio xo-3,4-dihydropyrimidin-1(2H)-yl)tetrahydrofuran-3-yloxy)-3-isopropyl-2,13-dimethyl-5,9-dioxa-11,12-dithia-3-aza-4-phosphatetradecane-7,7-dicarboxylate (compound 7)

Compound 8 (0.380 g, 62%) was obtained in the same manner as in step 1 in Example 2 using compound 4B (0.605 g, 0.701 mmol) and compound 6 (0.205 g, 0.601 mmol).
ESI-MS (m/z): 1015 (M+1)
¹H-NMR (CDCl₃, 400 MHz) δ (ppm): 8.62 - 8.00 (m, 1 H), 7.50 - 7.25 (m, 9H), 6.89 - 6.83 (m, 4H), 5.98 - 5.88 (m, 1 H), 5.36 - 5.31 (m, 1 H), 5.00 (d, J = 3.3 Hz, 0.5H), 4.87 (d, J = 3.3 Hz, 0.5H), 4.77 - 4.48 (m, 2H), 4.28 - 3.93 (m, 10H), 3.81 - 3.79 (m, 6H), 3.66 - 3.42 (m, 4H), 3.00 - 2.76 (m, 4H), 1.28 - 1.06 (m, 23H).
³¹P-NMR (CDCl₃, 162 MHz) δ (ppm): 150.6, 149.1.

### Example 10

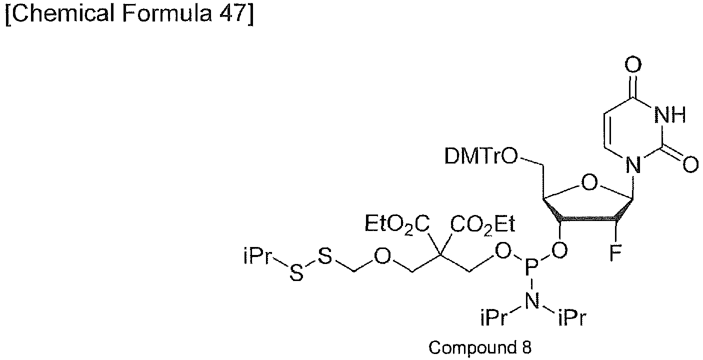

(wherein Et, iPr and DMTr are respectively as defined above.)

### Diethyl

### 2-((((((2R,3R,4R,5R)-2-((bis(4-methoxyphenyl)(phenyl)methoxy)methyl)-5-(2,4-dioxo -3,4-dihydropyrimidin-1(2H)-yl)-4-fluorotetrahydrofuranyl-3-yl)oxy)(diisopropylamino) phosphanyl)oxy)methyl)-2-(((isopropyldisulfanyl)methoxy)methyl)malonate (compound 8)

Compound 8 (98 mg, 23%) was obtained in the same manner as in step 1 in Example 2 using compound 2B (422 mg, 542 µmol) and compound 6 (142 mg, 417 µmol).
ESI-MS (m/z): 1019 (M+1)
¹H-NMR (CDCl₃, 400 MHz) δ (ppm): 8.60 - 8.00 (m, 1H), 7.48 - 7.22 (m, 10H), 6.88 - 6.82 (m, 4H), 6.01 - 5.98 (m, 1 H), 5.34 - 5.31 (m, 1 H), 5.00 (d, J = 3.3 Hz, 0.5H), 4.87 (d, J = 3.3 Hz, 0.5H), 4.74 - 4.45 (m, 2H), 4.25 - 3.93 (m, 10H), 3.80 - 3.79 (m, 6H), 3.64 - 3.43 (m, 4H), 3.00 - 2.87 (m, 1 H), 1.28 - 1.06 (m, 23H).
³¹P-NMR (CDCl₃, 162 MHz) δ (ppm): 150.9, 149.3.

### Example 11

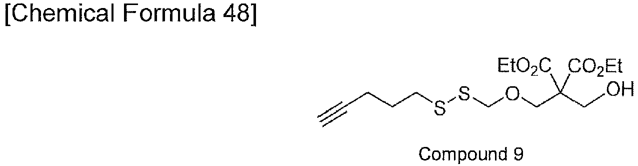

(wherein Et is as defined above.)

### Diethyl 2-(hydroxymethyl)-2-(((pent-4-ynyldisulfanyl)methoxy)methyl)malonate (compound 9)

Compound 9 (0.438 g, 93%) was obtained in the same manner as in Example 1 using compound 13A (0.615 g, 1.29 mmol).
ESI-MS (m/z): 365 (M+1)
¹H-NMR (CDCl₃, 400 MHz) δ (ppm): 4.83 (s, 2H), 4.24 (q, J = 7.2 Hz, 4H), 4.13 (d, J = 6.8 Hz, 2H), 4.04 (s, 2H), 3.00 (t, J = 6.8 Hz, 1 H), 2.85 (t, J = 7.2 Hz, 2H), 2.37 - 2.31 (m, 2H), 1.98 (t, J = 2.4 Hz, 1 H), 1.95 - 1.88 (m, 2H), 1.28 (t, J = 7.2 Hz, 6H).

### Example 12

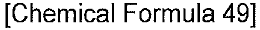

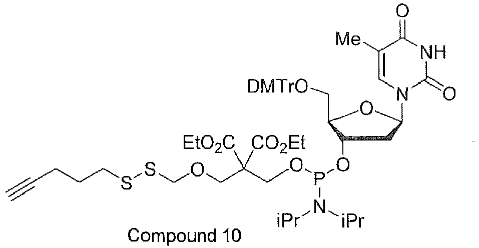

(wherein Me, Et, iPr and DMTr are respectively as defined above.)

### Diethyl

### 4-((2R,3S,5R)-2-((bis(4-methoxyphenyl)(phenyl)methoxy)methyl)-5-(5-methyl-2,4-dio xo-3,4-dihydropyrimidin-1(2H)-yl)tetrahydrofuran-3-yloxy)-3-isopropyl-2-methyl-5,9-di oxa-11,12-dithia-3-aza-4-phosphaheptadec-16-yn-7,7-dicarboxylate (compound 10)

Compound 10 (44 mg, 28%) was obtained in the same manner as in step 1 in Example 2 using compound 4B (0.152 g, 0.196 mmol) and compound 9 (55.0 mg, 0.151 mmol).
ESI-MS (m/z): 1038 (M+1)
¹H-NMR (CDCl₃, 400 MHz) δ (ppm): 8.09 - 7.86 (m, 2H), 7.50 - 7.20 (m, 10H), 6.88 - 6.79 (m, 4H), 6.06 - 6.02 (m, 1 H), 5.42 - 5.32 (m, 1 H), 5.33 (d, J = 8.2 Hz, 1 H), 5.01 (d, J = 3.8 Hz, 0.5H), 4.88 (d, J = 3.8 Hz, 0.5H), 4.72 (dd, J = 10.1, 11.2 Hz, 2H), 4.58 - 4.42 (m, 1H), 4.20 - 3.88 (m, 10H), 3.80 - 3.77 (m, 6H), 3.62 - 3.43 (m, 4H), 2.71 - 2.55 (m, 2H), 2.25 - 2.01 (m, 3H), 1.37 - 1.06 (m, 16H), 0.90 - 0.84 (m, 4H).
³¹P-NMR (CDCl₃, 162 MHz) δ (ppm): 150.6, 149.5.

### Example 13

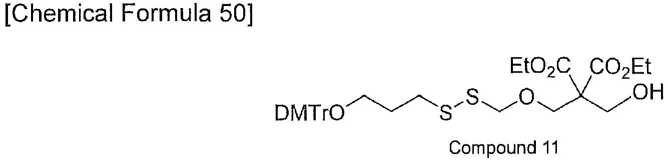

(wherein Et and DMTr are respectively as defined above.)

### Diethyl

### 2-((((3-(bis(4-methoxyphenyl)(phenyl)methoxy)propyl)disulfanyl)methoxy)methyl)-2-( hydroxymethyl)malonate (compound 11)

Compound 11 (0.143 g, 80%) was obtained in the same manner as in Example 1 using compound 14B (0.210 g, 0.272 mmol).
ESI-MS (m/z): 657 (M-1)
¹H-NMR (CDCl₃, 400 MHz) δ (ppm): 7.43 - 7.40 (m, 2H), 7.32 - 7.27 (m, 6H), 7.23 - 7.18 (m, 1 H), 6.84 - 6.80 (m, 4H), 4.79 (s, 2H), 4.22 (q, J = 7.0 Hz, 4H), 4.09 (d, J = 6.6 Hz, 2H), 4.02 (s, 2H), 3.79 (s, 6H), 3.15 (t, J = 6.1 Hz, 2H), 2.85 (t, J = 7.2 Hz, 2H), 2.37 (t, J = 6.8 Hz, 1 H), 2.00 - 1.93 (m, 2H), 1.26 (t, J = 7.0 Hz, 6H).

### Example 14

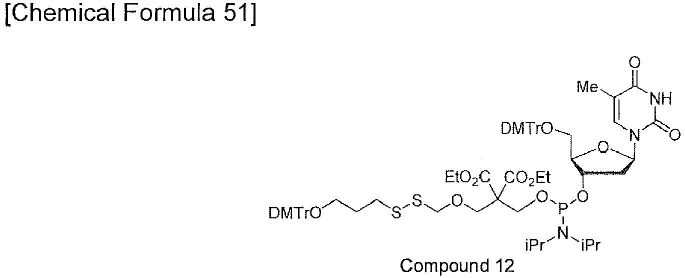

(wherein Me, Et, iPr and DMTr are respectively as defined above.)

### Diethyl 2-((((((2R, 3S, 5R)-2-((bis(4-methoxyphenyl)(phenyl)methoxy)methyl)-5-(5-methyl-2,4-dioxo-3,4-dih ydropyrimidin-1(2H)-yl)tetrahydrofuran-3-yl)oxy)(diisopropylamino)phosphanyl)oxy)m ethyl)-2-((((3-(bis(4-methoxyphenyl)(phenyl)methoxy)propyl)disulfanyl)methoxy)meth yl)malonate (compound 12)

Compound 12 (0.109 g, 42%) was obtained in the same manner as in Example 2 using compound 4B (0.199 g, 0.257 mmol) and compound 11 (0.130 g, 0.197 mmol).
ESI-MS (m/z): 1355 (M+Na)
¹H-NMR (CDCl₃, 400 MHz) δ (ppm): 7.89 - 7.19 (m ,19H), 6.85 - 6.79 (m, 8H), 6.40 - 6.35 (m, 1H), 4.78 - 4.57 (m, 3H), 4.19 - 3.93 (m, 9H), 3.80 - 3.76 (m, 12H), 3.57 - 3.27 (m, 4H), 3.16 - 3.11 (m, 2H), 2.82 - 2.76 (m, 2H), 2.52 - 2.24 (m, 4H), 1.97 - 1.88 (m, 2H), 1.44 - 1.38 (m, 3H), 1.29 - 1.00 (m, 18H).
³¹P-NMR (CDCl₃, 162 MHz) δ (ppm): 149.4, 147.8.

### Example 15

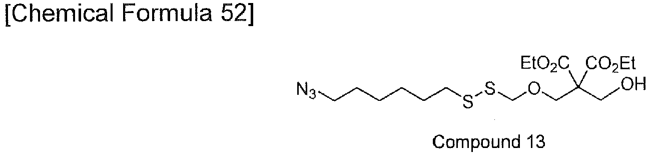

(wherein Et and N₃ are respectively as defined above.)

### Diethyl 2-((((6-azidohexyl)disulfanyl)methoxy)methyl)-2-(hydroxymethyl)malonate (compound 13)

Compound 15C (267 mg, 0.496 mmol) was dissolved in THF (2.5 mL) to which triethylamine trihydrofluoride (400 mg, 2.48 mmol) was added and stirred at room temperature for 7 days. Saturated sodium bicarbonate water was added to the reaction solution, the organic layer was then extracted with ethyl acetate, dried over anhydrous magnesium sulphate and concentrated under reduced pressure. The residue was purified by silica gel column chromatography (hexane/ethyl acetate) to give compound 13 (208 mg, 99%).
ESI-MS (m/z): 446 (M+Na)
¹H-NMR (CDCl₃, 400 MHz) δ (ppm): 4.82 (s, 2H), 4.24 (q, J = 7.2 Hz, 4H), 4.12 (d, J = 6.8 Hz, 2H), 4.04 (s, 2H), 3.27 (t, J = 6.8 Hz, 2H), 2.75 (t, J = 7.6 Hz, 2H), 2.42 (t, J = 6.8 Hz, 1 H), 1.73 - 1.58 (m, 4H), 1.47 - 1.34 (m, 4H), 1.28 (t, J = 7.2 Hz, 6H).

### Example 16

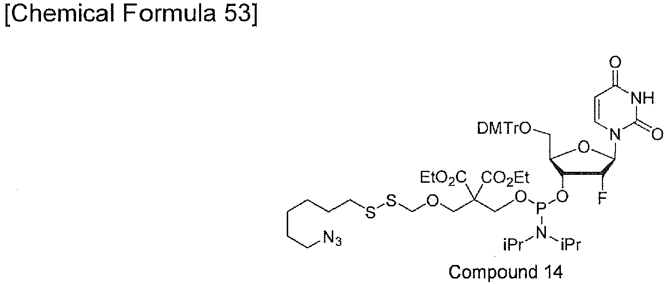

(wherein Et, iPr, DMTr and N₃ are respectively as defined above.)

### Diethyl

### 18-azido-4-((2R,3R,4R,5R)-2-((bis(4-methoxyphenyl)(phenyl)methoxy)methyl)-5-(2,4 -dioxo-3,4-dihydropyrimidin-1(2H)-yl)-4-fluorotetrahydrofuran-3-yloxy)-3-isopropyl-2-methyl-5,9-dioxa-11,12-dithia-3-aza-4-phosphaoctadecane-7,7-dicarboxylate (compound 14)

Compound 14 (0.058 g, 11 %) was obtained in the same manner as in step 1 in Example 2 using compound 2B (0.497 g, 0.638 mmol) and compound 13 (0.208 g, 0.491 mmol).
ESI-MS (m/z): 1124 (M+Na)
¹H-NMR (CDCl₃, 400 MHz) δ (ppm): 8.07 (d, J = 8.1 Hz, 1 H), 7.91 (br s, 1 H), 7.45 - 7.22 (m, 10H), 6.87 - 6.82 (m, 1H), 6.17 (d, , J = 16.0 Hz, 1 H), 5.32 (d, J = 8.2 Hz, 1 H), 5.00 (d, J = 3.8 Hz, 0.5H), 4.87 (d, J = 3.8 Hz, 0.5H), 4.70 (dd, J = 10.2, 11.3 Hz, 2H), 4.56 - 4.46 (m, 1 H), 4.23 - 3.93 (m, 10H), 3.80 - 3.79 (m, 6H), 3.62 - 3.23 (m, 6H), 2.71 - 2.59 (m, 2H), 1.40 - 1.11 (m, 25H).
³¹P-NMR (CDCl₃, 162 MHz) δ (ppm): 150.9.

### Example 17

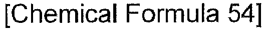

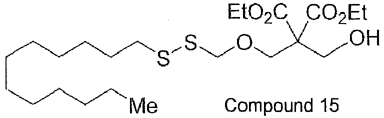

(wherein Me and Et are as defined above.)

### Diethyl 2-(((dodecyldisulfanyl)methoxy)methyl)-2-(hydroxymethyl)malonate (compound 19)

Compound 15 (371 mg, 100%) was obtained in the same manner as in Example 1 using compound 16A (463 mg, 0.797 mmol).
ESI-MS (m/z): 489 (M+Na)
¹H-NMR (CDCl₃, 400 MHz) δ (ppm): 4.82 (s, 2H), 4.24 (q, J = 7.2 Hz, 4H), 4.12 (d, J = 7.2 Hz, 2H), 4.04 (s, 2H), 2.74 (t, J = 7.6 Hz, 2H), 2.45 (t, J = 7.2 Hz, 1 H), 1.70 - 1.62 (m, 2H), 1.39 - 1.33 (m, 2H), 1.28 (t, J = 7.2 Hz, 6H), 1.30 - 1.26 (m, 16H), 0.88 (t, J = 6.4 Hz, 3H).

### Example 18

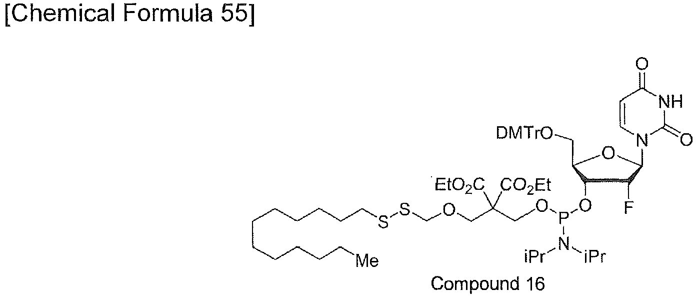

(wherein Me, Et, iPr and DMTr are respectively as defined above.)

### Diethyl

### 2-((((((2R,3R,4R,5R)-2-((bis(4-methoxyphenyl)(phenyl)methoxy)methyl)-5-(2,4-dioxo -3,4-dihydropyrimidin-1(2H)-yl)-4-fluorotetrahydrofuranyl-3-yl)oxy)(diisopropylamino) phosphanyl)oxy)methyl)-2-(((dodecylsulfanyl)methoxy)methyl)malonate (compound 16)

Compound 16 (62 mg, 22%) was obtained in the same manner as in Example 2 using compound 2B (339 mg, 0.436 mmol) and compound 15 (339 mg, 0.436 mmo).
ESI-MS (m/z): 1167 (M+Na)
¹H-NMR (CDCl₃, 400 MHz) δ (ppm): 8.08 - 7.92 (m, 2H), 7.52 - 7.22 (m, 10H), 6.87 - 6.82 (m, 4H), 6.06 - 6.00 (m, 1 H), 5.41 - 5.31 (m, 1 H), 5.32 (d, J = 8.2 Hz, 1 H), 5.00 (d, J = 3.8 Hz, 0.5H), 4.87 (d, J = 3.8 Hz, 0.5H), 4.70 (dd, J = 10.1, 11.2 Hz, 2H), 4.57 - 4.46 (m, 1 H), 4.22 - 3.93 (m, 10H), 3.80 - 3.79 (m, 6H), 3.62 - 3.43 (m, 4H), 2.74 - 2.57 (m, 2H), 1.35 - 1.06 (m, 34H), 0.90 - 0.84 (m, 4H).
³¹P-NMR (CDCl₃, 162 MHz) δ (ppm): 150.9, 149.2.

### Example 19

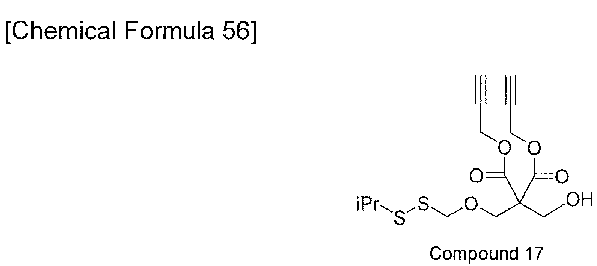

(wherein iPr is as defined above.)

### Di(prop-2-yn-1-yl)

### 2-(hydroxymethyl)-2-(((isopropyldisulfanyl)methoxy)methyl)malonate (compound 17)

Compound 17 (5.0 mg, 33%) was obtained in the same manner as in Example 1 using compound 17G (20.0 mg, 42.0 µmol).
¹H-NMR (CDCl₃, 400 MHz) δ (ppm): 4.81 (s, 2H), 4.78 (d, J = 2.5 Hz, 4H), 4.17 (d, J = 6.3 Hz, 2H), 4.09 (s, 2H), 3.04 (sep, J = 6.6 Hz, 1 H), 2.50 (t, J = 2.5 Hz, 2H), 2.37 (t, J = 6.3 Hz, 1 H), 1.31 (d, J = 6.6 Hz, 6H).

### Example 20

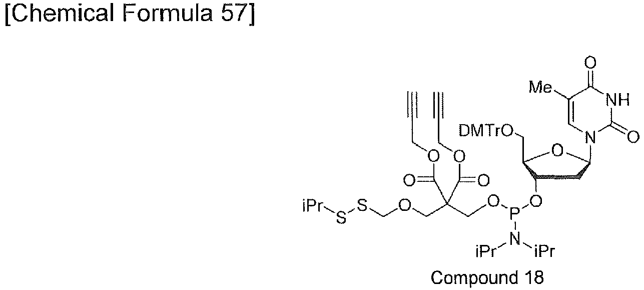

(wherein Me, iPr and DMTr are respectively as defined above.)

### Di(prop-2-yn-1-yl) 2-((((((2R, 3S, 5R)-2-((bis(4-methoxyphenyl)(phenyl)methoxy)methyl)-5-(5-methyl-2,4-dioxo-3,4-dih ydropyrimidin-1(2H)-yl)tetrahydrofuran-3-yl)oxy)(diisopropylamino)phosphanyl)oxy)m ethyl)-2-(((isopropyldisulfanyl)methoxy)methyl)malonate (compound 18)

Compound 18 (410 mg, 23%) was obtained in the same manner as in Example 2 using compound 17 (63.0 mg, 175 µmol) and compound 4B (176 mg, 227 µmol) obtained in Reference Example 4.
ESI-MS (m/z): 1034 (M+1)
¹H-NMR (CDCl₃, 400 MHz) δ (ppm): 8.04 (br s, 1H), 7.65 - 7.21 (m, 10H), 6.87 - 6.81 (m, 4H), 6.41 - 6.35 (m, 1 H), 4.83 - 4.59 (m, 7H), 4.21 - 3.98 (m, 5H), 3.81 - 3.77 (m, 6H), 3.62 - 3.26 (m, 4H), 3.03 - 2.94 (m, 1 H), 2.51 - 2.41 (m, 3H), 2.36 - 2.26 (m, 1 H), 1.46 - 1.38 (m ,3H), 1.28 - 1.02 (m, 18H).
³¹P-NMR (CDCl₃, 162 MHz) δ (ppm): 149.6, 148.2.

### Example 21

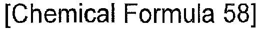

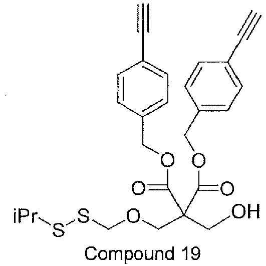

(wherein iPr is as defined above.)

### Bis(4-ethynylbenzyl)

### 2-(hydroxymethyl)-2-(((isopropyldisulfanyl)methoxy)methyl)malonate (compound 19)

Compound 19 (0.951 g, 83%) was obtained in the same manner as in Example 1 using compound 18G (1.41 g, 2.25 mmol).
ESI-MS (m/z): 513 (M+1)
¹H-NMR (CDCl₃, 400 MHz) δ (ppm): 7.46 - 7.41 (m, 4H), 7.23 - 7.19 (m, 4H), 5.15 (s, 4H), 4.75 (s, 2H), 4.16 (br d, J = 6.0 Hz, 2H), 4.07 (s, 2H), 3.10 (s, 2H), 3.01 (sep, J = 6.4 Hz, 1 H), 2.37 (br t, J = 6.0 Hz, 1 H), 1.28 (d, J = 6.4 Hz, 6H).

### Example 22

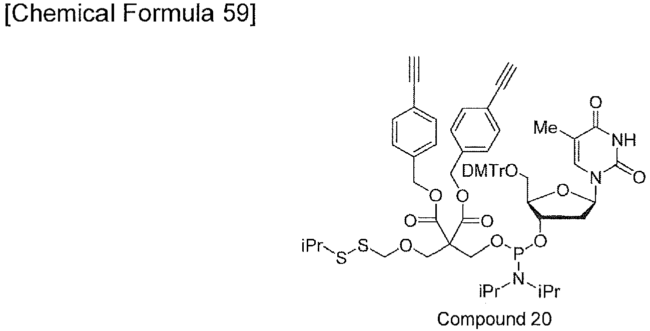

(wherein Me, iPr and DMTr are as defined above.)

### Bis(4-ethynylbenzyl)

### 2-((((((2R,3S,5R)-2-((bis(4-methoxyphenyl)(phenyl)methoxy)methyl)-5-(5-methyl-2,4-dioxo-3,4-dihydropyrimidin-1(2H)-yl)tetrahydrofuran-3-yl)oxy)(diisopropylamino)phos phanyl)oxy)methyl)-2-(((isopropyldisulfanyl)methoxy)methyl)malonate (compound 20)

Compound 20 (0.144 g, 42%) was obtained in the same manner as in Example 2 using compound 19 (0.150 g, 0.293 mmol) and compound 4B (0.295 g, 0.380 mmol).
ESI-MS (m/z): 1208 (M+Na)
¹H-NMR (CDCl₃, 400 MHz) δ (ppm): 7.90(br s, 1 H), 7.63 - 7.10 (m, 18H), 6.85 - 6.78 (m, 4H), 6.41 - 6.35 (m, 1 H), 5.11 - 4.95 (m, 4H), 4.75 - 4.57 (m, 3H), 4.19 - 4.00(6H), 3.79 - 3.75 (m ,6H), 3.54 - 3.26 (m, 3H), 3.10 - 3.07 (m, 2H), 3.00 - 2.91 (m, 1 H), 2.47 - 2.38 (m, 1 H), 2.34 - 2.23 (m, 1 H), 1.46 - 1.38 (m, 3H), 1.29 - 0.99 (m, 18H).
³¹P-NMR (CDCl₃, 162 MHz) δ (ppm): 149.5, 148.1.

### Example 23

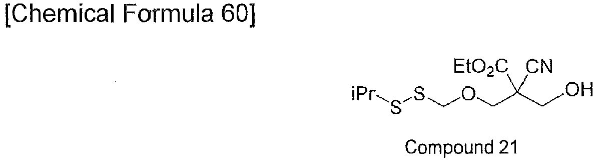

(wherein Et and iPr are as defined above.)

### Ethyl 2-cyano-3-hydroxy-2-(((isopropyldisulfanyl)methoxy)methyl)propanoate (compound 21)

Compound 21 (0.911 g, 85%) was obtained in the same manner as in Example 1 using compound 19F (1.48 g, 3.63 mmol).
¹H-NMR (CDCl₃, 400 MHz) δ (ppm): 4.89 (d, J = 11.2 Hz, 1H), 4.85 (d, J = 11.2 Hz, 1 H), 4.33 (q, J = 7.2 Hz, 2H), 4.10 - 4.01 (m, 2H), 4.01 (d, J = 9.4 Hz, 1 H), 3.98 (d, J = 9.4 Hz, 1 H), 3.13 - 3.03 (m, 1 H), 2.43 (br s, 1 H), 1.36 (t, J = 7.2 Hz, 3H), 1.31 (d, J = 6.4 Hz, 3H), 1.31 (d, J = 6.4 Hz, 3H).

### Example 24

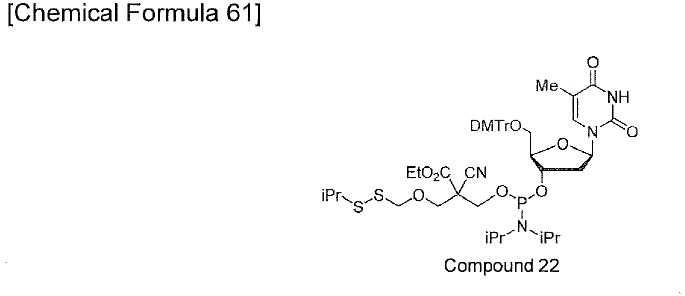

(wherein Me, Et, iPr and DMTr respectively are as defined above.)

### Ethyl

### 3-(((((2R,3S,5R)-2-((bis(4-methoxyphenyl)(phenyl)methoxy)methyl)-5-(5-methyl-2,4-dioxo-3,4-dihydropyrimidin-1(2H)-yl)tetrahydrofuran-3-yl)oxy)(diisopropylamino)phos phanyl)oxy)-2-cyano-2-(((isopropyldisulfanyl)methoxy)methyl)propanate (compound 22)

Compound 22 (0.420 g, 32%) was obtained in the same manner as in Example 2 using compound 21 (0.400 g, 1.36 mmol) and compound 4B (1.268 g, 1.636 mmol).
ESI-MS (m/z): 968 (M+1)
¹H-NMR (CDCl₃, 400 MHz) δ (ppm): 7.59 - 7.55 (m, 1 H), 7.43 - 7.37 (m, 2H), 7.33 - 7.26 (m, 6H), 6.85 - 6.82 (m, 4H), 6.41 - 6.37 (m, 1H), 4.90 - 4.77 (m, 2H), 4.71 - 4.65 (m, 1 H), 4.34 - 4.17 (m, 3H), 4.10 - 4.06 (m, 1 H), 4.04 - 3.82 (m, 4H), 3.79 (s, 6H), 3.61 - 3.45 (m, 3H), 3.38 - 3.28 (m, 1 H), 3.10 - 3.03 (m, 1 H), 2.48 - 2.35 (m, 2H), 1.46 - 1.41 (m, 2H), 1.39 - 1.24 (m, 11H), 1.20 - 1.12 (m, 8H), 1.07 - 1.02 (m, 4H).
³¹P-NMR (CDCl₃, 162 MHz) δ (ppm):150.6, 150.5.

### Example 25

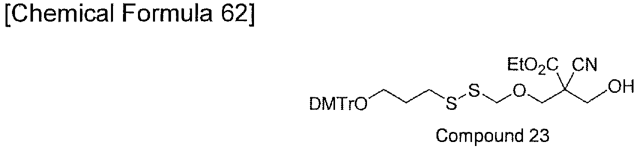

(wherein Et and DMTr are respectively as defined above.)

### Ethyl

### 3-(((3-(bis(4-methoxyphenyl)(phenyl)methoxy)propyl)disulfanyl)methoxy)-2-cyano-2-( hydroxymethyl)propanate (compound 23)

Compound 23 (2.15 g, 97%) was obtained in the same manner as in Example 1 using compound 20C (2.63 g, 3.62 mmol).
ESI-MS (m/z): 634 (M+Na)
¹H-NMR (CDCl₃, 400 MHz) δ (ppm): 7.43 - 7.40 (m, 2H), 7.32 - 7.26 (m, 6H), 7.23 - 7.18 (m, 1 H), 6.85 - 6.81 (m, 4H), 4.58 (dd, J = 21.6, 11.6 Hz, 2H), 4.34 - 4.26 (m, 2H), 4.06 - 3.96 (m, 2H), 3.95 (q, J = 7.2 Hz, 2H), 3.79 (s, 6H), 3.16 (t, J = 6.0 Hz, 2H), 2.88 (t, J = 6.8 Hz, 2H), 2.38 (t, J = 7.2 Hz, 1 H), 2.00 - 1.93 (m, 2H), 1.33 (t, J = 7.2 Hz, 3H).

### Example 26

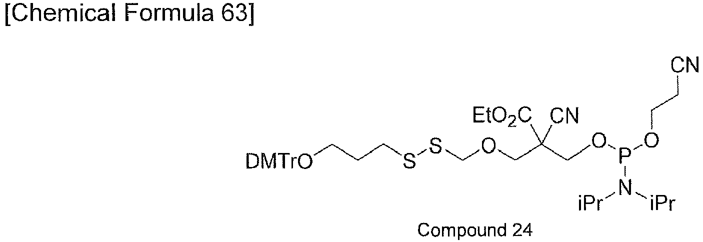

(wherein Et, iPr and DMTr are respectively as defined above.)

### Ethyl

### 3-(((3-(bis(4-methoxyphenyl)(phenyl)methoxy)propyl)disulfanyl)methoxy)-2-cyano-2-( (((2-cyanoethoxy)(diisopropylamino)phosphanyl)oxy)methyl)propanate (compound 24)

Compound 23 (166 mg, 0.271 mmol) was dissolved in THF (2.7 mL) to which N,N-diisopropylethylamine (0.237 mL, 1.36 mmol) and 3-((chloro(diisopropylamino)phosphanyl)oxy)propanenitrile (96.0 mg, 0.407 mmol) were added and stirred at room temperature for 30 minutes. Water was added to the reaction solution, the organic layer was extracted with ethyl acetate, washed with water and saturated saline, dried over anhydrous sodium sulphate and concentrated under reduced pressure. The residue was purified by silica gel column chromatography (hexane/ethyl acetate) to give compound 24 (133 mg, 60%).
ESI-MS (m/z): 835 (M+Na)
¹H-NMR (CDCl₃, 400 MHz) δ (ppm): 7.43 - 7.39 (m, 2H), 7.32 - 8.18 (m, 7H), 6.84 - 6.81 (m, 4H), 4.89 - 4.82 (m, 2H), 4.31 - 4.24 (m, 2H), 4.15 - 3.78 (m, 13 Hz), 3.65 - 3.55 (m, 2H), 3.18 - 3.13 (m, 2H), 2.90 - 2.86 (m, 2H), 2.00 - 1.93 (m, 5H), 1.34 - 1.16 (m, 13H).
³¹P-NMR (CDCl₃, 162 MHz) δ (ppm):150.2, 150.1.

### Example 27

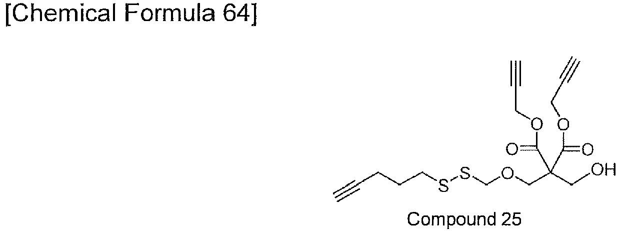

### Diisoprop-2-ynyl

### 2-(hydroxymethyl)-2-(((pent-4-ynyldisulfanyl)methoxy)methyl)malonate (compound 25)

Compound 25 (138 mg, 47%) was obtained in the same manner as in Example 1 using compound 21A (329 mg, 0.593 mmol).
ESI-MS (m/z): 407 (M+Na)
¹H-NMR (CDCl₃, 400 MHz) δ (ppm): 4.84 (s, 2H), 4.78 (d, J = 2.8 Hz, 4H), 4.17 (d, J = 6.8 Hz, 2H), 4.09 (s, 2H), 2.85 (t, J = 7.2 Hz, 2H), 2.50 (t, J = 2.8 Hz, 2H), 2.36 - 2.32 (m, 3H), 1.99 (t, J = 2.8 Hz, 1 H), 1.96 - 1.88 (m, 2H).

### Example 28

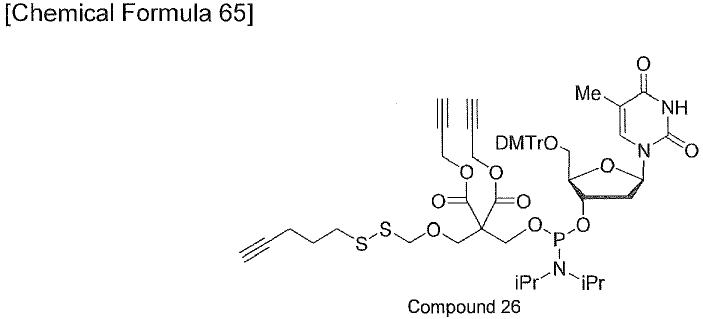

(wherein Me, iPr and DMTr are respectively as defined above.)

### Diisoprop-2-ynyl

### 4-((2R,3S,5R)-2-((bis(4-methoxyphenyl)(phenyl)methoxy)methyl)-5-(5-methyl-2,4-dio xo-3,4-dihydropyrimidin-1(2H)-yl)tetrahydrofuran-3-yloxy)-3-isopropyl-2-methyl-5,9-di oxa-11,12-dithia-3-aza-4-phosphaheptadec-16-yn-7,7-dicarboxylate (compound 26)

Compound 26 (175 mg, 58%) was obtained in the same manner as in Example 2 using compound 4B (288 mg, 0.372 mmol) and compound 25 (110 mg 0.286 mmol).
ESI-MS (m/z): 1180 (M+Na)
¹H-NMR (CDCl₃, 400 MHz) δ (ppm): 7.93 (br s, 1H), 7.64 - 7.58 (m, 1H), 7.42 - 7.22 (m, 10H), 6.86 - 6.81 (m, 4H), 6.42 - 6.36 (m, 1 H), 4.80 - 4.60 (m, 6H), 4.18 - 3.97 (m, 6H), 3.80 (s, 3H), 3.79 (s, 3H), 3.61 - 3.28 (m, 4H), 2.84 - 2.77 (m, 2H), 2.52 - 2.42 (m, 3H), 2.34 - 2.27 (m, 3H), 1.98 - 1.95 (m, 3H), 1.91 - 1.84 (m, 2H), 1.45 - 1.03 (m, 12H).
³¹P-NMR (CDCl₃, 162 MHz) δ (ppm): 149.6, 148.2.

### Example 29

The oligonucleotide CD45 x21C (SEQ ID NO: 11) targeting CD45 was obtained in the same manner as in step 1 in Example 5 and step 1 in Example 6 using compounds 2 and 3. The sulfurizing agent used during production of phosphorothioate was 3-((N,N-dimethylaminomethylidene)amino)-3H-1,2,4-dithiazole-5-thione (DDTT) in a mixed solvent of pyridine and acetonitrile and the reaction was carried out for 5 minutes.

**[Table 16]**

| **Oligonucleotide** | **SEQ ID NO** | **Sequence (5'→3')** |
|---|---|---|
| CD45 x2IC | 11 | mCsmCsmAsmAsmAsY¹GsCsCsAsAsGsmAsmGsZ¹T |

(In the above table, A, G, C and T represent 2'-deoxyadenosine, 2'-deoxyguanosine, 2'-deoxycytidine and thymidine, respectively; mA, mG and mC respectively are as defined above; s represents a phosphorothioate bond; and Y¹ and Z¹ represent a bivalent group represented by the following formulae, respectively.)

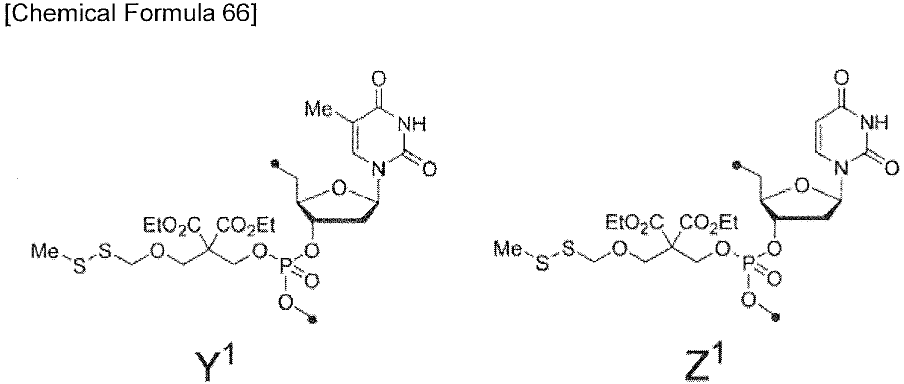

### Example 30

The oligonucleotide FGFR4 IC (SEQ ID NO: 14) targeting FGFR4 was obtained in the same manner as in Example 29 using compounds 2 and 3.

**[Table 17]**

| **Oligonucleotide** | **SEQ ID NO** | **Sequence (5'→3')** |
|---|---|---|
| FGFR4 IC | 14 | (T)s[C]s(T)s[C]s(T)sTsTsGsGsY¹CsAsCsAsCs[C]s[G]s(T)s[C]sT |

{In the above table, A, G, C and T represent 2'-deoxyadenosine, 2'-deoxyguanosine, 2'-deoxycytidine and thymidine, respectively; Y¹ and s respectively are as defined above; [ ] represents that a nucleoside in the [ ] has methoxy at the 2'-position; and () represents that a nucleoside in the () has LNA at a ribose moiety.}

### Example 31

The oligonucleotide KON708 (SEQ ID NO: 17) targeting PTEN was obtained in the same manner as in Example 29 using compound 3.

### Example 32

The oligonucleotide KON715 (SEQ ID NO: 18) targeting PTEN was obtained in the same manner as in step 1 in Example 5, step 1 in Example 6 and Example 29 using compound 3.

**[Table 18]**

| **Oligonucleotide** | **SEQ ID NO** | **Sequence (5'→3')** |
|---|---|---|
| KON708 | 17 | mTsmTsdAsdGsdCsdAsdCY²sdGsdGsmCsmCsT |
| KON715 | 18 | Z²mUsdAsdGsdCsdAsdCsTsdGsdGsmCsmCsT |

[In the above table, T represents thymidine; mC, mU, mT and s respectively are as defined above; dA, dG and dC represent 2'-deoxyadenosine, 2'-deoxyguanosine and 2'-deoxycytidine, respectively; Y² and Z² represent the bivalent group and the residue corresponding to the compound represented by the following formulae (wherein Me represents methyl; and Et represents ethyl), respectively.]

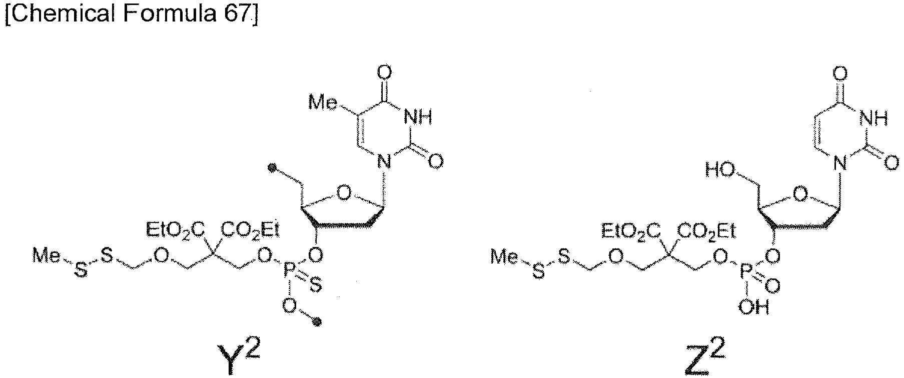

### Example 33

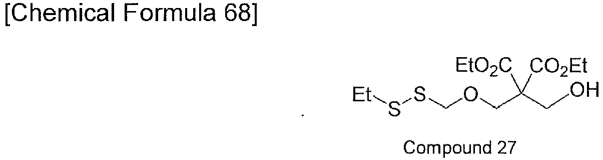

(wherein Et is as defined above.)

### Diethyl 2-(((ethyldisulfanyl)methoxy)methyl)-2-(hydroxymethyl)malonate (compound 27)

In an argon atmosphere, diethyl disulphide (0.440 mL, 3.57 mmol) was dissolved in acetonitrile anhydrous (2.0 mL) to which, after cooling on ice, trimethyloxonium tetrafluoroborate (527 mg, 3.57 mmol) was added and stirred under ice cooling for 2.5 hours. To the reaction solution was added a 0.4 mol/L sodium acetate buffer (0.72 mL) and stirred for 10 minutes. To the reaction solution was added dropwise a solution of compound 1E (200 mg, 0.357 mmol) in acetonitrile anhydrous (2.0 mL) under ice cooling and stirred for 1 hour while heating to room temperature. Water was added to the reaction solution, the organic layer was extracted with ethyl acetate, washed with saturated saline and dried over anhydrous sodium sulphate and the solvent was distilled off under reduced pressure. The residue was purified by silica gel column chromatography (n-heptane/ethyl acetate) to give compound 27 (10.0 mg, 52%).
¹H-NMR (CDCl₃, 400 MHz) δ (ppm): 4.83 (s, 2H), 4.24 (q, J = 7.3 Hz, 4H), 4.12 (br d, J = 6.0 Hz, 2H), 4.04 (s, 2H), 2.76 (q, J = 7.3 Hz, 2H), 1.32 (t, J = 7.3 Hz, 3H), 1.28 (t, J = 7.3 Hz, 6H).

### Example 34

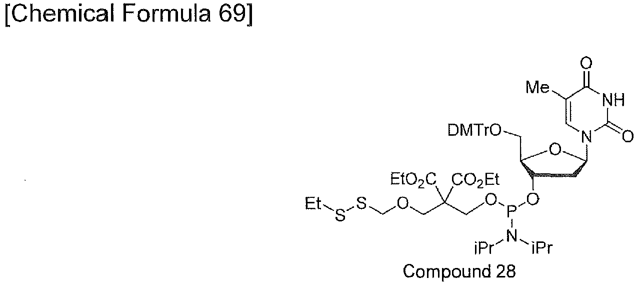

(wherein Me, Et, iPr and DMTr are respectively as defined above.)

### Diethyl

### 4-((2R,3S,5R)-2-((bis(4-methoxyphenyl)(phenyl)methoxy)methyl)-5-(5-methyl-2,4-dio xo-3,4-dihydropyrimidin-1(2H)-yl)tetrahydrofuran-3-yloxy)-3-isopropyl-2-methyl-5,9-di oxa-11,12-dithia-3-aza-4-phosphatetradecane-7,7-dicarboxylate (compound 28)

Compound 28 (0.130 g, 20%) was obtained in the same manner as in Example 2 using compound 4B (0.598 g, 0.772 mmol) and compound 27 (0.210 g, 0.643 mmol).
ESI-MS (m/z): 1001 (M+H).
¹H-NMR (CDCl₃, 400 MHz) δ (ppm): 7.97 (1 H, s), 7.65 - 7.54 (1 H, m), 7.43 - 7.38 (2H, m), 7.33 - 7.27 (6H, m), 7.20 - 7.14 (2H, m), 6.87 - 6.80 (4H, m), 6.42 - 6.36 (1 H, m), 4.84 - 4.61 (2H, m), 4.22 - 3.98 (7H, m), 3.79 (6H, s), 3.56 - 3.44 (2H, m), 3.40 - 3.28 (1H, m), 2.76 - 2.66 (2H, m), 2.51 - 2.43 (1H, m), 2.36 (3H, s), 2.34 - 2.27 (1H, m), 1.46 - 1.37 (2H, m), 1.32 - 1.13 (20H, m), 1.03 (2H, d, J = 6.8 Hz).
³¹P-NMR (CDCl₃, 162 MHz) δ (ppm): 149.6, 148.0.

### Example 35

### Step 1

The antisense strand (SEQ ID NO: 22) of KON789 was obtained in the same manner as in step 1 in Example 5 using compound 7.
ESI-MS theoretical: 6959 measured: 6959

### Step 2

KON789 was obtained in the same manner as in step 2 in Example 5 using the antisense strand of KON789.

**[Table 19]**

| si RNA | **SEQ ID NO** | **Strand** | **Sequence (5'→3')** |
|---|---|---|---|
| KON789 | 19 | **Sense** | GCCAGACUUUGUUGGAUUAGT |
| | 22 | **Antisense** | Z³AAUCCAACAAAGUCUGGCUT |

[In the above table, A, G, C, T and U represent adenosine, guanosine, cytidine, thymidine and uridine, respectively; and Z³ represents a residue corresponding to the compound represented by the following formula (wherein Me, Et and iPr are respectively as defined above).]

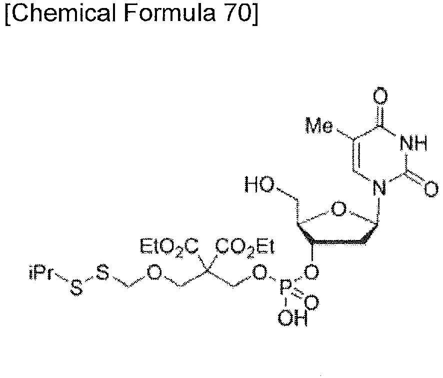

### Example 36

### Step 1

The antisense strand (SEQ ID NO: 23) of KON816 was obtained in the same manner as in step 1 in Example 5 using compound 28.

### ESI-MS theoretical: 6945 measured: 6944

### Step 2

KON816 was obtained in the same manner as in step 2 in Example 5 using the antisense strand of KON816.

**[Table 20]**

| si RNA | **SEQ ID NO** | **Strand** | **Sequence (5'→3')** |
|---|---|---|---|
| KON816 | 19 | **Sense** | GCCAGACUUUGUUGGAUUAGT |
| | 23 | **Antisense** | Y⁴AAUCCAACAAAGUCUGGCUT |

[In the above table, A, G, C, T and U represent adenosine, guanosine, cytidine, thymidine and uridine, respectively; and Y⁴ represents a residue corresponding to the compound represented by the following formula (wherein Me represents methyl; and Et represents ethyl).]

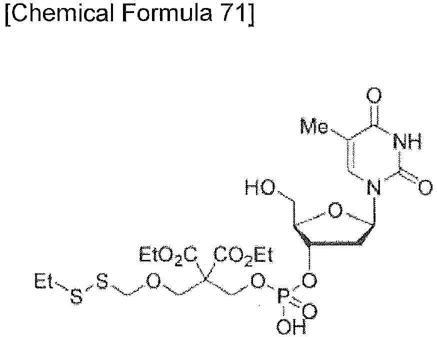

### Example 37

### Step 1

The antisense strand (SEQ ID NO: 24) of KON818 was obtained in the same manner as in step 1 in Example 5 using compound 10.
ESI-MS theoretical: 6983 measured: 6982

### Step 2

KON818 was obtained in the same manner as in step 2 in Example 5 using the antisense strand of KON818.

**[Table 21]**

| si RNA | **SEQ ID NO** | **Strand** | **Sequence (5'→3')** |
|---|---|---|---|
| KON818 | 19 | **Sense** | GCCAGACUUUGUUGGAUUAGT |
| | 24 | **Antisense** | Z⁴AAUCCAACAAAGUCUGGCUT |

[In the above table, A, G, C, T and U represent adenosine, guanosine, cytidine, thymidine and uridine, respectively; and Z⁴ represents a residue corresponding to the compound represented by the following formula (wherein Me represents methyl; and Et represents ethyl).]

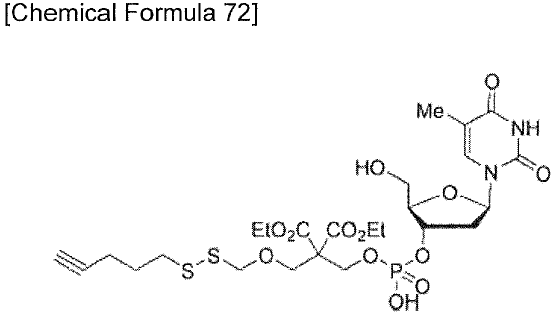

### Example 38

### Step 1

The antisense strand (SEQ ID NO: 26) of KON846 was obtained in the same manner as in step 1 in Example 5 using compound 18.
ESI-MS theoretical: 6980 measured: 6979

### Step 2

KON846 was obtained in the same manner as in step 2 in Example 5 using the antisense strand of KON846.

### Example 39

### Step 1

The antisense strand (SEQ ID NO: 27) of KON857 was obtained in the same manner as in step 1 in Example 5 using compound 22.
ESI-MS theoretical: 6898 measured: 6898

### Step 2

KON857 was obtained in the same manner as in step 2 in Example 5 using the antisense strand of KON857.

**[Table 22]**

| si RNA | **SEQ ID NO** | **Strand** | **Sequence (5'→3')** |
|---|---|---|---|
| KON846 | 19 | **Sense** | GCCAGACUUUGUUGGAUUAGT |
| | 26 | **Antisense** | Y⁵AAUCCAACAAAGUCUGGCUT |
| KON857 | 19 | **Sense** | GCCAGACUUUGUUGGAUUAGT |
| | 27 | **Antisense** | Z⁵AAUCCAACAAAGUCUGGCUT |

[In the above table, A, G, C, T and U represent adenosine, guanosine, cytidine, thymidine and uridine, respectively; and Y⁵ and Z⁵ represent residues corresponding to the compounds represented by the following formulae (wherein Me and iPr are respectively as defined above), respectively.]

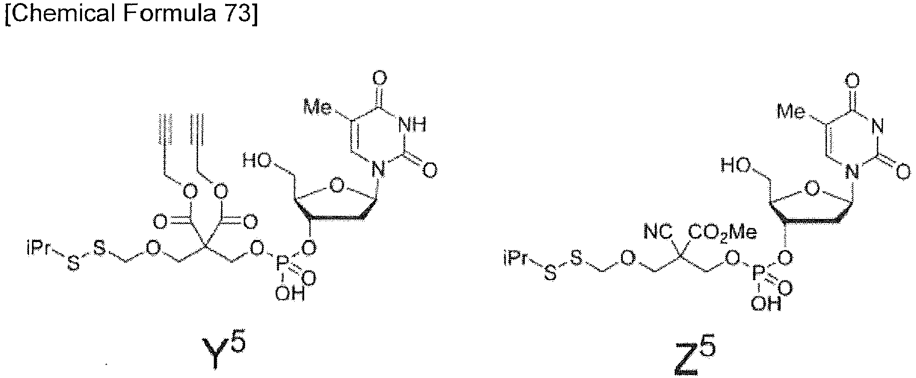

### Example 40

### Step 1

The antisense strand (SEQ ID NO: 29) of KON880 was obtained in the same manner as in step 1 in Example 5 using compound 16.
ESI-MS theoretical: 7090 measured: 7089

### Step 2

KON880 was obtained in the same manner as in step 2 in Example 5 using the antisense strand of KON880.

### Example 41

### Step 1

The antisense strand (SEQ ID NO: 30) of KON881 was obtained in the same manner as in step 1 in Example 5 using compound 8.
ESI-MS theoretical: 6963 measured: 6962

### Step 2

KON881 was obtained in the same manner as in step 2 in Example 5 using the antisense strand of KON881.

### Example 42

### Step 1

The antisense strand (SEQ ID NO: 31) of KON882 was obtained in the same manner as in step 1 in Example 5 and Example 29 using compound 22.
ESI-MS theoretical: 6914 measured: 6913

### Step 2

KON882 was obtained in the same manner as in step 2 in Example 5 using the antisense strand of KON882.

### Example 43

### Step 1

The antisense strand (SEQ ID NO: 32) of KON883 was obtained in the same manner as in step 1 in Example 5 using compound 24.
ESI-MS theoretical: 7012 measured: 7011

### Step 2

KON883 was obtained in the same manner as in step 2 in Example 5 using the antisense strand of KON883.

### Example 44

### Step 1

The antisense strand (SEQ ID NO: 33) of KON884 was obtained in the same manner as in step 1 in Example 5 and Example 29 using compound 24. ESI-MS theoretical: 7028 measured: 7027

### Step 2

KON884 was obtained in the same manner as in step 2 in Example 5 using the antisense strand of KON884.

**[Table 23]**

| si RNA | **SEQ ID NO** | **Strand** | **Sequence (5'→3')** |
|---|---|---|---|
| KON880 | 19 | **Sense** | GCCAGACUUUGUUGGAUUAGT |
| | 29 | **Antisense** | Y⁶AAUCCAACAAAGUCUGGCUT |
| KON881 | 19 | **Sense** | GCCAGACUUUGUUGGAUUAGT |
| | 30 | **Antisense** | Z⁶AAUCCAACAAAGUCUGGCUT |
| KON882 | 19 | **Sense** | GCCAGACUUUGUUGGAUUAGT |
| | 31 | **Antisense** | Y⁷AAUCCAACAAAGUCUGGCUT |
| KON883 | 19 | **Sense** | GCCAGACUUUGUUGGAUUAGT |
| | 32 | **Antisense** | Z⁷AAUCCAACAAAGUCUGGCUT |
| KON884 | 19 | Sense | GCCAGACUUUGUUGGAUUAGT |
| | 33 | Antisense | Y⁸AAUCCAACAAAGUCUGGCUT |

[In the above table, A, G, C, T and U represent adenosine, guanosine, cytidine, thymidine and uridine, respectively; Y⁶, Z⁶, Y⁷, Z⁷ and Y⁸ represent residues corresponding to the compounds represented by the following formulae (wherein Me, Et and iPr are respectively as defined above), respectively.]

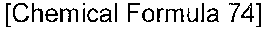

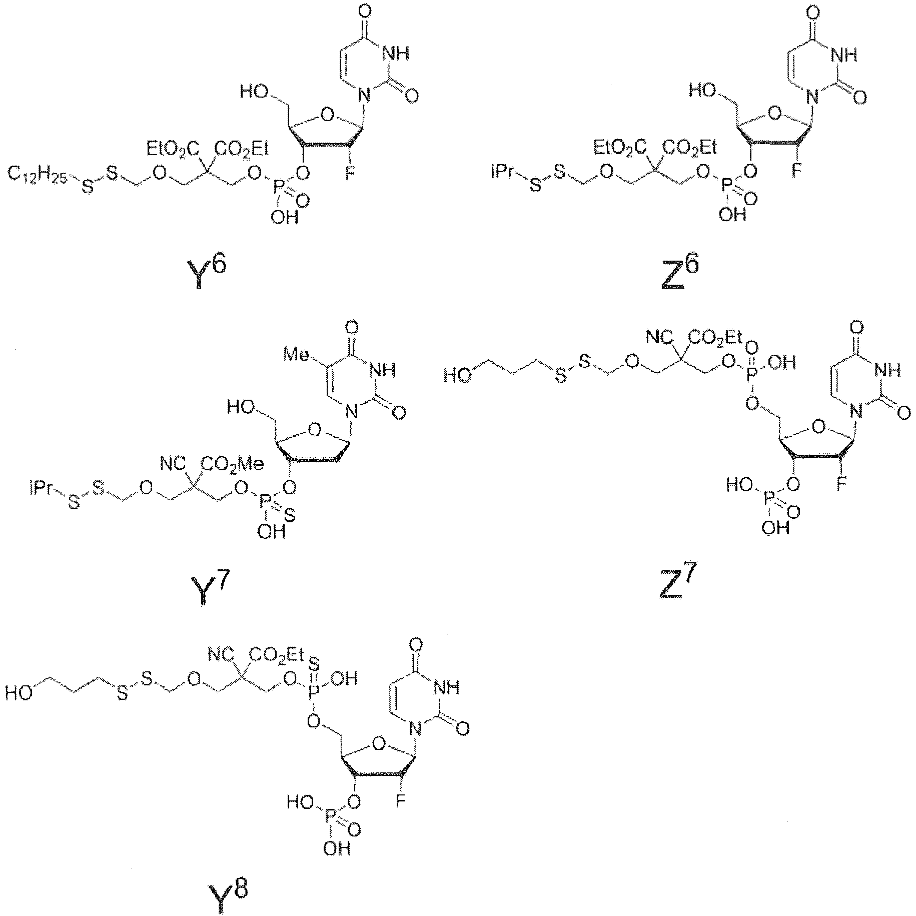

### Example 45

### Step 1

The antisense strand (SEQ ID NO: 34) of KON891 was obtained in the same manner as in step 1 in Example 5 using compound 20.
ESI-MS theoretical: 7132 measured: 7131

### Step 2

KON891 was obtained in the same manner as in step 2 in Example 5 using the antisense strand of KON891.

### Example 46

### Step 1

The antisense strand (SEQ ID NO: 35) of KON892 was obtained in the same manner as in step 1 in Example 5 using compound 12.
ESI-MS theoretical: 6976 measured: 6975

### Step 2

KON892 was obtained in the same manner as in step 2 in Example 5 using the antisense strand of KON892.

### Example 47

### Step 1

The antisense strand (SEQ ID NO: 36) of KON903 was obtained in the same manner as in step 1 in Example 5 using compound 14.
ESI-MS theoretical: 7047 measured: 7046

### Step 2

KON903 was obtained in the same manner as in step 2 in Example 5 using the antisense strand of KON903.

### Example 48

### Step 1

The antisense strand (SEQ ID NO: 37) of KON905 was obtained in the same manner as in step 1 in Example 5 and Example 29 using compound 18.
ESI-MS theoretical: 6996 measured: 6995

### Step 2

KON905 was obtained in the same manner as in step 2 in Example 5 using the antisense strand of KON905.

**[Table 24]**

| si RNA | **SEQ ID NO** | **Strand** | **Sequence (5'→3')** |
|---|---|---|---|
| KON880 | 19 | **Sense** | GCCAGACUUUGUUGGAUUAGT |
| | 29 | **Antisense** | Y⁶AAUCCAACAAAGUCUGGCUT |
| KON881 | 19 | **Sense** | GCCAGACUUUGUUGGAUUAGT |
| | 30 | **Antisense** | Z⁶AAUCCAACAAAGUCUGGCUT |
| KON882 | 19 | **Sense** | GCCAGACUUUGUUGGAUUAGT |
| | 31 | **Antisense** | Y⁷AAUCCAACAAAGUCUGGCUT |
| KON883 | 19 | **Sense** | GCCAGACUUUGUUGGAUUAGT |
| | 32 | **Antisense** | Z⁷AAUCCAACAAAGUCUGGCUT |
| KON884 | 19 | **Sense** | GCCAGACUUUGUUGGAUUAGT |
| | 33 | **Antisense** | Y⁸AAUCCAACAAAGUCUGGCUT |

[In the above table, A, G, C, T and U represent adenosine, guanosine, cytidine, thymidine and uridine, respectively; and Z⁸, Y⁹, Z⁹ and Y¹⁰ represent compounds represented by the following formulae (wherein Me, Et, iPr and N₃ are respectively as defined above), respectively.]

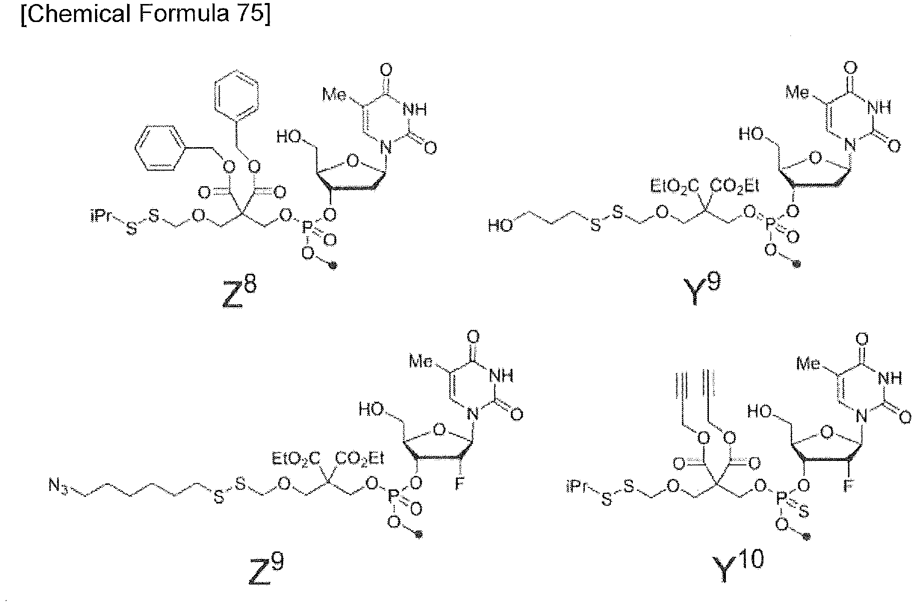

### Example 49

### Step 1

The antisense strand (SEQ ID NO: 38) of KON922 was obtained in the same manner as in step 1 in Example 5 using compound 26.
ESI-MS theoretical: 7003 measured: 7002

### Step 2

KON922 was obtained in the same manner as in step 2 in Example 5 using the antisense strand in KON922.

### Example 50

### Step 1

The antisense strand (SEQ ID NO: 39) of KON923 was obtained in the same manner as in step 1 in Example 5 and Example 29 using compound 26.
ESI-MS theoretical: 7019 measured: 7019

### Step 2

KON923 was obtained in the same manner as in step 2 in Example 5 using the antisense strand of KON923.

**[Table 25]**

| si RNA | **SEQ ID NO** | **Strand** | **Sequence (5'→3')** |
|---|---|---|---|
| KON922 | 19 | **Sense** | GCCAGACUUUGUUGGAUUAGT |
| | 38 | **Antisense** | Z¹⁰AAUCCAACAAAGUCUGGCUT |
| KON923 | 19 | **Sense** | GCCAGACUUUGUUGGAUUAGT |
| | 39 | **Antisense** | Y¹¹AAUCCAACAAAGUCUGGCUT |

[In the above table, A, G, C, T and U represent adenosine, guanosine, cytidine, thymidine and uridine, respectively; and Z¹⁰ and Y¹¹ represent compounds represented by the following formulae (wherein Me represents methyl), respectively.]

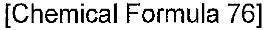

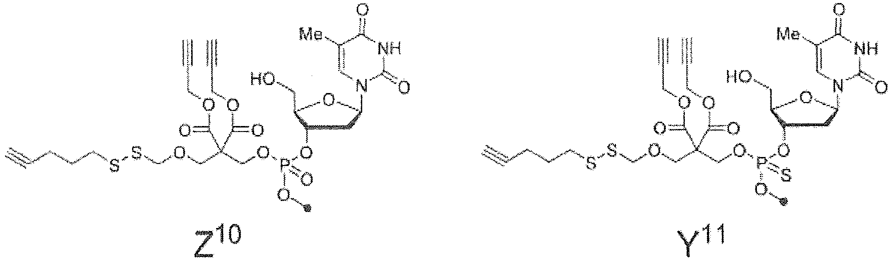

### Example 51

### Step 1

The antisense strand (SEQ ID NO: 44) of KON924 was obtained in the same manner as in step 1 in Example 5 using compound 18.
ESI-MS theoretical: 7004 measured: 7003

### Step 2

KON924 was obtained in the same manner as in step 2 in Example 5 using the antisense strand of KON924.

### Example 52

### Step 1

The antisense strand (SEQ ID NO: 45) of KON925 was obtained in the same manner as in step 1 in Example 5 and Example 29 using compound 18.
ESI-MS theoretical: 7020 measured: 7018

### Step 2

KON925 was obtained in the same manner as in step 2 in Example 5 using the antisense strand of KON925.

### Example 53

### Step 1

The antisense strand (SEQ ID NO: 46) of KON926 was obtained in the same manner as in step 1 in Example 5 using compound 24.

### ESI-MS theoretical: 7666 measured: 7664

### Step 2

KON926 was obtained in the same manner as in step 2 in Example 5 using the antisense strand of KON926.

**[Table 26]**

| si RNA | **SEQ ID NO** | **Strand** | **Sequence (5'→3')** |
|---|---|---|---|
| KON924 | 40 | **Sense** | GUCAUCACACUGAAUACCAAU |
| | 44 | **Antisense** | Z¹¹UUGGUAUUCAGUGUGAUGACAT |
| KON925 | 40 | **Sense** | GUCAUCACACUGAAUACCAAU |
| | 45 | **Antisense** | Y¹²UUGGUAUUCAGUGUGAUGACAT |
| KON926 | 40 | **Sense** | GUCAUCACACUGAAUACCAAU |
| | 46 | **Antisense** | Z¹²TUUGGUAUUCAGUGUGAUGACAT |

[In the above table, A, G, C, T and U represent adenosine, guanosine, cytidine, thymidine and uridine, respectively; and Z¹¹, Y¹² and Z¹² represent compounds represented by the following formulae (wherein Me, Et and iPr are respectively as defined above), respectively.]

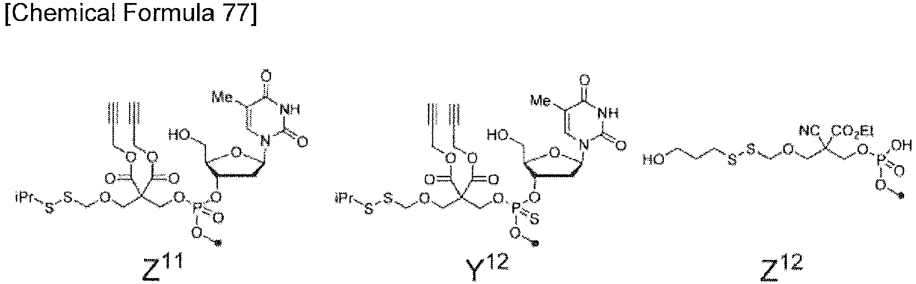

### Example 54

### Step 1

The antisense strand (SEQ ID NO: 51) of KON927 was obtained in the same manner as in step 1 in Example 5 using compound 18.
ESI-MS theoretical: 7682 measured: 7681

### Step 2

KON927 was obtained in the same manner as in step 2 in Example 5 using the antisense strand of KON927.

### Example 55

### Step 1

The antisense strand (SEQ ID NO: 52) of KON928 was obtained in the same manner as in step 1 in Example 5 and Example 29 using compound 18.
ESI-MS theoretical: 7695 measured: 7694

### Step 2

KON928 was obtained in the same manner as in step 2 in Example 5 using the antisense strand of KON928.

### Example 56

### Step 1

The antisense strand (SEQ ID NO: 53) of KON929 was obtained in the same manner as in step 1 in Example 5 using compound 24.
ESI-MS theoretical: 7111 measured: 7110

### Step 2

KON929 was obtained in the same manner as in step 2 in Example 5 using the antisense strand of KON929.

**[Table 27]**

| si RNA | **SEQ ID NO** | **Strand** | **Sequence (5'→3')** |
|---|---|---|---|
| KON927 | 47 | **Sense** | CCGUCGUAUUCGUGAGCAAGA |
| | 51 | **Antisense** | Z¹¹UGCUCACGAAUACGACGGUT |
| KON928 | 47 | **Sense** | CCGUCGUAUUCGUGAGCAAGA |
| | 52 | **Antisense** | Y¹²UGCUCACGAAUACGACGGUT |
| KON929 | 47 | **Sense** | CCGUCGUAUUCGUGAGCAAGA |
| | 53 | **Antisense** | Z¹²TUGCUCACGAAUACGACGGUT |

(In the above table, A, G, C, T and U represent adenosine, guanosine, cytidine, thymidine and uridine, respectively; and Z¹¹, Y¹² and Z¹² are respectively as defined above.)

### Example 57

### Step 1

The antisense strand (SEQ ID NO: 58) of KON930 was obtained in the same manner as in step 1 in Example 5 using compound 18.
ESI-MS theoretical: 7027 measured: 7027

### Step 2

KON930 was obtained in the same manner as in step 2 in Example 5 using the antisense strand of KON930.

### Example 58

### Step 1

The antisense strand (SEQ ID NO: 59) of KON931 was obtained in the same manner as in step 1 in Example 5 and Example 29 using compound 18.
ESI-MS theoretical: 7040 measured: 7039

### Step 2

KON931 was obtained in the same manner as in step 2 in Example 5 using the antisense strand of KON931.

### Example 59

### Step 1

The antisense strand (SEQ ID NO: 60) of KON932 was obtained in the same manner as in step 1 in Example 5 using compound 24.
ESI-MS theoretical: 7171 measured: 7170

### Step 2

KON932 was obtained in the same manner as in step 2 in Example 5 using the antisense strand of KON932.

**[Table 28]**

| si RNA | **SEQ ID NO** | **Strand** | **Sequence (5'→3')** |
|---|---|---|---|
| KON930 | 54 | **Sense** | CCUUCAUUGACCUCAACUACA |
| | 58 | **Antisense** | Z¹¹AGUUGAGGUCAAUGAAGGGT |
| KON931 | 54 | **Sense** | CCUUCAUUGACCUCAACUACA |
| | 59 | **Antisense** | Y¹²AGUUGAGGUCAAUGAAGGGT |
| KON932 | 54 | **Sense** | CCUUCAUUGACCUCAACUACA |
| | 60 | **Antisense** | Z¹²TAGUUGAGGUCAAUGAAGGGT |

(In the above table, A, G, C, T and U represent adenosine, guanosine, cytidine, thymidine and uridine, respectively; and Z¹¹, Y¹² and Z¹² are respectively as defined above.)

### Test Example 1: Knockdown activity by hypoxanthine-guanine phosphoribosyltransferase 1 (HPRT1)-targeting siRNAs

HeLa cells derived from human cervical cancer were suspended in RPMI1640 medium (Life Technologies Corporation, A10491-01) containing 10% foetal bovine serum and the cell suspension (80µL) was seeded into each well of a culture plate (Nunc 96-well microplate, 167008) to attain 5000 cells per well.

A cell line derived from human liver cancer (HepG2) was suspended in MEM medium (Life Technologies Corporation, 11095-080) containing 10% foetal bovine serum and the cell suspension (80µL) was seeded into each well of a culture plate (Nunc 96-well microplate, 167008) to attain 5000 cells per well.

siRNAs (1-fU), (3-Y) and (4-Z) targeting HPRT1 and Lipofectamine RNAiMAX (Life Technologies Corporation, 13778-150) were respectively diluted in Opti-MEM (Life Technologies Corporation, 31985-070). The thus prepared dilutions of siRNAs (1-fU), (3-Y) and (4-Z), respectively, were mixed with the dilution of Lipofectamine RNAiMAX to form complexes (siRNA-Lipofectamine RNAiMAX complexes) between the respective siRNAs (1-fU), (3-Y) and (4-Z) and Lipofectamine RNAiMAX. Each of the prepared siRNA-Lipofectamine RNAiMAX complex (20 µL) was added to the wells containing the cell suspensions to incorporate siRNAs (1-fU), (3-Y) and (4-Z), respectively, to HeLa cells. The final concentrations of each siRNA were varied over 4 points including 300 pmol/L, 94.9 pmol/L, 30.0 pmol/L and 9.49 pmol/L with N = 3. A negative control used was wells only containing Lipofectamine RNAiMAX (N = 12). The cells after introduction of siRNA were cultured under conditions of 37°C and 5% CO₂ for 48 hours.

A cell lysate containing RNA was prepared with SuperPrep Cell Lysis (Toyobo Co., Ltd., SCQ-101) and cDNA was prepared by reverse transcription using RT Kit for qPCR attached to the kit according to the instruction attached to the kit.

The cDNA was used as a template for PCR reaction. According to the Taqman probe method using ABI7900HT Fast (Applied Biosystems, Inc.), hypoxanthine-guanine phosphoribosyltransferase 1 (HPRT1, GenBank accession No. NM_000194.2) gene and, as a control, beta-actin (ACTB, GenBank accession No. NM_001101.3) gene were amplified. The level of mRNA amplification was measured and the semi-quantitative value of HPRT1 mRNA was calculated by using the level of mRNA amplification of ACTB as an internal control.

Taqman probe Hs02800695_m1 (Applied Biosystems, Inc.) was used for measurement of HPRT1 and Hs01060665_g1 (Applied Biosystems, Inc.) was used for measurement of ACTB. The mRNA level of HPRT1 and the level of mRNA amplification of ACTB were also measured in the negative control group and the semi-quantitative value of HPRT1 mRNA was calculated by using the level of mRNA amplification of ACTB as an internal control. The level of target mRNA of the siRNA-introduced sample was expressed as a proportion relative to the level of HPRT 1 mRNA in the non-siRNA-introduced group (negative control group) which was regarded as 1.

The results are shown in Figs. 1 and 2. As a result, it was found that siRNA (4-Z) showed knockdown activity similar to that of siRNA (1-fU). It was also found that siRNA (4-Z) showed higher knockdown activity than siRNA (3-Y). It was also confirmed that similar results were observed for another cell line.

Consequently, it was estimated that siRNA (4-Z) is quantitatively metabolised to siRNA (1-fU) in cells to exhibit knockdown activity.

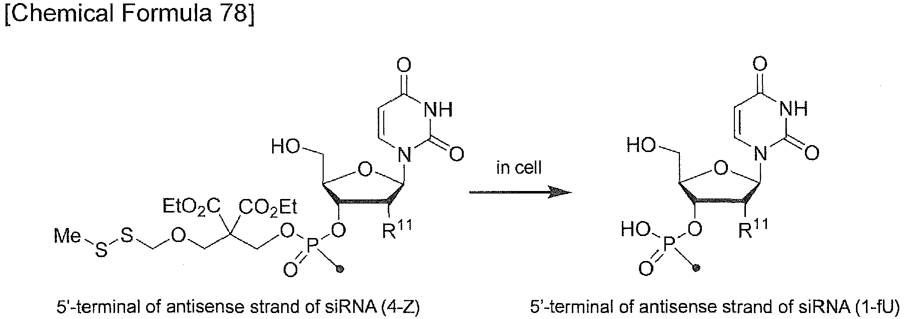

### Test Example 2: Knockdown activity by HPRT1-targeting cholesterol modified siRNAs

HeLa cells were suspended in RPMI1640 medium (Life Technologies Corporation, A10491-01) containing 10% foetal bovine serum and the cell suspension (100µL) was seeded into each well of a culture plate (Nunc 96-well microplate, 167008) to attain 5000 cells per well and cultured under conditions of 37°C and 5% CO₂ for 24 hours.

siRNAs (5-Z) and (6-fU) targeting HPRT1 were respectively diluted in Opti-MEM (Life Technologies Corporation, 31985-070). The culture supernatant was removed and 80 µL RPMI1640 medium (Life Technologies Corporation, A10491-01) without foetal bovine serum was added to each well. Each siRNA solution diluted with Opti-MEM (20 µL) was added to each well. The final concentrations of each siRNA were varied over 6 points including 1000 nmol/L, 316 nmol/L, 100 nmol/L, 31.6 nmol/L, 10.0 nmol/L and 3.16 nmol/L with N = 3. A negative control used was wells containing Opti-MEM (N = 6). The cells after introduction of siRNA were cultured under conditions of 37°C and 5% CO₂ for 48 hours.

In the same manner as in Test Example 1, cDNA was prepared from the cell lysate, and the cDNA was used as a template for PCR reaction. According to the Taqman probe method using ABI7900HT Fast (Applied Biosystems, Inc.), HPRT1 and, as control, ACTB were amplified. The level of mRNA amplification was measured and the semi-quantitative value of HPRT1 mRNA was calculated by using the level of mRNA amplification of ACTB as an internal control.

The results are shown in Fig. 3. As a result, it was found that siRNA (5-Z) showed knockdown activity equivalent to or higher than that of siRNA (6-fU).

Consequently, it was estimated that siRNA (5-Z) is quantitatively metabolised to siRNA (6-fU) in cells to exhibit knockdown activity.

Test Example 1 is directed to siRNA introduction using a transfection reagent while Test Example 2 is directed to carrier-free siRNA introduction. In addition, antisense strands used in both experiments are substantially identical and showed knockdown activity in both methods. Therefore, it was found that knockdown activity was exhibited regardless of the method of siRNA introduction.

### Test Example 3: Knockdown activity by CD45-targeting ASOs

Human acute monocytic leukaemia cells (THP-1) were suspended in RPMI1640 medium (Life Technologies Corporation, A10491-01) containing 10% foetal bovine serum and the cell suspension (80µL) was seeded into each well of a culture plate (Nunc 96-well microplate, 167008) to attain 5000 cells per well.

CD45-targeting ASOs, CD45 ABC, CD45 x2PO and CD45 x2IC and Lipofectamine 3000 (Life Technologies Corporation, L3000008) were respectively diluted in Opti-MEM (Life Technologies Corporation, 31985-070). The reagent P3000 attached to the kit of Lipofectamine 3000 was added to the Opti-MEM dilutions of CD45 ABC, CD45 x2PO and CD45 x2IC, respectively. The added solutions, respectively, were mixed with the Opti-MEM solution of Lipofectamine 3000 to form complexes (CD45-targeting ASO-Lipofectamine 3000 complexes) between the respective CD45-targeting ASOs (CD45 ABC, CD45 x2PO and CD45 x2IC) and Lipofectamine 3000. Each of the prepared CD45-targeting ASO-Lipofectamine 3000 complex (20 µL) was added to the wells containing the cell suspensions to incorporate the respective CD45-targeting ASOs into THP-1. The final concentrations of each ASO were varied over 5 points including 94.9 nmol/L, 30.0 nmol/L, 9.49 nmol/L, 3.00 nmol/L and 0.95 nmol/L with N = 3. A negative control used was wells containing the reagent P3000-containing Lipofectamine 3000 (N = 6). The cells after introduction of ASO were cultured under conditions of 37°C and 5% CO₂ for 24 hours.

In the same manner as in Test Example 1, cDNA was prepared from the cell lysate, and the cDNA was used as a template for PCR reaction. According to the Taqman probe method using ABI7900HT Fast (Applied Biosystems, Inc.), CD45 (PTPRC, protein tyrosine phosphatase receptor type C, GenBank accession No. NM_002838.4) gene and, as a control, ACTB (beta-actin, GenBank accession No. NM_001101.3) were amplified. The level of mRNA amplification was measured and the semi-quantitative value of PTPRC mRNA was calculated by using the level of mRNA amplification of ACTB as an internal control. The results are shown in Fig. 4. As a result, it was found that CD45 x2IC showed knockdown activity similar to those of CD45 ABC and CD45 x2PO.

### Test Example 4: Knockdown activity by fibroblast growth factor receptor 4 (FGFR4)-targeting ASOs

A cell line derived from human liver cancer (HepG2) was suspended in MEM medium (Life Technologies Corporation, 11095-080) containing 10% foetal bovine serum and the cell suspension (80µL) was seeded into each well of a culture plate (Nunc 96-well microplate, 167008) to attain 5000 cells per well.

A cell line derived from human liver cancer (HuH-7) was suspended in DMEM medium (Nacalai Tesque, Inc., 08458-16) containing 10% foetal bovine serum and the cell suspension (80µL) was seeded into each well of a culture plate (Nunc 96-well microplate, 167008) to attain 5000 cells per well.

In the same manner as in Test Example 3, complexes between the respective FGFR4-targeting ASOs (FGFR4 PS, FGFR PO and FGFR4 IC) and Lipofectamine 3000 were prepared and each of the prepared complexes was added to the wells containing the cell suspensions to incorporate the respective FGFR4-targeting ASOs into the cells. The final concentrations of each ASO were varied over 4 points including 30.0 nmol/L, 9.49 nmol/L, 3.00 nmol/L and 0.95 nmol/L with N = 3. A negative control used was wells containing the reagent P3000-containing Lipofectamine 3000 (N = 6). The cells after introduction of FGFR4-targeting ASO were cultured under conditions of 37°C and 5% CO₂ for 24 hours.

In the same manner as in Test Example 1, cDNA was prepared from the cell lysate, and the cDNA was used as a template for PCR reaction. According to the Taqman probe method using ABI7900HT Fast (Applied Biosystems, Inc.), FGFR4 (GenBank accession No. NM_002011.4) gene and, as a control, ACTB (beta-actin, GenBank accession No. NM_001101.3) were amplified. The level of mRNA amplification was measured and the semi-quantitative value of FGFR4 mRNA was calculated by using the level of mRNA amplification of ACTB as an internal control.

The results are shown in Figs. 5 and 6. As a result, it was found that FGFR4 IC showed knockdown activity similar to those of FGFR4 PS and FGFR4 PO.

### Test Example 5: Knockdown activity by Phosphatase and Tensin Homolog Deleted from Chromosome 10 (PTEN)-targeting ASOs

HeLa cells derived from human cervical cancer were suspended in RPMI1640 medium (Life Technologies Corporation, A10491-01) containing 10% foetal bovine serum, the cell suspension (100µL) was seeded into each well of a culture plate (Nunc 96-well microplate, 167008) to attain 5000 cells per well and cultured under conditions of 37°C and 5% CO₂ for 24 hours.

In the same manner as in Test Example 3, complexes between the respective PTEN-targeting ASOs, KON708, wt KON708, KON715 and wt KON715, and Lipofectamine 3000 were prepared and each of the prepared complexes was added to the wells containing the cell suspensions to incorporate the respective PTEN-targeting ASOs into the cells. The final concentrations of each PTEN-targeting ASO were varied over 4 points including 300 nmol/L, 94.9 nmol/L, 30.0 nmol/L and 9.49 nmol/L with N = 3. A negative control used was wells containing the reagent P3000-containing Lipofectamine 3000 (N = 12). The cells after introduction of PTEN-targeting ASO were cultured under conditions of 37°C and 5% CO₂ for 24 hours.

In the same manner as in Test Example 1, cDNA was prepared from the cell lysate, and the cDNA was used as a template for PCR reaction. According to the Taqman probe method using ABI7900HT Fast (Applied Biosystems, Inc.), PTEN (GenBank accession No. NM_000314.5) gene and, as a control, ACTB (beta-actin, GenBank accession No. NM_001101.3) were amplified. The level of mRNA amplification was measured and the semi-quantitative value of PTEN mRNA was calculated by using the level of mRNA amplification of ACTB as an internal control.

The results are shown in Fig. 7. As a result, it was found that KON708 and KON715 showed higher knockdown activity than comparative controls, wt KON708 and wt KON715.

### Test Example 6: Knockdown activity by HPRT1-targeting siRNAs

HeLa cells derived from human cervical cancer and a cell line derived from human liver cancer (HepG2) were seeded in the same manner as in Test Example 1.

A cell line derived from human liver cancer (HuH-7) was suspended in DMEM medium (DMEM High Glucose Medium, Nacalai Tesque, Inc., 08458-16) containing 10% foetal bovine serum and the cell suspension (80µL) was seeded into each well of a culture plate (Nunc 96-well microplate, 167008) to attain 5000 cells per well. Using HPRT1-targeting siRNAs, Ctrl (5',3' dT), KON788 and KON789, complexes between the respective HPRT1-targeting siRNAs and Lipofectamine RNAiMAX were formed in the same manner as in Test Example 1 and each of the prepared siRNA-Lipofectamine RNAiMAX complexes was added to the wells containing the cell suspensions to incorporate each of Ctrl (5', 3' dT), KON788 and KON789 into HeLa cells, HuH-7 cells and HepG2 cells.

The final concentrations of each siRNA were varied over 6 points including 1000 pmol/L, 316 pmol/L, 100 pmol/L, 31.6 pmol/L, 10 pmol/L and 3.16 pmol/L with N = 3. A negative control (nt) used was wells containing only Lipofectamine RNAiMAX (N = 6). Knockdown activity was evaluated in the same manner as in Test Example 1.

The results are shown in Fig. 8. As a result, it was found that in each cell line of HeLa cells, HuH-7 cells and HepG2 cells, KON789 showed knockdown activity similar to those of Ctrl (5',3' dT) and KON788.

### Test Example 7: Knockdown activity by HPRT1-targeting siRNAs

Using HPRT1-targeting siRNAs, Ctrl (5', 3' dT), KON789, KON816 and KON788, complexes between the respective siRNA and Lipofectamine RNAiMAX were formed in the same manner as in Test Example 1 and each of the prepared siRNA-Lipofectamine RNAiMAX complexes was added to the wells containing the cell suspensions to incorporate each of Ctrl (5', 3' dT), KON789, KON816 and KON788 into HeLa cells, HuH-7 cells and HepG2 cells.

The final concentrations of each siRNA were varied over 4 points including 300 pmol/L, 94.9 pmol/L, 30.0 pmol/L and 9.49 pmol/L with N = 3. A negative control (nt) used was wells containing only Lipofectamine RNAiMAX (N = 12). Knockdown activity was evaluated in the same manner as in Test Example 6.

The results are shown in Fig. 9. As a result, it was found that in HeLa cells, HuH-7 cells and HepG2 cells, the negative control, KON788, had reduced knockdown activity compared to that of Ctrl (5', 3' dT) while KON789 and KON816 showed knockdown activity similar to that of Ctrl (5', 3' dT).

### Test Example 8: Knockdown activity by HPRT1-targeting siRNAs

Using HPRT1-targeting siRNAs, Ctrl (5', 3' dT), KON816, KON818 and KON788, knockdown activity was evaluated in the same manner as in Test Example 7.

The results are shown in Fig. 10. As a result, it was found that in HeLa cells, HuH-7 cells and HepG2 cells, the negative control, KON788, had reduced knockdown activity compared to that of Ctrl (5', 3' dT) while KON816 and KON818 showed knockdown activity similar to that of Ctrl (5', 3' dT).

### Test Example 9: Knockdown activity by HPRT1-targeting siRNAs

Using HPRT1-targeting siRNAs, Ctrl (5', 3' dT), KON818, KON788, KON857, KON840 and KON846, knockdown activity was evaluated in the same manner as in Test Example 6.

The results are shown in Fig. 11 (HeLa cells), Fig. 12 (HuH-7 cells) and Fig. 13 (HepG2 cells). As a result, it was found that in HeLa cells, HuH-7 cells and HepG2 cells, the negative controls, KON788 and KON840 had reduced knockdown activity compared to that of Ctrl (5', 3' dT) while KON818, KON857 and KON846 showed knockdown activity similar to that of Ctrl (5', 3' dT).

### Test Example 10: Knockdown activity by HPRT1-targeting siRNA

Using HPRT1-targeting siRNAs, Ctrl (5'-F) and KON880 to KON884, knockdown activity was evaluated in the same manner as in Test Example 6.

The results are shown in Fig. 14 (HeLa cells), Fig. 15 (HuH-7 cells) and Fig. 16 (HepG2 cells). As a result, it was found that in HeLa cells, HuH-7 cells and HepG2 cells, KON880 to KON884 showed knockdown activity similar to that of Ctrl (5'-F).

### Test Example 11: Knockdown activity by HPRT1-targeting siRNAs

Using HPRT1-targeting siRNAs, Ctrl (5'-F), KON846, KON891, KON892, KON903 and KO905, knockdown activity was evaluated in the same manner as in Test Example 6.

The results are shown in Fig. 17 (HeLa cells), Fig. 18 (HuH-7 cells) and Fig. 19 (HepG2 cells). As a result, it was found that in HeLa cells, HuH-7 cells and HepG2 cells, KON846, KON891, KON892, KON903 and KON905 showed knockdown activity similar to that of Ctrl (5'-F).

### Test Example 12: Knockdown activity by HPRT1-targeting siRNAs

Using HPRT1-targeting siRNAs, Ctrl (5', 3' dT), KON922, KON923 and KON788, the final concentrations of each siRNA were varied over 4 points including 1000 pmol/L, 316 pmol/L, 100 pmol/L and 31.6 pmol/L with N = 3. A negative control used was wells containing only Lipofectamine RNAiMAX (N = 12). Knockdown activity was evaluated in the same manner as in Test Example 6.

The results are shown in Fig. 20 (HeLa cells), Fig. 21 (HuH-7 cells) and Fig. 22 (HepG2 cells). As a result, it was found that in HeLa cells, HuH-7 cells and HepG2 cells, KON922 and KON923 showed knockdown activity similar to that of Ctrl (5', 3' dT).

### Test Example 13: Knockdown activity by apolipoprotein B (ApoB)-targeting siRNAs

Using ApoB-targeting siRNAs, wt924, KON924, wt925, KON925, wt926 and KON926 and a Taqman probe for measurement of ApoB mRNA, Hs01071209_m1 (Applied Biosystems, Inc.), knockdown activity was evaluated in the same manner as in Test Example 6.

The results are shown in Fig. 23 (HepG2 cells) and Fig. 24 (HuH-7 cells). As a result, it was found that in HepG2 cells and HuH-7 cells, KON924, KON925 and KON926 showed knockdown activity similar to those of corresponding wt924, wt925 and wt926, respectively.

### Test Example 14: Knockdown activity by luciferase-targeting siRNAs

Using luciferase-targeting siRNAs, wt927, KON927, wt928, KON928, wt929 and KON929, knockdown activity was evaluated as follows. Luciferase-expressing HeLa cells (HeLa-Luc), namely HeLa cells derived from human cervical cancer into which a luciferase expression vector [pGL4.50 (luc2/CMV/Hygro) Vector, Promega Corporation] was introduced, were suspended in RPMI medium (Invitrogen Corporation, 11875093) containing 10% foetal bovine serum, the cell suspension (80µL) was seeded in each well of a culture plate (Culture Plate 96, PerkinElmer Inc., 9005680) to attain 7500 cells per well. The final concentrations of each siRNA were varied over 1000 pmol/L, 316 pmol/L, 100 pmol/L, 31.6 pmol/L, 10 pmol/L and 3.16 pmol/L and the nucleic acid solutions were added to cells in the same manner as in Test Example 1 and cultured under conditions of 37°C and 5% CO₂ for 48 hours. To the cells after cultivation, 40 µL of a commercially available luciferase assay reagent, Steady-Glo Luciferase Assay System (Promega Corporation, E2520), was added to each well according to the protocol attached to the assay reagent. After incubation of 10 minutes, the luminescence per second (cps) of each well was measured on ARVO (PerkinElmer Inc.). The luminescence of the negative control group was also measured in parallel to the luminescence of the luciferase-targeting siRNA treated group to express the RNAi effect of siRNA-introduced samples as a proportion relative to the luminescence of the non-siRNA-introduced group (negative control group) which was regarded as 1.

The results are shown in Fig. 25. As a result, it was found that KON927, KON928 and KON929 showed knockdown activity similar to those of corresponding wt927, wt928 and wt929, respectively.

### Test Example 15: Knockdown activity by glyceraldehyde 3-phosphate dehydrogenase (GAPDH)-targeting siRNAs

Using GAPDH-targeting siRNAs, wt930, KON930, wt931, KON931, wt932 and KON932 and a Taqman probe for GAPDH mRNA measurement, Hs02758991_g1 (Applied Biosystems, Inc.), knockdown activity was evaluated in the same manner as in Test Example 6.

The results are shown in Fig. 26 (HeLa cells), Fig. 27 (HuH-7 cells) and Fig. 28 (HepG2 cells). As a result, it was found that in HeLa cells, HuH-7 cells and HepG2 cells, KON930, KON931 and KON932 showed knockdown activity similar to those of corresponding wt930, wt931 and wt932, respectively.

### Industrial Applicability

The oligonucleotide derivative and the like of the present invention are useful as nucleic acid medicine.

### Sequence Listing Free Text

SEQ ID NO: 1 represents the base sequence of the sense strand of siRNAs (1-fU), (3-Y) and (4-Z).
SEQ ID NO: 2 represents the base sequence of the antisense strand of siRNA (1-fU).
SEQ ID NO: 4 represents the base sequence of the antisense strand of siRNA (3-Y).
SEQ ID NO: 5 represents the base sequence of the antisense strand of siRNA (4-Z).
SEQ ID NO: 6 represents the base sequence of the sense strand of siRNAs (5-Z) and (6-fU).
SEQ ID NO: 7 represents the base sequence of the antisense strand of siRNA (5-Z).
SEQ ID NO: 8 represents the base sequence of the antisense strand of siRNA (6-fU).
SEQ ID NO: 9 represents the base sequence of a CD45-targeting oligonucleotide CD45 ABC.
SEQ ID NO: 10 represents the base sequence of a CD45-targeting oligonucleotide CD45 x2PO.
SEQ ID NO: 11 represents the base sequence of a CD45-targeting oligonucleotide CD45 x2IC.
SEQ ID NO: 12 represents the base sequence of a FGFR4-targeting oligonucleotide FGFR4 PS.
SEQ ID NO: 13 represents the base sequence of a FGFR4-targeting oligonucleotide FGFR4 PO.
SEQ ID NO: 14 represents the base sequence of a CD45-targeting oligonucleotide FGFR4 IC.
SEQ ID NO: 15 represents the base sequence of a PTEN-targeting oligonucleotide wtKON708.
SEQ ID NO: 16 represents the base sequence of a PTEN-targeting oligonucleotide wtKON715.
SEQ ID NO: 17 represents the base sequence of a PTEN-targeting oligonucleotide KON708.
SEQ ID NO: 18 represents the base sequence of a PTEN-targeting oligonucleotide KON715.
SEQ ID NO: 19 represents the base sequence of the sense strand of Ctrl (5',3'dT).
SEQ ID NO: 20 represents the base sequence of the antisense strand of Ctrl (5',3'dT).
SEQ ID NO: 21 represents the base sequence of the antisense strand of KON788.
SEQ ID NO: 22 represents the base sequence of the antisense strand of KON789.
SEQ ID NO: 23 represents the base sequence of the antisense strand of KON816.
SEQ ID NO: 24 represents the base sequence of the antisense strand of KON818.
SEQ ID NO: 25 represents the base sequence of the antisense strand of KON840.
SEQ ID NO: 26 represents the base sequence of the antisense strand of KON846.
SEQ ID NO: 27 represents the base sequence of the antisense strand of KON857.
SEQ ID NO: 28 represents the base sequence of the antisense strand of Ctrl (5'-F).
SEQ ID NO: 29 represents the base sequence of the antisense strand of KON880.
SEQ ID NO: 30 represents the base sequence of the antisense strand of KON881.
SEQ ID NO: 31 represents the base sequence of the antisense strand of KON882.
SEQ ID NO: 32 represents the base sequence of the antisense strand of KON883.
SEQ ID NO: 33 represents the base sequence of the antisense strand of KON884.
SEQ ID NO: 34 represents the base sequence of the antisense strand of KON891.
SEQ ID NO: 35 represents the base sequence of the antisense strand of KON892.
SEQ ID NO: 36 represents the base sequence of the antisense strand of KON903.
SEQ ID NO: 37 represents the base sequence of the antisense strand of KON905.
SEQ ID NO: 38 represents the base sequence of the antisense strand of KON922.
SEQ ID NO: 39 represents the base sequence of the antisense strand of KON923.
SEQ ID NO: 40 represents the base sequence of the sense strand of wt924.
SEQ ID NO: 41 represents the base sequence of the antisense strand of wt924.
SEQ ID NO: 42 represents the base sequence of the antisense strand of wt925.
SEQ ID NO: 43 represents the base sequence of the antisense strand of wt926.
SEQ ID NO: 44 represents the base sequence of the antisense strand of KON924.
SEQ ID NO: 45 represents the base sequence of the antisense strand of KON925.
SEQ ID NO: 46 represents the base sequence of the antisense strand of KON926.
SEQ ID NO: 47 represents the base sequence of the sense strand of wt927.
SEQ ID NO: 48 represents the base sequence of the antisense strand of wt927.
SEQ ID NO: 49 represents the base sequence of the antisense strand of wt928.
SEQ ID NO: 50 represents the base sequence of the antisense strand of wt929.
SEQ ID NO: 51 represents the base sequence of the antisense strand of KON927.
SEQ ID NO: 52 represents the base sequence of the antisense strand of KON928.
SEQ ID NO: 53 represents the base sequence of the antisense strand of KON929.
SEQ ID NO: 54 represents the base sequence of the sense strand of wt930.
SEQ ID NO: 55 represents the base sequence of the antisense strand of wt930.
SEQ ID NO: 56 represents the base sequence of the antisense strand of wt931.
SEQ ID NO: 57 represents the base sequence of the antisense strand of wt932.
SEQ ID NO: 58 represents the base sequence of the antisense strand of KON930.
SEQ ID NO: 59 represents the base sequence of the antisense strand of KON931.
SEQ ID NO: 60 represents the base sequence of the antisense strand of KON932.

## Claims

1. An oligonucleotide derivative having, at an oxygen atom of at least one phosphate group of an oligonucleotide, a group represented by formula (I): 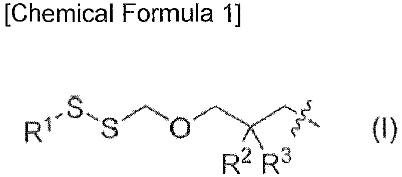 wherein R¹ represents lower alkyl optionally substituted with lower alkoxy; R² and R³ are the same or different and respectively represent an electron-withdrawing group or R², R³ and the carbon atom adjacent to them together represent formula (II): 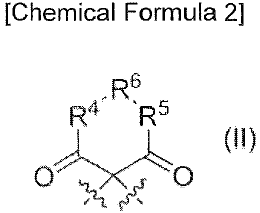 wherein R⁴ and R⁵ are the same or different and respectively represent oxygen atom, CH₂ or NR⁷ wherein R⁷ represents hydrogen atom or lower alkyl; and R⁶ represents a bond or CR⁸R⁹ wherein R⁸ and R⁹ are the same or different and respectively represent hydrogen atom or lower alkyl,
or a salt thereof.

2. The oligonucleotide derivative or a salt thereof according to claim 1, having, at an oxygen atom of one phosphate group of the oligonucleotide, the group represented by formula (I).

3. The oligonucleotide derivative or a salt thereof according to claim 1, comprising one or more structures represented by the following formula (III): 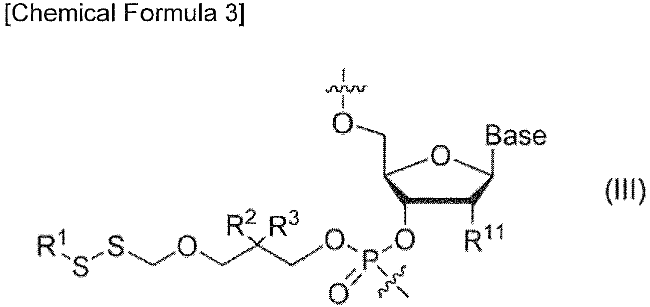 wherein Base represents a nucleobase; R¹, R² and R³ respectively are as defined above; and R¹¹ represents hydrogen atom, fluorine atom or lower alkoxy; provided that when the oligonucleotide derivative or a salt thereof has two or more structures represented by formula (III), Base, R¹, R², R³ and R¹¹ may respectively be the same or different between the structures.

4. The oligonucleotide derivative or a salt thereof according to any of claims 1 to 3, wherein R¹ represents lower alkyl.

5. The oligonucleotide derivative or a salt thereof according to any of claims 1 to 4, wherein R² and R³ are the same or different and respectively represent carboxy or lower alkoxycarbonyl.

6. The oligonucleotide derivative or a salt thereof according to any of claims 1 to 4, wherein R² and R³ are the same or different and respectively represent cyano, nitro, lower alkanoyl or lower alkylsulfonyl.

7. The oligonucleotide derivative or a salt thereof according to any of claims 3 to 6, comprising only one structure represented by formula (III).

8. The oligonucleotide derivative or a salt thereof according to any of claims 1 to 7, having a base length of 5 to 100.

9. The oligonucleotide derivative or a salt thereof according to any of claims 1 to 7, having a base length of 10 to 80.

10. The oligonucleotide derivative or a salt thereof according to any of claims 1 to 9, wherein the oligonucleotide is a double strand.

11. The oligonucleotide derivative or a salt thereof according to any of claims 1 to 9, wherein the oligonucleotide is a single strand.

12. The oligonucleotide derivative or a salt thereof according to any of claims 1 to 11, wherein the oligonucleotide is a small interfering RNA (siRNA).

13. An oligonucleotide derivative having, at an oxygen atom of at least one phosphate group of an oligonucleotide, a group represented by formula (I-0): 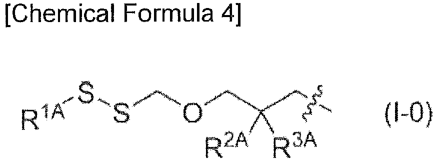 wherein R^{1A} represents optionally substituted lower alkyl, optionally substituted lower alkenyl or optionally substituted lower alkynyl; R^{2A} and R^{3A} are the same or different and respectively represent an electron-withdrawing group or R^{2A}, R^{3A} and the carbon atom adjacent to them together represent formula (II-0): 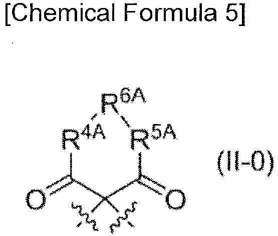 wherein R^{4A} and R^{5A} are the same or different and respectively represent oxygen atom, CH₂ or NR^{7A} wherein R^{7A} represents hydrogen atom or lower alkyl; and R^{6A} represents a bond or CR^{8A}R^{9A} wherein R^{8A} and R^{9A} are the same or different and respectively represent hydrogen atom or lower alkyl,
or a salt thereof.

14. The oligonucleotide derivative or a salt thereof according to claim 13, having, at an oxygen atom of one phosphate group of the oligonucleotide, the group represented by formula (I-0).

15. The oligonucleotide derivative or a salt thereof according to claim 13, comprising one or more structures represented by formula (III-0): 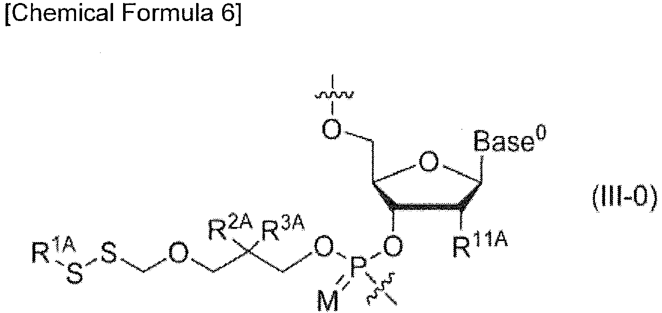 wherein Base⁰ represents a nucleobase; M represents oxygen atom or sulfur atom; R^{1A} , R^{2A} and R^{3A} respectively are as defined above; R^{11A} represents hydrogen atom, fluorine atom or lower alkoxy; provided that when the oligonucleotide derivative or a salt thereof has two or more structures represented by formula (III-0), Base⁰, M, R^{1A}, R^{2A}, R^{3A} and R^{11A} may respectively be the same or different between the structures.

16. The oligonucleotide derivative or a salt thereof according to any one of claims 13 to 15, wherein R^{1A} represents optionally substituted lower alkyl.

17. The oligonucleotide derivative or a salt thereof according to any one of claims 13 to 16, wherein R^{2A} and R^{3A} are the same or different and respectively represent carboxy, optionally substituted aralkyloxycarbonyl, lower alkoxycarbonyl, lower alkenyloxycarbonyl or lower alkynyloxycarbonyl.

18. The oligonucleotide derivative or a salt thereof according to any one of claims 13 to 16, wherein R^{2A} and R^{3A} are the same or different and respectively represent cyano, nitro, lower alkanoyl or lower alkylsulfonyl.

19. The oligonucleotide derivative or a salt thereof according to any one of claims 15 to 18, comprising only one structure represented by formula (III-0).

20. The oligonucleotide derivative or a salt thereof according to claim 13, comprising a structure represented by formula (III-1): 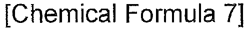 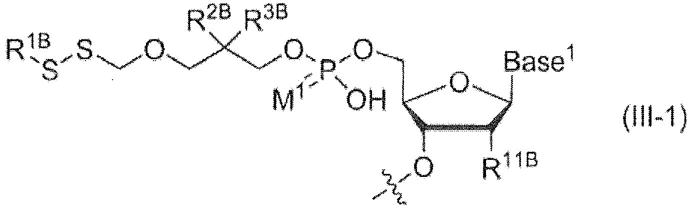 wherein Base represents a nucleobase; M¹ represents oxygen atom or sulfur atom; R^{1B} represents optionally substituted lower alkyl, optionally substituted lower alkenyl or optionally substituted lower alkynyl; R^{2B} and R^{3B} are the same or different and respectively represent an electron-withdrawing group or R^{2B}, R^{3B} and the carbon atom adjacent to them together represent formula (II-1): 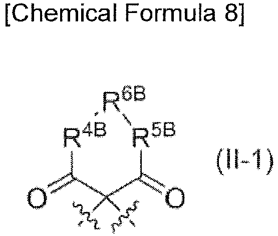 wherein R^{4B} and R^{5B} are the same or different and respectively represent oxygen atom, CH₂ or NR^{7B} wherein R^{7B} represents hydrogen atom or lower alkyl; R^{6B} represents a bond or CR^{8B}R^{9B} wherein R^{8B} and R^{9B} are the same or different and respectively represent hydrogen atom or lower alkyl; and R^{11B} represents hydrogen atom, fluorine atom or lower alkoxy.

21. The oligonucleotide derivative or a salt thereof according to claim 20, wherein R^{1B} represents optionally substituted lower alkyl.

22. The oligonucleotide derivative or a salt thereof according to claim 20 or 21, wherein R^{2B} and R^{3B} are the same or different and respectively represent carboxy, optionally substituted aralkyloxycarbonyl, lower alkoxycarbonyl, lower alkenyloxycarbonyl or lower alkynyloxycarbonyl.

23. The oligonucleotide derivative or a salt thereof according to claim 20 or 21, wherein R^{2B} and R^{3B} are the same or different and respectively represent cyano, nitro, lower alkanoyl or lower alkylsulfonyl.

24. The oligonucleotide derivative or a salt thereof according to any one of claims 13 to 23, having a base length of 5 to 100.

25. The oligonucleotide derivative or a salt thereof according to any one of claims 13 to 23, having a base length of 10 to 80.

26. The oligonucleotide derivative or a salt thereof according to any one of claims 13 to 25, wherein the oligonucleotide is a double strand.

27. The oligonucleotide derivative or a salt thereof according to any one of claims 13 to 25, wherein the oligonucleotide is a single strand.

28. The oligonucleotide derivative or a salt thereof according to any one of claims 13 to 27, wherein the oligonucleotide is a small interfering RNA (siRNA).

29. A compound represented by formula (IV): 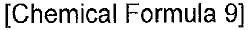 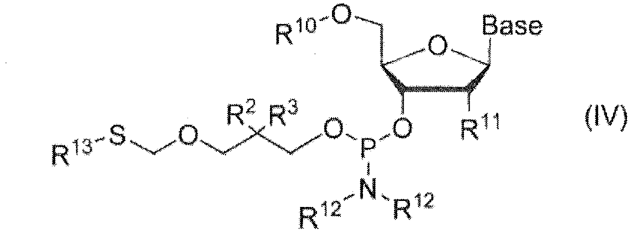 wherein Base represents a nucleobase; R² and R³ are the same or different and respectively represent an electron-withdrawing group or R², R³ and the carbon atom adjacent to them together represent formula (II): 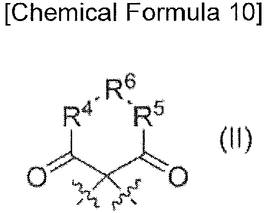 wherein R⁴ and R⁵ are the same or different and respectively represent oxygen atom, CH₂ or NR⁷ wherein R⁷ represents hydrogen atom or lower alkyl; R⁶ represents a bond or CR⁸R⁹ wherein R⁸ and R⁹ are the same or different and respectively represent hydrogen atom or lower alkyl; R¹⁰ represents a protecting group of hydroxy group; R¹¹ represents hydrogen atom, fluorine atom or lower alkoxy; R¹² represents lower alkyl; and R¹³ represents lower alkylthio or formula (V): 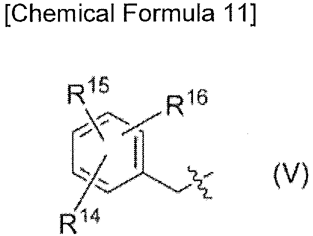 wherein R¹⁴, R¹⁵ and R¹⁶ are the same or different and respectively represent hydrogen atom, lower alkyl, lower alkoxy or NR¹⁷R¹⁸ wherein R¹⁷ and R¹⁸ are the same or different and respectively represent lower alkyl,
or a salt thereof.

30. A compound represented by formula (IV-0): 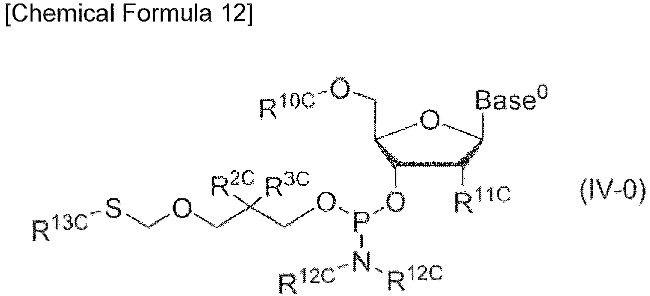 wherein Base⁰ represents a nucleobase, R^{2C} and R^{3C} are the same or different and respectively represent an electron-withdrawing group or R^{2C}, R^{3C} and the carbon atom adjacent to them together represent formula (II-2): 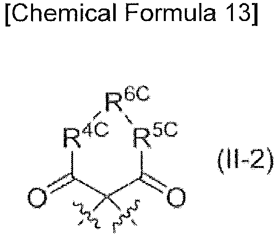 wherein R^{4C} and R^{5C} are the same or different and respectively represent oxygen atom, CH₂ or NR^{7C} wherein R^{7C} represents hydrogen atom or lower alkyl; R^{6C} represents a bond or CR^{8C}R^{9C} wherein R^{8C} and R^{9C} are the same or different and respectively represent hydrogen atom or lower alkyl; R^{10C} represents a protecting group of hydroxy group; R^{11C} represents hydrogen atom, fluorine atom or lower alkoxy; R^{12C} represents lower alkyl; and R^{13C} represents optionally substituted lower alkylthio, optionally substituted lower alkenylthio, optionally substituted lower alkynylthio or formula (V-0): 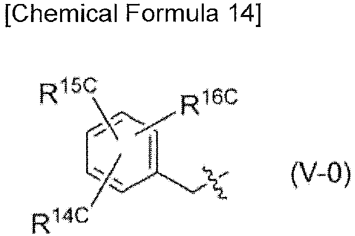 wherein R^{14C}, R^{15C} and R^{16C} are the same or different and respectively represent hydrogen atom, lower alkyl, lower alkoxy or NR^{17C}R^{18C} wherein R^{17C} and R^{18C} are the same or different and respectively represent lower alkyl,
or a salt thereof.

31. The compound or a salt thereof according to claim 30, wherein R^{2C} and R^{3C} are the same or different and respectively represent carboxy, optionally substituted aralkyloxycarbonyl, lower alkoxycarbonyl, lower alkenyloxycarbonyl or lower alkynyloxycarbonyl.

32. The compound or a salt thereof according to claim 30, wherein R^{2C} and R^{3C} are the same or different and respectively represent cyano, nitro, lower alkanoyl or lower alkylsulfonyl.

33. The compound or a salt thereof according to any one of claims 30 to 32, wherein R^{10C} represents trityl, 4-methoxytrityl or 4,4'-dimethoxytrityl.

34. A compound represented by formula (IV-1): 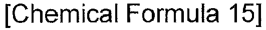 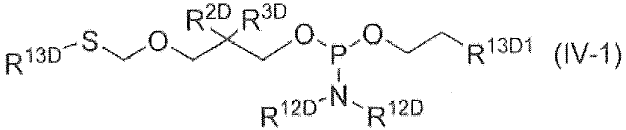 wherein R^{2D} and R^{3D} are the same or different and respectively represent an electron-withdrawing group or R^{2D}, R^{3D} and the carbon atom adjacent to them together represent formula (II-3): 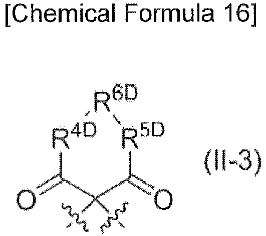 wherein R^{4D} and R^{5D} are the same or different and respectively represent oxygen atom, CH₂ or NR^{7D} wherein R^{7D} represents hydrogen atom or lower alkyl; R^{6D} represents a bond or CR^{8D}R^{9D} wherein R^{8D} and R^{9D} are the same or different and respectively represent hydrogen atom or lower alkyl; R^{12D} represents lower alkyl; R^{13D} represents optionally substituted lower alkylthio, optionally substituted lower alkenylthio, optionally substituted lower alkynylthio or formula (V-1): 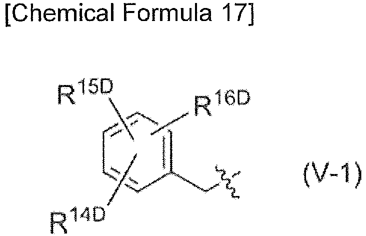 wherein R^{14D}, R^{15D} and R^{16D} are the same or different and respectively represent hydrogen atom, lower alkyl, lower alkoxy or NR^{17D}R^{18D} wherein R^{17D} and R^{18D} are the same or different and respectively represent lower alkyl; and R^{13D1} represents cyano, nitro, carboxy, lower alkoxycarbonyl, lower alkylsulfonyl or optionally substituted arylsulfonyl,
or a salt thereof.

35. The compound or a salt thereof according to claim 34, wherein R^{2D} and R^{3D} are the same or different and respectively represent carboxy, optionally substituted aralkyloxycarbonyl, lower alkoxycarbonyl, lower alkenyloxycarbonyl or lower alkynyloxycarbonyl.

36. The compound or a salt thereof according to claim 34, wherein R^{2D} and R^{3D} are the same or different and respectively represent cyano, nitro, lower alkanoyl or lower alkylsulfonyl.

37. A compound represented by formula (VI): 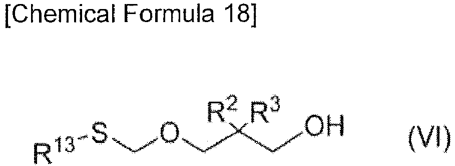 wherein R² and R³ are the same or different and respectively represent an electron-withdrawing group or R², R³ and the carbon atom adjacent to them together represent formula (II): 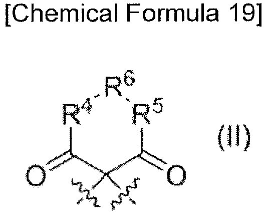 wherein R⁴ and R⁵ are the same or different and respectively represent oxygen atom, CH₂ or NR⁷ wherein R⁷ represents hydrogen atom or lower alkyl; R⁶ represents a bond or CR⁸R⁹ wherein R⁸ and R⁹ are the same or different and respectively represent hydrogen atom or lower alkyl; and R¹³ represents lower alkylthio or formula (V): 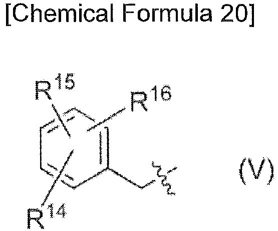 wherein R¹⁴, R¹⁵ and R¹⁶ are the same or different and respectively represent hydrogen atom, lower alkyl, lower alkoxy or NR¹⁷R¹⁸ wherein R¹⁷ and R¹⁸ are the same or different and respectively represent lower alkyl,
or a salt thereof.

38. A compound represented by formula (VI-0): 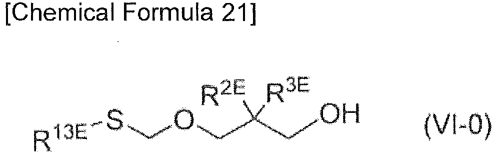 wherein R^{2E} and R^{3E} are the same or different and respectively represent an electron-withdrawing group or R^{2E}, R^{3E} and the carbon atom adjacent to them together represent formula (II-4): 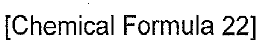 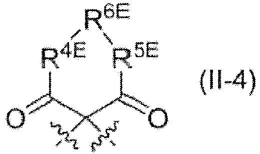 wherein R^{4E} and R^{5E} are the same or different and respectively represent oxygen atom, CH₂ or NR^{7E} wherein R^{7E} represents hydrogen atom or lower alkyl; R^{6E} represents a bond or CR^{8E}R^{9E} wherein R^{8E} and R^{9E} are the same or different and respectively represent hydrogen atom or lower alkyl; and R^{13E} represents optionally substituted lower alkylthio, optionally substituted lower alkenylthio, optionally substituted lower alkynylthio or formula (V-2): 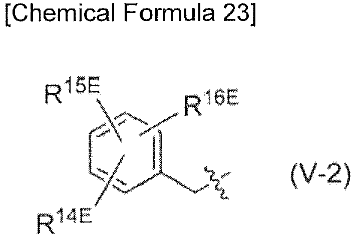 wherein R^{14E}, R^{15E} and R^{16E} are the same or different and respectively represent hydrogen atom, lower alkyl, lower alkoxy or NR^{17E}R^{18E} wherein R^{17E} and R^{18E} are the same or different and respectively represent lower alkyl,
or a salt thereof.

39. The compound or a salt thereof according to claim 38, R^{2E} and R^{3E} are the same or different and respectively represent carboxy, optionally substituted aralkyloxycarbonyl, lower alkoxycarbonyl, lower alkenyloxycarbonyl or lower alkynyloxycarbonyl.

40. The compound or a salt thereof according to claim 38, wherein R^{2E} and R^{3E} are the same or different and respectively represent cyano, nitro, lower alkanoyl or lower alkylsulfonyl.


SEQUENCE LISTING

<110>  Kyowa Hakko Kirin Co., Ltd.

<120>  oligonucleotide derivative

<130>  K1635AIP0003

<150>  JP 2015-128416
<151>  2015-06-26

<160>  60

<170>  PatentIn version 3.5

<210>  1
<211>  21
<212>  DNA
<213>  Artificial Sequence

<220>
<223>  1-fU sense, 3-Y sense, 4-Z sense


<220>
<221>  misc_feature
<222>  (1)..(20)
<223>  RNA

<220>
<221>  misc_feature
<222>  (2)..(2)
<223>  2'-O-methyl cytidine

<220>
<221>  misc_feature
<222>  (4)..(4)
<223>  2'-O-methyl adenosine

<220>
<221>  misc_feature
<222>  (6)..(6)
<223>  2'-O-methyl adenosine

<220>
<221>  misc_feature
<222>  (8)..(8)
<223>  2'-O-methyl uridine

<220>
<221>  misc_feature
<222>  (10)..(10)
<223>  2'-O-methyl uridine

<220>
<221>  misc_feature
<222>  (12)..(12)
<223>  2'-O-methyl uridine

<220>
<221>  misc_feature
<222>  (14)..(14)
<223>  2'-O-methyl guanosine

<220>
<221>  misc_feature
<222>  (16)..(16)
<223>  2'-O-methyl adenosine

<220>
<221>  misc_feature
<222>  (18)..(18)
<223>  2'-O-methyl uridine

<220>
<221>  misc_feature
<222>  (20)..(20)
<223>  2'-O-methyl guanosine

<400>  1
gccagacuuu guuggauuag t                                                21


<210>  2
<211>  21
<212>  DNA
<213>  Artificial Sequence

<220>
<223>  1-fU antisense



<220>
<221>  misc_feature
<222>  (1)..(1)
<223>  2'-fluoro uridine

<220>
<221>  misc_feature
<222>  (2)..(20)
<223>  RNA

<220>
<221>  misc_feature
<222>  (3)..(3)
<223>  2'-O-methyl adenosine

<220>
<221>  misc_feature
<222>  (5)..(5)
<223>  2'-O-methyl cytidine

<220>
<221>  misc_feature
<222>  (7)..(7)
<223>  2'-O-methyl adenosine

<220>
<221>  misc_feature
<222>  (9)..(9)
<223>  2'-O-methyl cytidine

<220>
<221>  misc_feature
<222>  (11)..(11)
<223>  2'-O-methyl adenosine

<220>
<221>  misc_feature
<222>  (13)..(13)
<223>  2'-O-methyl guanosine

<220>
<221>  misc_feature
<222>  (15)..(15)
<223>  2'-O-methyl cytidine

<220>
<221>  misc_feature
<222>  (17)..(17)
<223>  2'-O-methyl guanosine

<220>
<221>  misc_feature
<222>  (19)..(19)
<223>  2'-O-methyl cytidine

<400>  2
uaauccaaca aagucuggcu t                                                21


<210>  3
<400>  3
000


<210>  4
<211>  21
<212>  DNA
<213>  Artificial Sequence

<220>
<223>  3-Y antisense


<220>
<221>  misc_feature
<222>  (1)..(1)
<223>  2'-fluoro uridine

<220>
<221>  misc_feature
<222>  (2)..(20)
<223>  RNA

<220>
<221>  misc_feature
<222>  (3)..(3)
<223>  2'-O-methyl adenosine

<220>
<221>  misc_feature
<222>  (5)..(5)
<223>  2'-O-methyl cytidine

<220>
<221>  misc_feature
<222>  (7)..(7)
<223>  2'-O-methyl adenosine

<220>
<221>  misc_feature
<222>  (9)..(9)
<223>  2'-O-methyl cytidine

<220>
<221>  misc_feature
<222>  (11)..(11)
<223>  2'-O-methyl adenosine

<220>
<221>  misc_feature
<222>  (13)..(13)
<223>  2'-O-methyl guanosine

<220>
<221>  misc_feature
<222>  (15)..(15)
<223>  2'-O-methyl cytidine

<220>
<221>  misc_feature
<222>  (17)..(17)
<223>  2'-O-methyl guanosine

<220>
<221>  misc_feature
<222>  (19)..(19)
<223>  2'-O-methyl cytidine

<400>  4
uaauccaaca aagucuggcu t                                                21


<210>  5
<211>  21
<212>  DNA
<213>  Artificial Sequence

<220>
<223>  4-Z antisense



<220>
<221>  misc_feature
<222>  (1)..(1)
<223>  2'-fluoro uridine

<220>
<221>  misc_feature
<222>  (2)..(20)
<223>  RNA

<220>
<221>  misc_feature
<222>  (3)..(3)
<223>  2'-O-methyl adenosine

<220>
<221>  misc_feature
<222>  (5)..(5)
<223>  2'-O-methyl cytidine

<220>
<221>  misc_feature
<222>  (7)..(7)
<223>  2'-O-methyl adenosine

<220>
<221>  misc_feature
<222>  (9)..(9)
<223>  2'-O-methyl cytidine

<220>
<221>  misc_feature
<222>  (11)..(11)
<223>  2'-O-methyl adenosine

<220>
<221>  misc_feature
<222>  (13)..(13)
<223>  2'-O-methyl guanosine

<220>
<221>  misc_feature
<222>  (15)..(15)
<223>  2'-O-methyl cytidine

<220>
<221>  misc_feature
<222>  (17)..(17)
<223>  2'-O-methyl guanosine

<220>
<221>  misc_feature
<222>  (19)..(19)
<223>  2'-O-methyl cytidine

<400>  5
uaauccaaca aagucuggcu t                                                21


<210>  6
<211>  21
<212>  DNA
<213>  Artificial Sequence

<220>
<223>  5-Z sense, 6-fU sense


<220>
<221>  misc_feature
<222>  (1)..(8)
<223>  RNA

<220>
<221>  misc_feature
<222>  (2)..(2)
<223>  2'-O-methyl cytidine

<220>
<221>  misc_feature
<222>  (4)..(4)
<223>  2'-O-methyl adenosine

<220>
<221>  misc_feature
<222>  (6)..(6)
<223>  2'-O-methyl adenosine

<220>
<221>  misc_feature
<222>  (8)..(8)
<223>  2'-O-methyl uridine

<220>
<221>  misc_feature
<222>  (9)..(9)
<223>  2'-fluoro uridine

<220>
<221>  misc_feature
<222>  (10)..(12)
<223>  RNA

<220>
<221>  misc_feature
<222>  (10)..(10)
<223>  2'-O-methyl uridine

<220>
<221>  misc_feature
<222>  (12)..(12)
<223>  2'-O-methyl uridine

<220>
<221>  misc_feature
<222>  (13)..(13)
<223>  2'-fluoro uridine

<220>
<221>  misc_feature
<222>  (14)..(16)
<223>  RNA

<220>
<221>  misc_feature
<222>  (14)..(14)
<223>  2'-O-methyl guanosine

<220>
<221>  misc_feature
<222>  (16)..(16)
<223>  2'-O-methyl adenosine

<220>
<221>  misc_feature
<222>  (17)..(17)
<223>  2'-fluoro uridine

<220>
<221>  misc_feature
<222>  (18)..(20)
<223>  RNA

<220>
<221>  misc_feature

<222>  (18)..(18)
<223>  2'-O-methyl uridine

<220>
<221>  misc_feature
<222>  (20)..(20)
<223>  2'-O-methyl guanosine

<400>  6
gccagacuuu guuggauuag t                                                21


<210>  7
<211>  21
<212>  DNA
<213>  Artificial Sequence

<220>
<223>  5-Z antisense



<220>
<221>  misc_feature
<222>  (1)..(1)
<223>  2'-fluoro uridine

<220>
<221>  misc_feature
<222>  (2)..(3)
<223>  RNA

<220>
<221>  misc_feature
<222>  (3)..(3)
<223>  2'-O-methyl adenosine

<220>
<221>  misc_feature
<222>  (4)..(4)
<223>  2'-fluoro uridine

<220>
<221>  misc_feature
<222>  (5)..(13)
<223>  RNA

<220>
<221>  misc_feature
<222>  (5)..(5)
<223>  2'-O-methyl cytidine

<220>
<221>  misc_feature
<222>  (7)..(7)
<223>  2'-O-methyl adenosine

<220>
<221>  misc_feature
<222>  (9)..(9)
<223>  2'-O-methyl cytidine

<220>
<221>  misc_feature

<222>  (11)..(11)
<223>  2'-O-methyl adenosine

<220>
<221>  misc_feature
<222>  (13)..(13)
<223>  2'-O-methyl guanosine

<220>
<221>  misc_feature
<222>  (14)..(14)
<223>  2'-fluoro uridine

<220>
<221>  misc_feature
<222>  (15)..(15)
<223>  RNA

<220>
<221>  misc_feature
<222>  (15)..(15)
<223>  2'-O-methyl cytidine

<220>
<221>  misc_feature
<222>  (16)..(16)
<223>  2'-fluoro uridine

<220>
<221>  misc_feature
<222>  (17)..(19)
<223>  RNA

<220>
<221>  misc_feature
<222>  (17)..(17)
<223>  2'-O-methyl guanosine

<220>
<221>  misc_feature
<222>  (19)..(19)
<223>  2'-O-methyl cytidine

<220>
<221>  misc_feature
<222>  (20)..(20)
<223>  2'-fluoro uridine

<400>  7
uaauccaaca aagucuggcu t                                                21


<210>  8
<211>  21
<212>  DNA
<213>  Artificial Sequence

<220>
<223>  6-fU antisense


<220>
<221>  misc_feature

<222>  (1)..(1)
<223>  2'-fluoro uridine

<220>
<221>  misc_feature
<222>  (2)..(3)
<223>  RNA

<220>
<221>  misc_feature
<222>  (3)..(3)
<223>  2'-O-methyl adenosine

<220>
<221>  misc_feature
<222>  (4)..(4)
<223>  2'-fluoro uridine

<220>
<221>  misc_feature
<222>  (5)..(13)
<223>  RNA

<220>
<221>  misc_feature
<222>  (5)..(5)
<223>  2'-O-methyl cytidine

<220>
<221>  misc_feature
<222>  (7)..(7)
<223>  2'-O-methyl adenosine

<220>
<221>  misc_feature
<222>  (9)..(9)
<223>  2'-O-methyl cytidine

<220>
<221>  misc_feature
<222>  (11)..(11)
<223>  2'-O-methyl adenosine

<220>
<221>  misc_feature
<222>  (13)..(13)
<223>  2'-O-methyl guanosine

<220>
<221>  misc_feature
<222>  (14)..(14)
<223>  2'-fluoro uridine

<220>
<221>  misc_feature
<222>  (15)..(15)
<223>  RNA

<220>
<221>  misc_feature
<222>  (15)..(15)
<223>  2'-O-methyl cytidine

<220>
<221>  misc_feature
<222>  (16)..(16)
<223>  2'-fluoro uridine

<220>
<221>  misc_feature
<222>  (17)..(19)
<223>  RNA

<220>
<221>  misc_feature
<222>  (17)..(17)
<223>  2'-O-methyl guanosine

<220>
<221>  misc_feature
<222>  (19)..(19)
<223>  2'-O-methyl cytidine

<220>
<221>  misc_feature
<222>  (20)..(20)
<223>  2'-fluoro uridine

<400>  8
uaauccaaca aagucuggcu t                                                21


<210>  9
<211>  16
<212>  DNA
<213>  Artificial Sequence

<220>
<223>  CD45 ABC



<220>
<221>  misc_feature
<222>  (1)..(2)
<223>  2'-O-methyl cytidine

<220>
<221>  misc_feature
<222>  (1)..(4)
<223>  RNA

<220>
<221>  misc_feature
<222>  (2)..(16)
<223>  5'-phosphorothioated

<220>
<221>  misc_feature
<222>  (3)..(4)
<223>  2'-O-methyl adenosine

<220>
<221>  misc_feature
<222>  (13)..(13)
<223>  2'-O-methyl adenosine

<220>
<221>  misc_feature
<222>  (13)..(15)
<223>  RNA

<220>
<221>  misc_feature
<222>  (14)..(14)
<223>  2'-O-methyl guanosine

<220>
<221>  misc_feature
<222>  (15)..(15)
<223>  2'-O-methyl uridine

<400>  9
ccaaatgcca agagut                                                       16


<210>  10
<211>  16
<212>  DNA
<213>  Artificial Sequence

<220>
<223>  CD45 x2PO



<220>
<221>  misc_feature
<222>  (1)..(2)
<223>  2'-O-methyl cytidine

<220>
<221>  misc_feature
<222>  (1)..(5)
<223>  RNA

<220>
<221>  misc_feature
<222>  (2)..(6)
<223>  5'-phosphorothioated

<220>
<221>  misc_feature
<222>  (3)..(5)
<223>  2'-O-methyl adenosine

<220>
<221>  misc_feature
<222>  (8)..(15)
<223>  5'-phosphorothioated

<220>
<221>  misc_feature
<222>  (13)..(13)
<223>  2'-O-methyl adenosine

<220>
<221>  misc_feature
<222>  (13)..(15)
<223>  RNA

<220>
<221>  misc_feature
<222>  (14)..(14)
<223>  2'-O-methyl guanosine

<220>
<221>  misc_feature
<222>  (15)..(15)
<223>  2'-O-methyl uridine

<400>  10
ccaaatgcca agagut                                                    16


<210>  11
<211>  16
<212>  DNA
<213>  Artificial Sequence

<220>
<223>  CD45 x2IC



<220>
<221>  misc_feature
<222>  (1)..(2)
<223>  2'-O-methyl cytidine

<220>
<221>  misc_feature
<222>  (1)..(5)
<223>  RNA

<220>
<221>  misc_feature
<222>  (2)..(6)
<223>  5'-phosphorothioated

<220>
<221>  misc_feature
<222>  (3)..(5)
<223>  2'-O-methyl adenosine

<220>
<221>  misc_feature
<222>  (6)..(6)
<223>  n = thymidine with a substituted phosphate group at 3'-position
       (Y1)

<220>
<221>  misc_feature
<222>  (8)..(15)
<223>  5'-phosphorothioated

<220>
<221>  misc_feature
<222>  (13)..(14)
<223>  RNA

<220>
<221>  misc_feature
<222>  (13)..(13)
<223>  2'-O-methyl adenosine

<220>
<221>  misc_feature
<222>  (14)..(14)
<223>  2'-O-methyl guanosine

<220>
<221>  misc_feature
<222>  (15)..(15)
<223>  n = 2'-deoxy uridine with a substituted phosphate group at
       3'-position (Z1)

<400>  11
ccaaangcca agagut                                                    16


<210>  12
<211>  20
<212>  DNA
<213>  Artificial Sequence

<220>
<223>  FGFR4 PS


<220>
<221>  misc_feature
<222>  (1)..(1)
<223>  LNA

<220>
<221>  misc_feature
<222>  (2)..(2)
<223>  2'-O-methyl cytidine

<220>
<221>  misc_feature
<222>  (2)..(2)
<223>  RNA

<220>
<221>  misc_feature
<222>  (2)..(10)
<223>  5'-phosphorothioated

<220>
<221>  misc_feature
<222>  (3)..(3)
<223>  LNA

<220>
<221>  misc_feature
<222>  (4)..(4)
<223>  2'-O-methyl cytidine

<220>
<221>  misc_feature
<222>  (4)..(4)
<223>  RNA

<220>
<221>  misc_feature
<222>  (5)..(5)

<223>  LNA

<220>
<221>  misc_feature
<222>  (12)..(20)
<223>  5'-phosphorothioated

<220>
<221>  misc_feature
<222>  (16)..(16)
<223>  2'-O-methyl cytidine

<220>
<221>  misc_feature
<222>  (16)..(17)
<223>  RNA

<220>
<221>  misc_feature
<222>  (17)..(17)
<223>  2'-O-methyl guanosine

<220>
<221>  misc_feature
<222>  (18)..(18)
<223>  LNA

<220>
<221>  misc_feature
<222>  (19)..(19)
<223>  2'-O-methyl cytidine

<220>
<221>  misc_feature
<222>  (19)..(19)
<223>  RNA

<400>  12
tctctttggt cacaccgtct                                                    20


<210>  13
<211>  20
<212>  DNA
<213>  Artificial Sequence

<220>
<223>  FGFR4 PO



<220>
<221>  misc_feature
<222>  (1)..(1)
<223>  LNA

<220>
<221>  misc_feature
<222>  (2)..(2)
<223>  RNA

<220>
<221>  misc_feature
<222>  (2)..(2)

<223>  2'-O-methyl cytidine

<220>
<221>  misc_feature
<222>  (2)..(10)
<223>  5'-phosphorothioated

<220>
<221>  misc_feature
<222>  (3)..(3)
<223>  LNA

<220>
<221>  misc_feature
<222>  (4)..(4)
<223>  RNA

<220>
<221>  misc_feature
<222>  (4)..(4)
<223>  2'-O-methyl cytidine

<220>
<221>  misc_feature
<222>  (5)..(5)
<223>  LNA

<220>
<221>  misc_feature
<222>  (12)..(20)
<223>  5'-phosphorothioated

<220>
<221>  misc_feature
<222>  (16)..(17)
<223>  RNA

<220>
<221>  misc_feature
<222>  (16)..(16)
<223>  2'-O-methyl cytidine

<220>
<221>  misc_feature
<222>  (17)..(17)
<223>  2'-O-methyl guanosine

<220>
<221>  misc_feature
<222>  (18)..(18)
<223>  LNA

<220>
<221>  misc_feature
<222>  (19)..(19)
<223>  RNA

<220>
<221>  misc_feature
<222>  (19)..(19)
<223>  2'-O-methyl cytidine

<400>  13

tctctttggt cacaccgtct                                                    20

<210>  14
<211>  20
<212>  DNA
<213>  Artificial Sequence

<220>
<223>  FGFR4 IC

<220>
<221>  misc_feature
<222>  (1)..(1)
<223>  LNA

<220>
<221>  misc_feature
<222>  (2)..(2)
<223>  RNA

<220>
<221>  misc_feature
<222>  (2)..(2)
<223>  2'-O-methyl cytidine

<220>
<221>  misc_feature
<222>  (2)..(10)
<223>  5'-phosphorothioated

<220>
<221>  misc_feature
<222>  (3)..(3)
<223>  LNA

<220>
<221>  misc_feature
<222>  (4)..(4)
<223>  RNA

<220>
<221>  misc_feature
<222>  (4)..(4)
<223>  2'-O-methyl cytidine

<220>
<221>  misc_feature
<222>  (5)..(5)
<223>  LNA

<220>
<221>  misc_feature
<222>  (10)..(10)
<223>  n = thymidine with a substituted phosphate group at 3'-position
       (Y1)

<220>
<221>  misc_feature
<222>  (12)..(20)
<223>  5'-phosphorothioated

<220>
<221>  misc_feature
<222>  (16)..(17)
<223>  RNA

<220>
<221>  misc_feature
<222>  (16)..(16)
<223>  2'-O-methyl cytidine

<220>
<221>  misc_feature
<222>  (17)..(17)
<223>  2'-O-methyl guanosine

<220>
<221>  misc_feature
<222>  (18)..(18)
<223>  LNA

<220>
<221>  misc_feature
<222>  (19)..(19)
<223>  RNA

<220>
<221>  misc_feature
<222>  (19)..(19)
<223>  2'-O-methyl cytidine

<400>  14
tctctttggn cacaccgtct                                                    20


<210>  15
<211>  13
<212>  DNA
<213>  Artificial Sequence

<220>
<223>  wtKON708



<220>
<221>  misc_feature
<222>  (1)..(2)
<223>  RNA

<220>
<221>  misc_feature
<222>  (1)..(2)
<223>  n = 2'-O-methyl 2-metyl uridine (2'-O-methyl thymidine)

<220>
<221>  misc_feature
<222>  (2)..(13)
<223>  5'-phosphorothioated

<220>
<221>  misc_feature
<222>  (11)..(12)
<223>  RNA

<220>
<221>  misc_feature
<222>  (11)..(12)
<223>  2'-O-methyl cytidine

<400>  15
nnagcactgg cct                                                          13


<210>  16
<211>  13
<212>  DNA
<213>  Artificial Sequence

<220>
<223>  wtKON715


<220>
<221>  misc_feature
<222>  (1)..(2)
<223>  2'-O-methyl uridine

<220>
<221>  misc_feature
<222>  (1)..(2)
<223>  RNA

<220>
<221>  misc_feature
<222>  (3)..(13)
<223>  5'-phosphorothioated

<220>
<221>  misc_feature
<222>  (11)..(12)
<223>  2'-O-methyl cytidine

<220>
<221>  misc_feature
<222>  (11)..(12)
<223>  RNA

<400>  16
uuagcactgg cct                                                          13


<210>  17
<211>  13
<212>  DNA
<213>  Artificial Sequence

<220>
<223>  KON708


<220>
<221>  misc_feature
<222>  (1)..(2)
<223>  n = 2'-O-methyl 2-metyl uridine (2'-O-methyl thymidine)

<220>
<221>  misc_feature

<222>  (1)..(2)
<223>  RNA

<220>
<221>  misc_feature
<222>  (2)..(13)
<223>  5'-phosphorothioated

<220>
<221>  misc_feature
<222>  (8)..(8)
<223>  n = thymidine with a substituted phosphate group at 3'-position
       (Y2)

<220>
<221>  misc_feature
<222>  (11)..(12)
<223>  RNA

<220>
<221>  misc_feature
<222>  (11)..(12)
<223>  2'-O-methyl cytidine

<400>  17
nnagcacngg cct                                                          13


<210>  18
<211>  13
<212>  DNA
<213>  Artificial Sequence

<220>
<223>  KON715


<220>
<221>  misc_feature
<222>  (1)..(1)
<223>  n = 2'-deoxy uridine with a substituted phosphate group at
       3'-position (Z2)

<220>
<221>  misc_feature
<222>  (2)..(2)
<223>  RNA

<220>
<221>  misc_feature
<222>  (2)..(2)
<223>  2'-O-methyl uridine

<220>
<221>  misc_feature
<222>  (3)..(13)
<223>  5'-phosphorothioated

<220>
<221>  misc_feature
<222>  (11)..(12)
<223>  RNA

<220>
<221>  misc_feature
<222>  (11)..(12)
<223>  2'-O-methyl cytidine

<400>  18
nuagcactgg cct                                                          13


<210>  19
<211>  21
<212>  DNA
<213>  Artificial Sequence

<220>
<223>  Ctrl(5',3'dT) sense


<220>
<221>  misc_feature
<222>  (1)..(20)
<223>  RNA

<400>  19
gccagacuuu guuggauuag t                                                 21


<210>  20
<211>  21
<212>  DNA
<213>  Artificial Sequence

<220>
<223>  Ctrl(5',3'dT) antisense


<220>
<221>  misc_feature
<222>  (2)..(20)
<223>  RNA

<400>  20
taauccaaca aagucuggcu t                                                 21


<210>  21
<211>  21
<212>  DNA
<213>  Artificial Sequence

<220>
<223>  KON788 antisense


<220>
<221>  misc_feature
<222>  (1)..(1)
<223>  n = thymidine with a substituted phosphate group at 3'-position
       (X4)

<220>
<221>  misc_feature
<222>  (2)..(20)

<223>  RNA

<400>  21
naauccaaca aagucuggcu t                                                21


<210>  22
<211>  21
<212>  DNA
<213>  Artificial Sequence

<220>
<223>  KON789 antisense


<220>
<221>  misc_feature
<222>  (1)..(1)
<223>  n = thymidine with a substituted phosphate group at 3'-position
       (Z3)

<220>
<221>  misc_feature
<222>  (2)..(20)
<223>  RNA

<400>  22
naauccaaca aagucuggcu t                                                21


<210>  23
<211>  21
<212>  DNA
<213>  Artificial Sequence

<220>
<223>  KON816 antisense


<220>
<221>  misc_feature
<222>  (1)..(1)
<223>  n = thymidine with a substituted phosphate group at 3'-position
       (Y4)

<220>
<221>  misc_feature
<222>  (2)..(20)
<223>  RNA

<400>  23
naauccaaca aagucuggcu t                                                21


<210>  24
<211>  21
<212>  DNA
<213>  Artificial Sequence

<220>
<223>  KON818 antisense

<220>
<221>  misc_feature
<222>  (1)..(1)
<223>  n = thymidine with a substituted phosphate group at 3'-position
       (Z4)

<220>
<221>  misc_feature
<222>  (2)..(20)
<223>  RNA

<400>  24
naauccaaca aagucuggcu t                                                21


<210>  25
<211>  21
<212>  DNA
<213>  Artificial Sequence

<220>
<223>  KON840 antisense


<220>
<221>  misc_feature
<222>  (1)..(1)
<223>  n = thymidine with a substituted phosphate group at 3'-position
       (X5)

<220>
<221>  misc_feature
<222>  (2)..(20)
<223>  n = thymidine with a substituted phosphate group at 3'-position
       (X5)

<400>  25
naauccaaca aagucuggcu t                                                21


<210>  26
<211>  21
<212>  DNA
<213>  Artificial Sequence

<220>
<223>  KON846 antisense


<220>
<221>  misc_feature
<222>  (1)..(1)
<223>  n = thymidine with a substituted phosphate group at 3'-position
       (Y5)

<220>
<221>  misc_feature
<222>  (2)..(20)
<223>  RNA

<400>  26
naauccaaca aagucuggcu t                                                21

<210>  27
<211>  21
<212>  DNA
<213>  Artificial Sequence

<220>
<223>  KON857 antisense


<220>
<221>  misc_feature
<222>  (1)..(1)
<223>  n = thymidine with a substituted phosphate group at 3'-position
       (Z5)

<220>
<221>  misc_feature
<222>  (2)..(20)
<223>  RNA

<400>  27
naauccaaca aagucuggcu t                                                21


<210>  28
<211>  21
<212>  DNA
<213>  Artificial Sequence

<220>
<223>  Ctrl(5'-F) antisense


<220>
<221>  misc_feature
<222>  (1)..(1)
<223>  2'-fluoro uridine

<220>
<221>  misc_feature
<222>  (2)..(20)
<223>  RNA

<400>  28
uaauccaaca aagucuggcu t                                                21


<210>  29
<211>  21
<212>  DNA
<213>  Artificial Sequence

<220>
<223>  KON880 antisense


<220>
<221>  misc_feature
<222>  (1)..(1)
<223>  n = 2'-fluoro uridine with a substituted phosphate group at
       3'-position (Y6)

<220>
<221>  misc_feature
<222>  (2)..(20)
<223>  RNA

<400>  29
naauccaaca aagucuggcu t                                                21


<210>  30
<211>  21
<212>  DNA
<213>  Artificial Sequence

<220>
<223>  KON881 antisense


<220>
<221>  misc_feature
<222>  (1)..(1)
<223>  n = 2'-fluoro uridine with a substituted phosphate group at
       3'-position (Z6)

<220>
<221>  misc_feature
<222>  (2)..(20)
<223>  RNA

<400>  30
naauccaaca aagucuggcu t                                                21


<210>  31
<211>  21
<212>  DNA
<213>  Artificial Sequence

<220>
<223>  KON882 antisense


<220>
<221>  misc_feature
<222>  (1)..(1)
<223>  n = thymidine with a substituted phosphate group at 3'-position
       (Y7)

<220>
<221>  misc_feature
<222>  (2)..(20)
<223>  RNA

<400>  31
naauccaaca aagucuggcu t                                                21


<210>  32
<211>  21
<212>  DNA
<213>  Artificial Sequence

<220>

<223>  KON883 antisense

<220>
<221>  misc_feature
<222>  (1)..(1)
<223>  n = 2'-fluoro uridine with a substituted phosphate group at
       5'-position (Z7)

<220>
<221>  misc_feature
<222>  (2)..(20)
<223>  RNA

<400>  32
naauccaaca aagucuggcu t                                                21

<210>  33
<211>  21
<212>  DNA
<213>  Artificial Sequence

<220>
<223>  KON884 antisense

<220>
<221>  misc_feature
<222>  (1)..(1)
<223>  n = 2'-fluoro uridine with a substituted phosphate group at
       5'-position (Y8)

<220>
<221>  misc_feature
<222>  (2)..(20)
<223>  RNA

<400>  33
naauccaaca aagucuggcu t                                                21

<210>  34
<211>  21
<212>  DNA
<213>  Artificial Sequence

<220>
<223>  KON891 antisense

<220>
<221>  misc_feature
<222>  (1)..(1)
<223>  n = thymidine with a substituted phosphate group at 3'-position
       (Z8)

<220>
<221>  misc_feature
<222>  (2)..(20)
<223>  RNA

<400>  34

taauccaaca aagucuggcu t                                                21

<210>  35
<211>  21
<212>  DNA
<213>  Artificial Sequence

<220>
<223>  KON892 antisense

<220>
<221>  misc_feature
<222>  (1)..(1)
<223>  n = thymidine with a substituted phosphate group at 3'-position
       (Y9)

<220>
<221>  misc_feature
<222>  (2)..(20)
<223>  RNA

<400>  35
naauccaaca aagucuggcu t                                                21

<210>  36
<211>  21
<212>  DNA
<213>  Artificial Sequence

<220>
<223>  KON903 antisense

<220>
<221>  misc_feature
<222>  (1)..(1)
<223>  n = 2'-fluoro thymidine with a substituted phosphate group at
       3'-position (Z9)

<220>
<221>  misc_feature
<222>  (2)..(20)
<223>  RNA

<400>  36
naauccaaca aagucuggcu t                                                21

<210>  37
<211>  21
<212>  DNA
<213>  Artificial Sequence

<220>
<223>  KON905 antisense

<220>
<221>  misc_feature
<222>  (1)..(1)

<223>  n = 2′-fluoro thymidine with a substituted phosphate group at
       3′-position (Y10)

<220>
<221>  misc_feature
<222>  (2)..(20)
<223>  RNA

<400>  37
naauccaaca aagucuggcu t                                                21


<210>  38
<211>  21
<212>  DNA
<213>  Artificial Sequence

<220>
<223>  KON922 antisense


<220>
<221>  misc_feature
<222>  (1)..(1)
<223>  n = thymidine with a substituted phosphate group at 3′-position
       (Z10)

<220>
<221>  misc_feature
<222>  (2)..(20)
<223>  RNA

<400>  38
naauccaaca aagucuggcu t                                                21


<210>  39
<211>  21
<212>  DNA
<213>  Artificial Sequence

<220>
<223>  KON923 antisense


<220>
<221>  misc_feature
<222>  (1)..(1)
<223>  n = thymidine with a substituted phosphate group at 3′-position
       (Y11)

<220>
<221>  misc_feature
<222>  (2)..(20)
<223>  RNA

<400>  39
naauccaaca aagucuggcu t                                                21


<210>  40
<211>  21
<212>  RNA

<213>  Artificial Sequence

<220>
<223>  wt924 sense

<400>  40
gucaucacac ugaauaccaa u                                                21


<210>  41
<211>  23
<212>  DNA
<213>  Artificial Sequence

<220>
<223>  wt924 antisense


<220>
<221>  misc_feature
<222>  (2)..(20)
<223>  RNA

<400>  41
tuugguauuc agugugauga cat                                              23


<210>  42
<211>  23
<212>  DNA
<213>  Artificial Sequence

<220>
<223>  wt925 antisense


<220>
<221>  misc_feature
<222>  (2)..(20)
<223>  RNA

<220>
<221>  misc_feature
<222>  (2)..(2)
<223>  5'-phosphorothioated

<400>  42
tuugguauuc agugugauga cat                                              23


<210>  43
<211>  23
<212>  DNA
<213>  Artificial Sequence

<220>
<223>  wt926 antisense


<220>
<221>  misc_feature
<222>  (1)..(1)
<223>  5'-phosphate

<220>
<221>  misc_feature
<222>  (2)..(20)
<223>  RNA

<400>  43
   tuugguauuc agugugauga cat                                              23


<210>  44
<211>  23
<212>  DNA
<213>  Artificial Sequence

<220>
<223>  KON924 antisense


<220>
<221>  misc_feature
<222>  (1)..(1)
<223>  n = thymidine with a substituted phosphate group at 3'-position
       (Z11)

<220>
<221>  misc_feature
<222>  (2)..(20)
<223>  RNA

<400>  44
   nuugguauuc agugugauga cat                                              23


<210>  45
<211>  23
<212>  DNA
<213>  Artificial Sequence

<220>
<223>  KON925 antisense


<220>
<221>  misc_feature
<222>  (1)..(1)
<223>  n = thymidine with a substituted phosphate group at 3'-position
       (Y12)

<220>
<221>  misc_feature
<222>  (2)..(20)
<223>  RNA

<400>  45
   nuugguauuc agugugauga cat                                              23


<210>  46
<211>  23
<212>  DNA
<213>  Artificial Sequence

<220>
<223>  KON926 antisense


<220>
<221>  misc_feature
<222>  (1)..(1)
<223>  n = thymidine with a substituted phosphate group of Z12 at
       5'-position

<220>
<221>  misc_feature
<222>  (2)..(20)
<223>  RNA

<400>  46
nuugguauuc agugugauga cat                                              23


<210>  47
<211>  21
<212>  RNA
<213>  Artificial Sequence

<220>
<223>  wt927 sense

<400>  47
ccgucguauu cgugagcaag a                                                21


<210>  48
<211>  21
<212>  DNA
<213>  Artificial Sequence

<220>
<223>  wt927 antisense


<220>
<221>  misc_feature
<222>  (2)..(20)
<223>  RNA

<400>  48
tugcucacga auacgacggu t                                                21


<210>  49
<211>  21
<212>  DNA
<213>  Artificial Sequence

<220>
<223>  wt928 antisense


<220>
<221>  misc_feature
<222>  (2)..(2)
<223>  5'-phosphorothioated

<220>
<221>  misc_feature
<222>  (2)..(20)
<223>  RNA

<400>  49
tugcucacga auacgacggu t                                                21


<210>  50
<211>  21
<212>  DNA
<213>  Artificial Sequence

<220>
<223>  wt929 antisense


<220>
<221>  misc_feature
<222>  (1)..(1)
<223>  5'-phosphate

<220>
<221>  misc_feature
<222>  (2)..(20)
<223>  RNA

<400>  50
tugcucacga auacgacggu t                                                21


<210>  51
<211>  21
<212>  DNA
<213>  Artificial Sequence

<220>
<223>  KON927 antisense


<220>
<221>  misc_feature
<222>  (1)..(1)
<223>  n = thymidine with a substituted phosphate group at 3'-position
       (Z11)

<220>
<221>  misc_feature
<222>  (2)..(20)
<223>  RNA

<400>  51
nugcucacga auacgacggu t                                                21


<210>  52
<211>  21
<212>  DNA
<213>  Artificial Sequence

<220>
<223>  KON928 antisense

<220>
<221>  misc_feature
<222>  (1)..(1)
<223>  n = thymidine with a substituted phosphate group at 3'-position
      (Y12)

<220>
<221>  misc_feature
<222>  (2)..(20)
<223>  RNA

<400>  52
nugcucacga auacgacggu t                                                21


<210>  53
<211>  21
<212>  DNA
<213>  Artificial Sequence

<220>
<223>  KON929 antisense


<220>
<221>  misc_feature
<222>  (1)..(1)
<223>  n = thymidine with a substituted phosphate group of Z12 at
      5'-position

<220>
<221>  misc_feature
<222>  (2)..(20)
<223>  RNA

<400>  53
nugcucacga auacgacggu t                                                21


<210>  54
<211>  21
<212>  RNA
<213>  Artificial Sequence

<220>
<223>  wt930 sense

<400>  54
ccuucauuga ccucaacuac a                                                21


<210>  55
<211>  21
<212>  DNA
<213>  Artificial Sequence

<220>
<223>  wt930 antisense


<220>

<221>  misc_feature
<222>  (2)..(20)
<223>  RNA

<400>  55
taguugaggu caaugaaggg t                                                 21


<210>  56
<211>  21
<212>  DNA
<213>  Artificial Sequence

<220>
<223>  wt931 antisense


<220>
<221>  misc_feature
<222>  (2)..(2)
<223>  5'-phosphorothioated

<220>
<221>  misc_feature
<222>  (2)..(20)
<223>  RNA

<400>  56
taguugaggu caaugaaggg t                                                 21


<210>  57
<211>  21
<212>  DNA
<213>  Artificial Sequence

<220>
<223>  wt932 antisense


<220>
<221>  misc_feature
<222>  (1)..(1)
<223>  5'-phosphate

<220>
<221>  misc_feature
<222>  (2)..(20)
<223>  RNA

<400>  57
taguugaggu caaugaaggg t                                                 21


<210>  58
<211>  21
<212>  DNA
<213>  Artificial Sequence

<220>
<223>  KON930 antisense

<220>
<221>  misc_feature
<222>  (1)..(1)
<223>  n = thymidine with a substituted phosphate group at 3'-position
       (Z11)

<220>
<221>  misc_feature
<222>  (2)..(20)
<223>  RNA

<400>  58
naguugaggu caaugaaggg t                                                21


<210>  59
<211>  21
<212>  DNA
<213>  Artificial Sequence

<220>
<223>  KON931 antisense


<220>
<221>  misc_feature
<222>  (1)..(1)
<223>  n = thymidine with a substituted phosphate group at 3'-position
       (Y12)

<220>
<221>  misc_feature
<222>  (2)..(20)
<223>  RNA

<400>  59
naguugaggu caaugaaggg t                                                21


<210>  60
<211>  21
<212>  DNA
<213>  Artificial Sequence

<220>
<223>  KON932 antisense


<220>
<221>  misc_feature
<222>  (1)..(1)
<223>  n = thymidine with a substituted phosphate group of Z12 at
       5'-position

<220>
<221>  misc_feature
<222>  (2)..(20)
<223>  RNA

<400>  60
naguugaggu caaugaaggg t                                                21